# EUROPEAN PATENT APPLICATION

(11) **EP 4 582 544 A1**
(43) Date of publication of application: **09.07.2025**
(21) Application number: 23860450.8
(22) Date of filing: 30.08.2023
(51) Int. Cl.: C12N 15/12, A61K 35/12, A61K 38/16, A61K 48/00, A61P 27/02, C07K 14/47, C12N 1/15, C12N 1/19, C12N 1/21, C12N 5/10

(54) **LIGHT-RESPONSIVE MODIFIED OPSIN**

(30) Priority: 31.08.2022 JP 2022138707
(71) Applicant: Kyoto University, Kyoto-shi, Kyoto 606-8501 (JP); Restore Vision Inc., Tokyo, 105-6415 (JP)
(72) Inventor: YAMASHITA, Takahiro, Kyoto-shi, Kyoto 606-8501 (JP); SAKAI, Kazumi, Kyoto-shi, Kyoto 606-8501 (JP); SHICHIDA, Yoshinori, Kyoto-shi, Kyoto 606-8501 (JP); SATO, Keita, Kyoto-shi, Kyoto 606-8501 (JP); AOKI, Shion, Kyoto-shi, Kyoto 606-8501 (JP); KATADA, Yusaku, Tokyo 105-6415 (JP)
(74) Representative: Vossius & Partner Patentanwälte Rechtsanwälte mbB
(86) International application number: PCT/JP2023/031644
(87) International publication number: WO 2024/048688

(57) **Abstract**

The present disclosure provides an opsin that has a light cycle characteristic. Specifically, the present disclosure provides a protein including an amino acid sequence of an opsin, wherein the amino acid sequence of the opsin includes a modification of the amino acid corresponding to position 188 when aligned with SEQ ID NO: 1. The present disclosure also provides a medicine, preparation, composition, treatment method, and the like that include said protein and/or a nucleic acid molecule, a nucleic acid construct, and/or cells related to said protein.

## Description

### [Technical Field]

The present disclosure relates to a protein including an amino acid sequence of an opsin, a nucleic acid molecule including a nucleic acid encoding the amino acid sequence of the protein, a nucleic acid construct including the nucleic acid molecule, a cell including the nucleic acid construct, and a medicament including the cell.

### [Background Art]

Opsin is a photoreceptive protein that is ubiquitous in animals and is classified into three types based on its photoreactivity. Vertebrate rhodopsin, a kind of an opsin, forms an active state upon light irradiation (photostimulation) but does not return to its original dark state by a light reaction or a thermal reaction. On the other hand, many opsins other than animal rhodopsin, such as channel rhodopsin, can form an active state upon light irradiation (photostimulation) and then return to a dark state by a thermal reaction.

### [Summary of Invention]

### [Solution to Problem]

The prevent inventors found that even in vertebrate rhodopsin, introduction of a single mutation at position 188 results in acquisition of a photocycle and photoreversibility and thus that a residue at position 188 contributes to diversification of photoreactivities of an opsin by controlling recovery from an active state to an original dark state.

Therefore, the present disclosure provides the following items.

### (Item X1)

A protein including
an amino acid sequence of an opsin,
the protein including a modification at an amino acid corresponding to position 188 when aligned with SEQ ID NO: 1 in the amino acid sequence of the opsin.

### (Item X2-1)

A protein including
an amino acid sequence of an opsin,
the protein including a modification at an amino acid corresponding to G, T, S, or E at position 188 when aligned with SEQ ID NO: 1 in the amino acid sequence of the opsin.

### (Item X2-2)

A protein including
an amino acid sequence of an opsin,
the protein including a modification at an amino acid corresponding to G at position 188 when aligned with SEQ ID NO: 1 in the amino acid sequence of the opsin.

### (Item X3)

The protein according to any one of the above items, including a modification to cysteine of an amino acid corresponding to position G188 when aligned with SEQ ID NO: 1 in the amino acid sequence.

### (Item X4)

The protein according to any one of the above items, in which the protein is inactivated without releasing a photoreceptive factor after being activated by photostimulation.

### (Item X5)

The protein according to any one of the above items, in which the amino acid sequence includes
1) an amino acid sequence including the modification in an amino acid sequence represented by any one of SEQ ID NOs: 1 to 34;
2) an amino acid sequence including a sequence other than a site of the modification having an identity of at least about 80% to the sequence in 1), and encoding a protein having substantially the same biological activity as that of a protein obtained from the sequence in 1);
3) an amino acid sequence having one or more mutations in the sequence in 1) other than the site of the modification and encoding a protein having substantially the same biological activity as that of a protein obtained from the sequence in 1);
4) an amino acid sequence including the modification in an amino acid sequence encoded by a nucleic acid to which a nucleic acid encoding the sequence in 1) hybridizes; or
5) an amino acid sequence including the modification in an amino acid sequence encoded by an allelic variant of the nucleic acid encoding the sequence in 1).

### (Item X6)

The protein according to any one of the above items, further including a modification of an amino acid corresponding to position 122 when aligned with SEQ ID NO: 1 in the amino acid sequence of the opsin.

### (Item X7)

The protein according to any one of the above items, further including a modification of an amino acid corresponding to E at position 122 when aligned with SEQ ID NO: 1 in the amino acid sequence of the opsin.

### (Item X8)

The protein according to any one of the above items, further including a modification to glutamine of an amino acid corresponding to position 122 when aligned with SEQ ID NO: 1 in the amino acid sequence of the opsin.

### (Item X9)

The protein according to any one of the above items, in which the photoreceptive factor includes retinal.

### (Item X9-1)

The protein according to any one of the above items, further including modifications of amino acids corresponding to an amino acid belonging to a portion of a N-terminal domain (positions 1 to 11) and an amino acid belonging to a portion of an extracellular third loop (positions 279 to 285) when aligned with SEQ ID NO: 1 in the amino acid sequence of the opsin.

### (Item X9-2)

The protein according to any one of the above items, further including modifications to cysteine of amino acids corresponding to an amino acid belonging to a portion of the N-terminal domain (positions 1 to 11) and an amino acid belonging to a portion of the extracellular third loop (positions 279 to 285) when aligned with SEQ ID NO: 1 in the amino acid sequence of the opsin.

### (Item X9-3)

The protein according to any one of the above items, further including modifications of amino acids corresponding to positions 2 and 282 when aligned with SEQ ID NO: 1 in the amino acid sequence of the opsin.

### (Item X9-4)

The protein according to any one of the above items, in which the modifications of the amino acids corresponding to positions 2 and 282 improves a thermal stability of the opsin.

### (Item X9-5)

The protein according to any one of the above items, in which the modifications of the amino acids corresponding to positions 2 and 282 includes a modification to cysteine of the amino acids corresponding to positions 2 and 282.

### (Item X10A)

The protein according to any one of the above items, in which the opsin is a chimeric opsin.

### (Item X10B)

The protein according to any one of the above items, in which the opsin is a chimeric opsin in which an amino acid sequence that binds to a G protein in an amino acid sequence of one opsin is replaced by an amino acid sequence that binds to a G protein in another opsin.

### (Item X10C)

The protein according to any one of the above items, in which the opsin is a chimeric opsin in which an amino acid sequence that binds to a G protein in an amino acid sequence of one opsin is replaced by a functional sequence derived from another organism.

### (Item X10D)

The protein according to any one of the above items, in which the opsin is a chimeric opsin from a same or different organism.

### (Item X10E)

The protein according to any one of the above items, in which the organism is selected from the group consisting of a vertebrate, an invertebrate, and a microorganism.

### (Item X10F1)

The protein according to any one of the above items, in which the opsin is a chimeric opsin of a microorganism opsin and a vertebrate opsin.

### (Item X10F2)

The protein according to any one of the above items, in which the opsin is a chimeric opsin of a microorganism opsin and an invertebrate opsin.

### (Item X10F3)

The protein according to any one of the above items, in which the opsin is a chimeric opsin of a vertebrate opsin and an invertebrate opsin.

### (Item X10F4)

The protein according to any one of the above items, in which the opsin is a chimeric opsin of microorganism opsins.

### (Item X10F5)

The protein according to any one of the above items, in which the opsin is a chimeric opsin of vertebrate opsins.

### (Item X10F6)

The protein according to any one of the above items, in which the opsin is a chimeric opsin of invertebrate opsins.

### (Item X10)

The protein according to any one of the above items, including a sequence of SEQ ID NO: 1, 3, or 5.

### (Item X11)

A nucleic acid molecule including a nucleic acid encoding the amino acid sequence of the protein according to any one of the above items.

### (Item X12)

A nucleic acid construct including the nucleic acid molecule according to any one of the above items.

### (Item X13)

A cell including:
the protein according to any one of the above items;
the nucleic acid molecule according to any one of the above items; and/or
the nucleic acid construct according to any one of the above items.

### (Item X14)

A medicament including:
the protein according to any one of the above items;
the nucleic acid molecule according to any one of the above items;
the nucleic acid construct according to any one of the above items; and/or
the cell according to any one of the above items.

### (Item X15)

The medicament according to any one of the above items for regenerating vision or for preventing or treating a visual disorder or disease.

### (Item 1)

A composition including
opsin that is inactivated without releasing a photoreceptive factor.

### (Item 2)

The composition according to any one of the above items, in which the opsin makes a transient change in a cAMP concentration upon photostimulation.

### (Item 3)

The composition according to any one of the above items, in which the transient change in the cAMP concentration is a decrease in the cAMP concentration.

### (Item 4)

The composition according to any one of the above items, in which the opsin includes an amino acid sequence represented by any one of SEQ ID NOs: 1 to 34.

### (Item 5)

The composition according to any one of the above items, in which the opsin includes a G protein-coupled receptor rhodopsin.

### (Item 6)

The composition according to any one of the above items, in which the G protein-coupled receptor rhodopsin is (visual rhodopsin) derived from a mammal.

### (Item 7)

The composition according to any one of the above items, in which the G protein-coupled receptor rhodopsin is a modified G protein-coupled receptor rhodopsin.

### (Item 8)

The composition according to any one of the above items, in which the modification includes G188C when aligned with SEQ ID NO: 1.

### (Item 9)

The composition according to any one of the above items, in which the photoreceptive factor includes retinal.

### (Item 10)

The composition according to any one of the above items, in which the modification further includes E122Q when aligned with SEQ ID NO: 1.

### (Item 11)

The composition according to any one of the above items, in which the modification further includes modifications to cysteine of amino acids corresponding to positions 2 and 282 when aligned with SEQ ID NO: 1.

### (Item 12)

The composition according to any one of the above items for use in a medicament.

### (Item 13)

The composition according to any one of the above items for regenerating vision or for preventing or treating a visual disorder or disease.

### (Item Z1)

A method for modifying or imparting a visual function, the method including
using opsin that is inactivated without releasing a photoreceptive factor.

### (Item Z2)

A method for using as a photoswitch, the method including
using opsin that is inactivated without releasing a photoreceptive factor.

### (Item Z3)

A method including
using opsin that is inactivated without releasing a photoreceptive factor. (Item A1)

A protein including an amino acid sequence of an opsin, in which the protein has a modification so as to be inactivated without releasing a photoreceptive factor after being activated by photostimulation in the opsin and the opsin activates a Gs or Gq subfamily of a G protein.

### (Item A1a)

The protein according to any one of the above items, in which the opsin activates the Gs subfamily of the G protein.

### (Item A1b)

The protein according to any one of the above items, in which the opsin increases a cAMP concentration by activating the G protein.

### (Item A1c)

The protein according to any one of the above items, in which the opsin is a chimeric opsin in which an intracellular second and third loops in the opsin are replaced by those in an activated Gs- or Gq-type G protein-coupled receptor.

### (Item A2)

The protein according to any one of the above items, in which the modification so as to be inactivated without releasing a photoreceptive factor after being activated by photostimulation is achieved by modifying an amino acid corresponding to position 188 when aligned with SEQ ID NO: 1 in the amino acid sequence.

### (Item A3)

The protein according to any one of the above items, in which the amino acid corresponding to position 188 includes G, T, S, or E.

### (Item A4)

The protein according to any one of the above items, in which the amino acid corresponding to position 188 is G.

### (Item A5)

The protein according to any one of the above items, including a modification to cysteine of an amino acid corresponding to position G188 when aligned with SEQ ID NO: 1 in the amino acid sequence.

### (Item A6)

The protein according to any one of the above items, in which the amino acid sequence includes
1) an amino acid sequence including the modification in an amino acid sequence represented by any one of SEQ ID NOs: 1 to 34;
2) an amino acid sequence including a sequence other than a site of the modification having an identity of at least about 80% to the sequence in 1), and encoding a protein having substantially the same biological activity as that of a protein obtained from the sequence in 1);
3) an amino acid sequence having one or more mutations in the sequence in 1) other than the site of the modification and encoding a protein having substantially the same biological activity as that of a protein obtained from the sequence in 1);
4) an amino acid sequence including the modification in an amino acid sequence encoded by a nucleic acid to which a nucleic acid encoding the sequence in 1) hybridizes; or
5) an amino acid sequence including the modification in an amino acid sequence encoded by an allelic variant of the nucleic acid encoding the sequence in 1).

### (Item A7)

The protein according to any one of the above items, further including a modification of an amino acid corresponding to position 122 when aligned with SEQ ID NO: 1 in the amino acid sequence of the opsin.

### (Item A8)

The protein according to any one of the above items, in which the amino acid corresponding to position 122 is E.

### (Item A9)

The protein according to any one of the above items, including a modification to glutamine of an amino acid corresponding to position G122 when aligned with SEQ ID NO: 1 in the amino acid sequence.

### (Item A10)

The protein according to any one of the above items, in which the photoreceptive factor includes retinal.

### (Item A11-1)

The protein according to any one of the above items, further including modifications of amino acids corresponding to an amino acid belonging to a portion of a N-terminal domain (positions 1 to 11) and an amino acid belonging to a portion of an extracellular third loop (positions 278 to 285) when aligned with SEQ ID NO: 1 in the amino acid sequence of the opsin.

### (Item A11-2)

The protein according to any one of the above items, further including modifications to cysteine of amino acids corresponding to an amino acid belonging to a portion of the N-terminal domain (positions 1 to 11) and an amino acid belonging to a portion of the extracellular third loop (positions 278 to 285) when aligned with SEQ ID NO: 1 in the amino acid sequence of the opsin.

### (Item A11-3)

The protein according to any one of the above items, further including modifications of amino acids corresponding to positions 2 and 282 when aligned with SEQ ID NO: 1 in the amino acid sequence of the opsin.

### (Item A11-4)

The protein according to any one of the above items, in which the modifications of the amino acids corresponding to positions 2 and 282 improves a thermal stability of the opsin.

### (Item A11-5)

The protein according to any one of the above items, in which the modifications of the amino acids corresponding to positions 2 and 282 includes a modification to cysteine of the amino acids corresponding to positions 2 and 282.

### (Item A12a)

The protein according to any one of the above items, including a sequence of SEQ ID NO: 1, 3, or 5.

### (Item A12)

A nucleic acid molecule including a nucleic acid encoding an amino acid sequence of the protein according to any one of the above items.

### (Item A13)

A nucleic acid construct including the nucleic acid molecule according to any one of the above items.

### (Item A14)

A cell including:
the protein according to any one of the above items;
the nucleic acid molecule according to any one of the above items; and/or
the nucleic acid construct according to any one of the above items.

### (Item A15)

A medicament including:
a cell including the protein according to any one of the above items, the nucleic acid molecule according to any one of the above items, and/or the nucleic acid construct according to any one of the above items.

### (Item A16)

The medicament according to any one of the above items for regenerating vision or for preventing or treating a visual disorder or disease.

In the present disclosure, it is intended that one or more of the above features may be provided in further combinations in addition to the explicit combinations. Those skilled in the art will recognize further embodiments and advantages of the present disclosure upon reading and understanding the following detailed description, if necessary.

### [Advantageous Effects of Invention]

The present disclosure has confirmed that even in vertebrate rhodopsin, introduction of a single mutation at position 188 results in acquisition of a photocycle and photoreversibility, that is, a property of automatically returning to dark adaptation after light irradiation, which could not be achieved with a conventional technology, indicating a possibility of its application as an optogenetics tool.

### [Brief Description of Drawings]

[FIG. 1] FIG. 1 shows a thermal recovery of a G188C mutant of bovine rhodopsin after irradiation with yellow light.
[FIG. 2] FIG. 2 shows a photoresponse, a retinal arrangement, and a G protein activation of a G188C mutant of bovine rhodopsin.
[FIG. 3] FIG. 3 shows faster recovery of a photocycle property of a G188C mutant of bovine rhodopsin by introducing an E122Q mutation.
[FIG. 4] FIG. 4 shows suppression of an intracellular cAMP level by light in a bovine rhodopsin mutant.
[FIG. 5] FIG. 5 shows formation of a photopigment in a G188C mutant of bovine rhodopsin after incubation with all-trans retinal.
[FIG. 6] FIG. 6 shows a change in a cAMP concentration as measured using a chimeric opsin in which intracellular second and third loops in an E122Q/G188C mutant of bovine rhodopsin were replaced by those of a mouse histamine H2 receptor.
[FIG. 7] FIG. 7 shows acquisition of a photocycle property in an Opn5m T188C mutant of Western clawed frog (Xenopus tropicalis).
[FIG. 8] FIG. 8 is a graph showing a change in a cAMP concentration as measured using an E122Q/G188C mutant of human rhodopsin.
[FIG. 9] FIG. 9 is an alignment diagram of amino acid sequences of opsins.
[FIG. 9-2] Ditto.
[FIG. 9-3] Ditto.
[FIG. 9-4] Ditto.
[FIG. 9-5] Ditto.
[FIG. 9-6] Ditto.
[FIG. 9-7] Ditto.
[FIG. 9-8] Ditto.
[FIG. 9-9] Ditto.
[FIG. 10] FIG. 10 is a graph showing a change in a cAMP concentration as measured using a G188C mutant of canine rhodopsin.
[FIG. 11] FIG. 11 is a graph showing a change in a cAMP concentration as measured using a G188C mutant of medaka rhodopsin.
[FIG. 12] FIG. 12 shows a change in a cAMP concentration as measured using a G6A/G188C/N2C/D282C mutant and a V337A/G188C/N2C/D282C mutant of human rhodopsin.
[FIG. 13] FIG. 13 shows a change in a cAMP concentration as measured using a G188C/N2C/G3C/G280C mutant, a G188C/N2C/G3C/S281C mutant, and a G188C/G3C/N282C mutant of human rhodopsin.

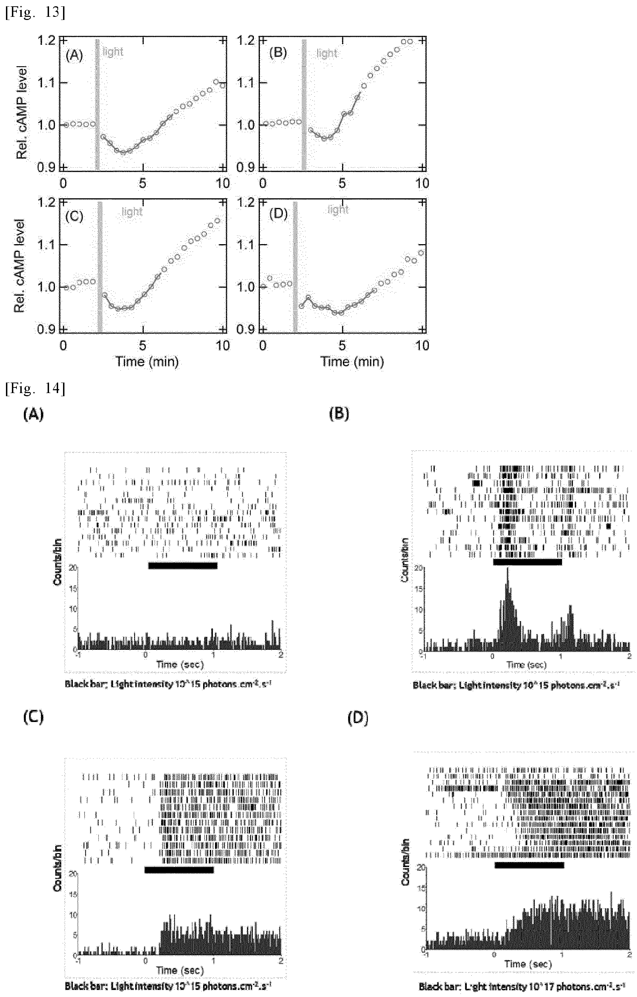

[FIG. 14] FIG. 14 is a graph showing a frequency of a neural activity extracted from extracellular potentials of ganglion cells (RGCs) measured using a multi-electrode array (MEA). FIG. 14A shows an untreated rd1 mouse (no photoresponse is observed because it is blind). FIG. 14B shows the case in which a G188C/N2C/D282C mutant of human rhodopsin was introduced with a viral vector, FIG. 14C shows the case in which intracellular second and third loops of a G188C/N2C/D282C mutant of human rhodopsin were replaced by those of a human histamine H2 receptor, and FIG. 14D shows the case in which intracellular second and third loops of an E122Q/G188C/N2C/D282C mutant of human rhodopsin were replaced by those of a human histamine H2 receptor.
[FIG. 15] FIG. 15 shows a change in a cAMP concentration as measured using an N2C/D282C mutant and an N2C/D282C/G188C mutant of bovine rhodopsin.

### [Description of Embodiments]

The present disclosure will be described with reference to the best mode thereof. It should be understood that throughout this specification, singular expressions also include the concept of their plural forms unless otherwise noted. Thus, it should be understood that singular articles (e.g., "a", "an", "the", etc. in English) also include the concept of their plural forms, unless otherwise noted. It should also be understood that terms used herein are to be used in the sense commonly used in the art unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail.

### (Definitions)

Definitions and/or basic technical details of terms specifically used herein will be described as appropriate.

As used herein, the term "opsin" or "opsins" refers to a protein or proteins that bind to retinal, a pigment functioning as a photoreceptive factor, or an analogue thereof and utilize the retinal or the analogue thereof as a chromophore.

Types of opsins depend on a species of organism and ttypes of a visual cell, but may include, for example, rod opsin and cone opsin (e.g., blue opsin, green opsin, red opsin). The opsin herein also includes, but is not limited to, melanopsin, encephalopsin, OPN5, RGR, and peropsin. For example, animal opsin is a G protein-coupled receptor (GPCR) having a seven-transmembrane structure, and, when a pigment retinal is bound thereto, constitutes rhodopsin which exhibits a photoreceptive ability. The opsin transduces an external light signal into a cell by activating a trimeric G protein upon photoreception.

The opsin can be broadly classified into microbial opsin (Type I opsin) and animal opsin (Type II opsin), and the animal opsin can be further classified into vertebrate visual opsin, vertebrate non-visual opsin, and invertebrate opsin. The vertebrate non-visual opsin and the invertebrate opsin are also referred to as bistable opsin and, like the microbial opsin, include opsin using all-trans-retinal as a chromophore. The microbial opsin and some of the vertebrate non-visual opsins or the invertebrate opsins are opsins that do not release retinal from rhodopsin that is composed of the opsin and the retinal even upon photoreception and can be used as opsin of the present disclosure. Opsin that can be advantageously used in the present disclosure is opsin that does not release retinal even upon photoreception, such as the microbial opsin and some of the vertebrate non-visual opsins or the invertebrate opsins, as well as the bistable opsin including insect opsin. Opsin to be used in the present disclosure may be advantageously used even if it does not strictly correspond to the microbial opsin, the vertebrate non-visual opsin, or the invertebrate opsin, as long as it is a functional equivalent that has an equivalent function to the opsin that can be advantageously used.

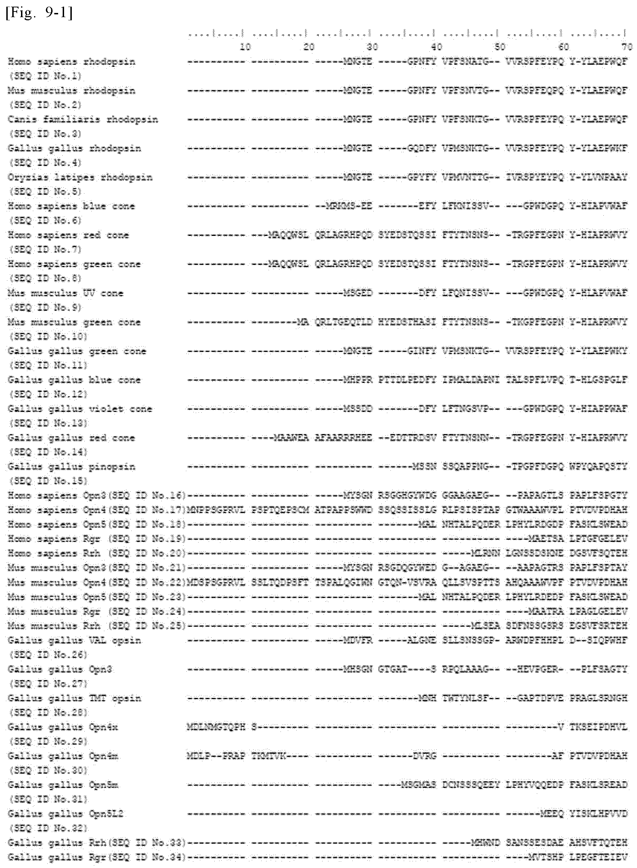

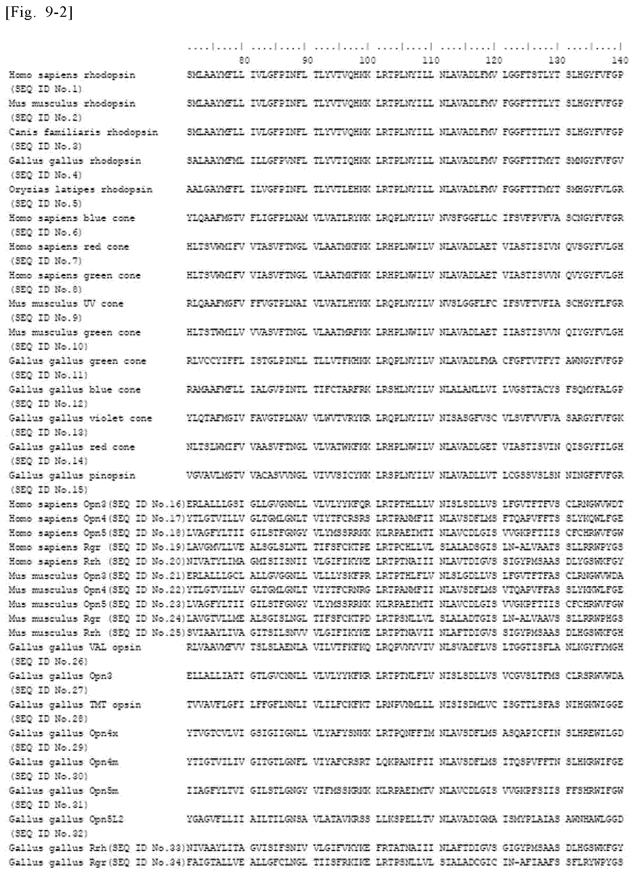

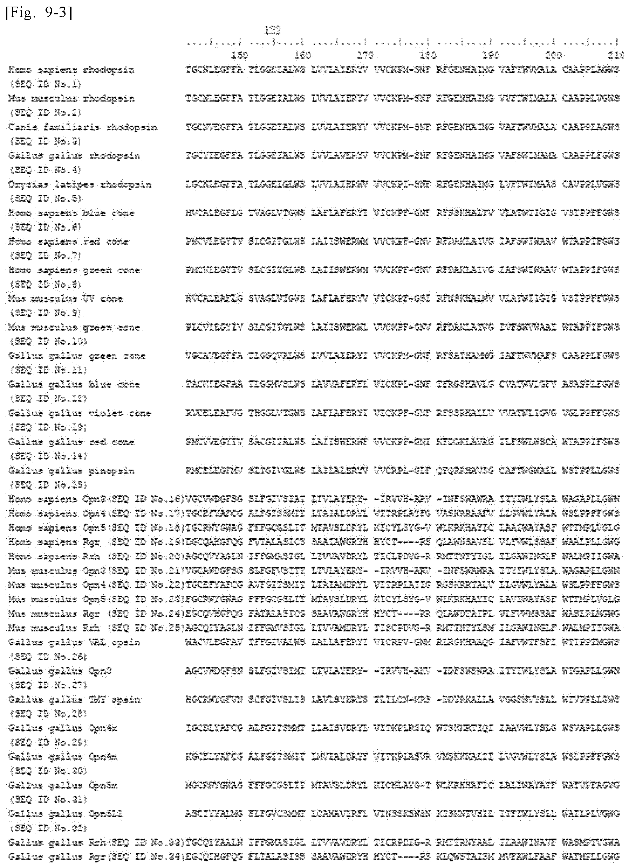

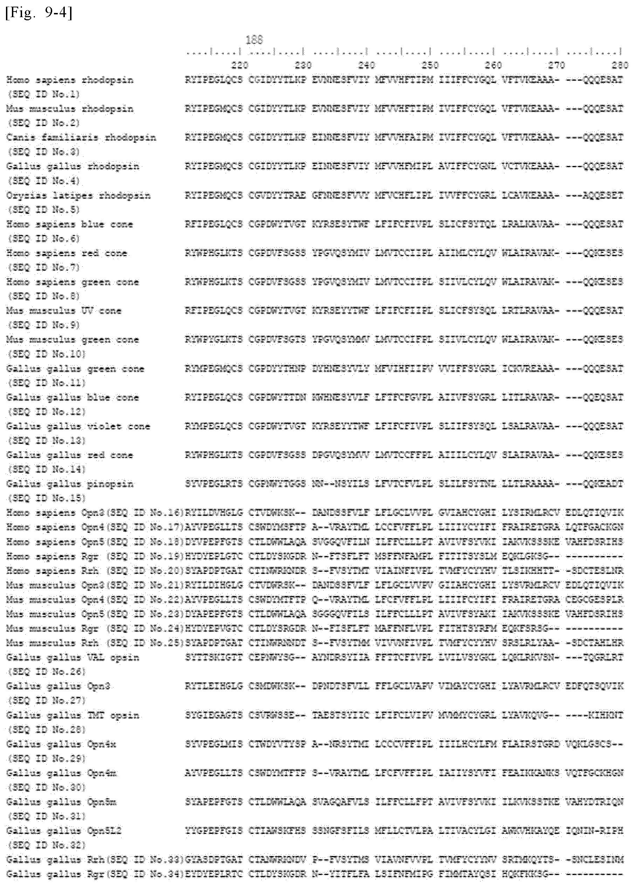

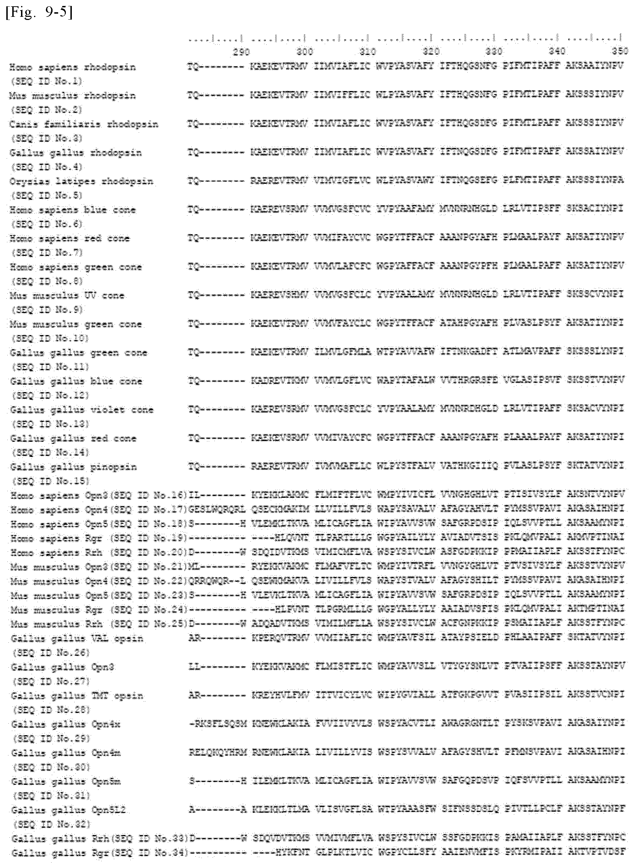

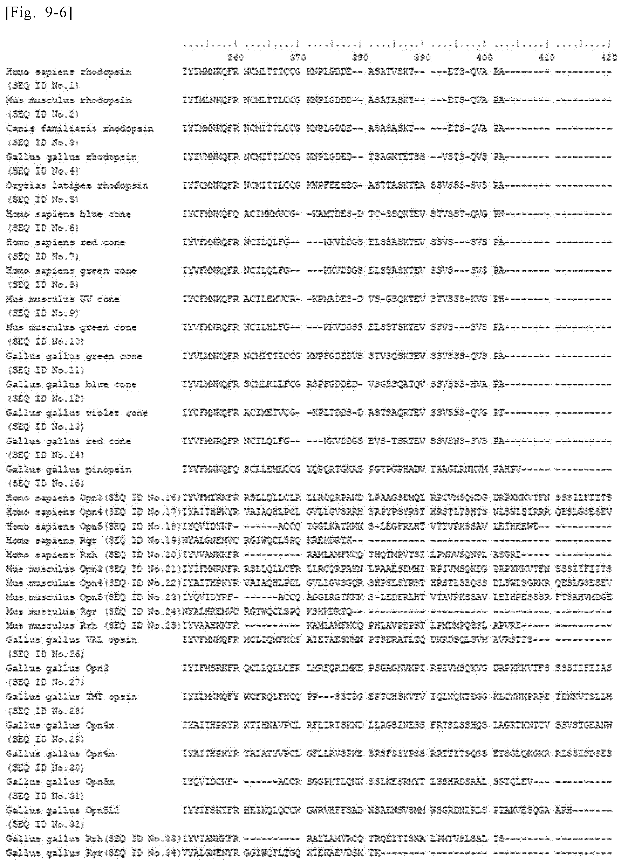

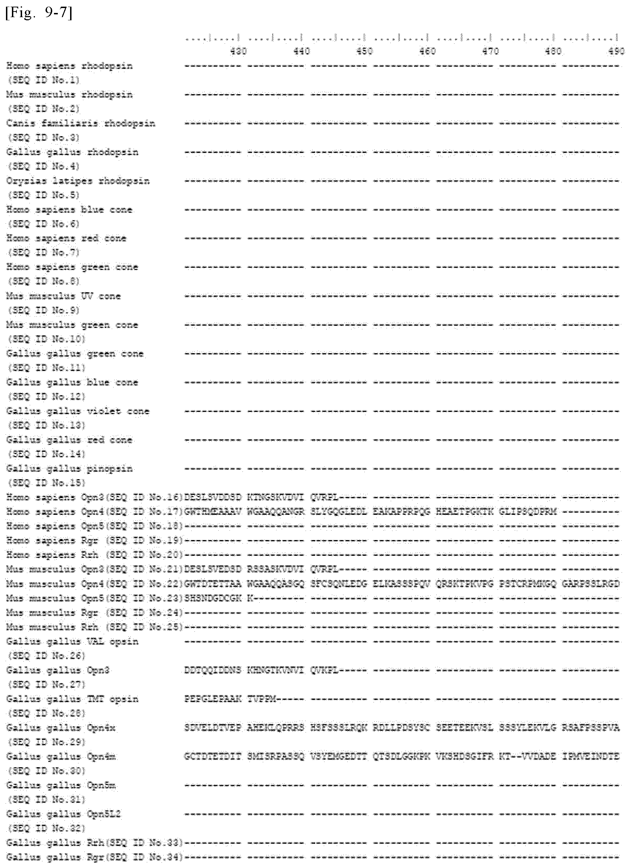

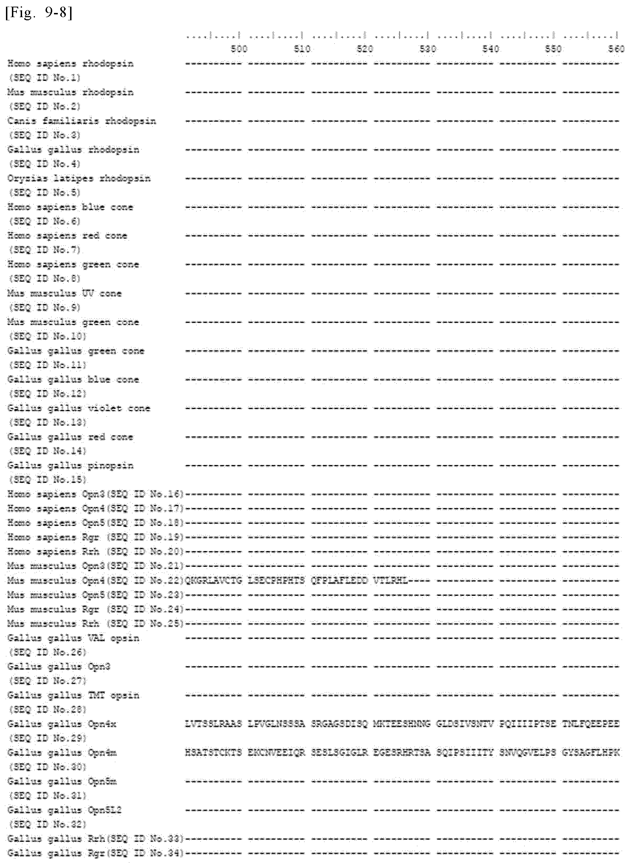

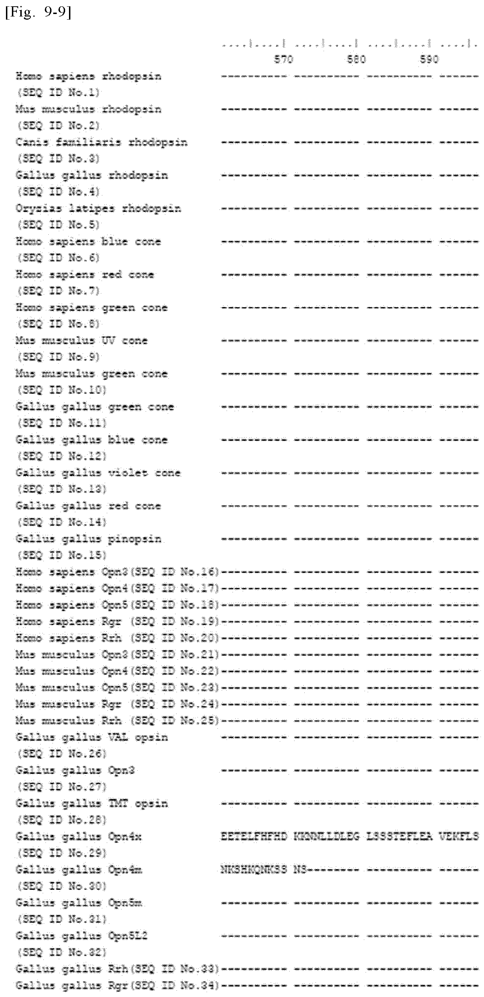

Specific amino acid sequences of opsins are as shown in SEQ ID NOs: 1 to 34, and 188th amino acids thereof are as shown in the alignment diagram in FIG. 9. That is, the 188th amino acid refers to one corresponding to G, the 188th amino acid in SEQ ID NO: 1, when aligned with SEQ ID NO: 1 using CLUSTRAL W (Version 2.0, released in 2007) (see the alignment diagram in FIG. 9), and the specific number may be shifted in another sequence ID number, for example, the 188th amino acid is G in SEQ ID NO: 2, G in SEQ ID NO: 3, G in SEQ ID NO: 4, G in SEQ ID NO: 5, G in SEQ ID NO: 6, G in SEQ ID NO: 7, G in SEQ ID NO: 8, G in SEQ ID NO: 9, G in SEQ ID NO: 10, G in SEQ ID NO: 11, G in SEQ ID NO: 12, G in SEQ ID NO: 13, G in SEQ ID NO: 14, G in SEQ ID NO: 15, T in SEQ ID NO: 16, S in SEQ ID NO: 17, T in SEQ ID NO: 18, T in SEQ ID NO: 19, T in SEQ ID NO: 20, T in SEQ ID NO: 21, S in SEQ ID NO: 22, T in SEQ ID NO: 23, T in SEQ ID NO: 24, T in SEQ ID NO: 25, E in SEQ ID NO: 26, S in SEQ ID NO: 27, S in SEQ ID NO: 28, T in SEQ ID NO: 29, S in SEQ ID NO: 30, T in SEQ ID NO: 31, T in SEQ ID NO: 32, T in SEQ ID NO: 33, and T in SEQ ID NO: 34, and those skilled in the art can determine the 188th amino acid even for a sequence ID number that is not listed herein by an alignment procedure. The same is applied to a 122nd amino acid. The 122nd amino acid refers to one corresponds to E, the 122nd amino acid in SEQ ID NO: 1, when aligned with SEQ ID NO: 1 using CLUSTRAL W (Version 2.0, released in 2007). Specifically, the 122nd amino acid is E in SEQ ID NO: 2, E in SEQ ID NO: 3, E in SEQ ID NO: 4, E in SEQ ID NO: 5, L in SEQ ID NO: 6, I in SEQ ID NO: 7, I in SEQ ID NO: 8, L in SEQ ID NO: 9, I in SEQ ID NO: 10, Q in SEQ ID NO: 11, M in SEQ ID NO: 12, L in SEQ ID NO: 13, I in SEQ ID NO: 14, I in SEQ ID NO: 15, I in SEQ ID NO: 16, I in SEQ ID NO: 17, C in SEQ ID NO: 18, L in SEQ ID NO: 19, M in SEQ ID NO: 20, F in SEQ ID NO: 21, I in SEQ ID NO: 22, C in SEQ ID NO: 23, L in SEQ ID NO: 24, M in SEQ ID NO: 25, I in SEQ ID NO: 26, I in SEQ ID NO: 27, I in SEQ ID NO: 28, I in SEQ ID NO: 29, I in SEQ ID NO: 30, C in SEQ ID NO: 31, V in SEQ ID NO: 32, M in SEQ ID NO: 33, and L in SEQ ID NO: 34, and those skilled in the art can determine the 122nd amino acid even for a sequence ID number that is not listed herein by an alignment procedure.

As used herein, the term "rhodopsin" refers to a protein with a pigment called retinal therein, which is activated by light and then transmits a visual signal to the brain. The rhodopsin herein may refer not only to having retinal therein but also opsin (protein portion), in which case it is interpreted interchangeably with the opsin. An ion-transporting receptor rhodopsin as represented by microbial-derived rhodopsin can be repeatedly activated by absorbing light because retinal is not released even upon light irradiation, but cannot activate a G protein unlike a G protein-coupled receptor rhodopsin as represented by an animal-derived rhodopsin.

A protein of the present disclosure may include any opsin and may also include a chimeric opsin, as long as the purpose of the present disclosure can be achieved. The chimeric opsin can be produced by combining portions of two or more opsins known in the art. The opsins to be combined may be derived from organisms that are the same as or different from each other. Those skilled in the art can select appropriate opsins in accordance with the intended use and/or function of the resulting opsin as appropriate to provide an appropriate chimeric opsin, and if an amino acid corresponding to an amino acid 188 of SEQ ID NO: 1 is modified, preferably to a cysteine, then the chimeric opsin can be used in the present disclosure. The present disclosure also encompasses such any chimeric opsin in which an amino acid corresponding to amino acid 188 of SEQ ID NO: 1 is modified, preferably to cysteine in the scope of the present disclosure. Opsins that can constitute the chimeric opsin may be, for example, rod opsin and cone opsin (e.g., blue opsin, green opsin, red opsin), melanopsin, encephalopsin, OPN5, RGR, and peropsin, may be microbial opsin (Type I opsin) or animal opsin (Type II opsin), and may be selected from vertebrate non-visual opsin, invertebrate opsin, etc.

Examples of the chimeric opsin that may be used in the present disclosure include a chimeric opsin of a G protein-coupled receptor and animal opsin such as a chimeric protein including at least a portion of mammalian opsin and at least a portion of an activated Gs- or Gq-type G protein-coupled receptor. To illustrate a typical example, a Gs- or Gq-activity can be obtained by fusing a portion of mammalian opsin with a portion of an activated Gs- or Gq-type G protein-coupled receptor. In one embodiment, the chimeric opsin of the present disclosure is one in which an amino acid sequence of an intracellular second loop and/or an intracellular third loop that binds to a G protein in an amino acid sequence of one opsin is replaced by an amino acid sequence of an intracellular second loop and/or an intracellular third loop of another G protein-coupled receptor, or a functional sequence. This allows the protein of the present disclosure to activate a Gi-type G protein in a light-dependent manner to decrease an intracellular cAMP level, activate a Gs-type G protein in a light-dependent manner to increase an intracellular cAMP level, or activate a Gq-type G protein to increase an intracellular Ca²⁺ level. Without wishing to be bound by any theory, since the chimeric protein to be utilized in this embodiment of the present disclosure can be expressed with a sufficient activity in a mammal such as a rodent and a primate, a preventive or progression inhibitory effect on a retinal disease, disorder or symptom, in particular, prevention or progression inhibition of retinitis pigmentosa can be achieved; or a visual cognitive-behavioral function (e.g., improvement of a light/dark judgment function, a light-avoidance function, and/or a crisis avoidance function) is improved; or enhancement of a visual function such as improved visual acuity is exerted.

Examples of a G protein-coupled receptor rhodopsin to be used in the present disclosure include human rhodopsin, bovine rhodopsin, and canine rhodopsin.

As used herein, the term "retinal" refers to a substance that is a kind of vitamin A1, also called retinaldehyde or retinene, and is a component of rhodopsin, a visual pigment included in a rod visual cell of the retina. Retinal which is bound to opsin and forms rhodopsin takes a molecular configuration of 11-cis-retinal, which is isomerized to all-trans-retinal upon exposure to light, thereby unbinding from opsin. The all-trans-retinal then returns to the 11-cis-retinal in a visual cycle and binds to opsin. The all-trans-retinal is phototoxic and has been suggested to be implicated in a variety of retinal and other vision-related diseases, including age-related macular degeneration, Stargardt's disease, fundus flavimaculatus, and recessively inherited retinitis pigmentosa.

As used herein, the phrase an "analogue of retinal (retinal analogue)" refers to a compound having substantially the same properties as those of retinal, including a naturally occurring native form or those having a different modification, addition, substitution of a functional group, etc. from those of the native form. Examples of the analogue of retinal include a natural analogue such as A2-retinal (3,4-dehydro retinal), A3-retinal (3-hydroxy retinal), A4-retinal (4-hydroxy retinal), and an artificial analogue such as 9-ethyl-retinal, 9-propyl-retinal. For the retinal analog, see, for example, Akimori Wada, YAKUGAKU ZASSHI 141 (4), 557-577, 2021-04-01.

As used herein, the phrase "visual disorder" refers to any disease, disorder, or symptom related to vision, for example, in the retina and may include a retinal degenerative disease (e.g., retinitis pigmentosa, age-related macular degeneration), retinopathy (e.g., diabetic retinopathy, proliferative retinopathy, simple retinopathy), floater, retinal tear, retinal detachment (e.g., rhegmatogenous retinal detachment, non-rhegmatogenous retinal detachment), as well as retinitis pigmentosa, age-related macular degeneration, myopic maculopathy, macular dystrophy, diabetic retinopathy, retinal detachment, etc. The disorder or symptom may also include disorders in vision, contrast sensitivity, light/dark adaptation, color vision, etc., and symptoms related thereto.

The terms "protein", "polypeptide", "oligopeptide", and "peptide" are used in the same meaning herein and refer to a polymer of amino acids having any length. The polymer may be linear, branched, or cyclic. The amino acid may be a naturally occurring, non-naturally occurring, or modified amino acid. The terms can also encompass a complex assembled from multiple polypeptide chains. The terms also encompass a polymer of naturally occurring or artificially modified amino acids. Examples of such a modification include, for example, formation of a disulfide bond, glycosylation, lipidation, acetylation, phosphorylation, or any other manipulation or modification (e.g., conjugation with a labeled component). This definition also encompasses, for example, a polypeptide containing one or two or more amino acid analogs (including, for example, non-naturally occurring amino acids), a peptide-like compound (e.g., peptoid), and other modifications known in the art. As used herein, the phrase "amino acid" is a generic term for organic compounds each having an amino group and a carboxyl group. When an antibody according to an embodiment of the present disclosure includes a "specific amino acid sequence", any of amino acids in the amino acid sequence may be chemically modified. Any of the amino acids in the amino acid sequence may also form a salt or a solvate. In addition, any of the amino acids in the amino acid sequence may be in a L-form or a D-form. Even in these cases, a protein according to the embodiment of the present disclosure can be said to include the above "specific amino acid sequence". Examples of known chemical modifications that an amino acid included in a protein undergoes in vivo include an N-terminal modification (e.g., acetylation, myristoylation, etc.), a C-terminal modification (e.g., amidation, glycosylphosphatidylinositol addition, etc.), or a side chain modification (e.g., phosphorylation, glycosylation, etc.). The amino acid may or may not be naturally occurring as long as it achieves the purpose of the present disclosure.

As used herein, the term "chimera" (e.g., protein, opsin, etc.) is a state in which a plurality of genetic information derived from the same or different organisms is mixed in the same entity (in this case, protein, etc.). A chimeric protein includes, for example, a mixture of gene sequences derived from two or three or more organisms. Sequence information included in the chimeric protein may include another sequence other than those derived from the organisms to be mixed.

As used herein, the terms "polynucleotide", "oligonucleotide", "nucleic acid", and "nucleic acid molecule" are used in the same meaning herein and refer to a polymer of nucleotides having any length. These terms also include an "oligonucleotide derivative" or a "polynucleotide derivative". The phrases "oligonucleotide derivative" and "polynucleotide derivative" refer to an oligonucleotide or polynucleotide that includes a nucleotide derivative or has an unusual bond between nucleotides and are used interchangeably. Specific examples of such an oligonucleotide include a 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to an N3'-P5' phosphoramidate bond, an oligonucleotide derivative in which a ribose-phosphodiester bond in an oligonucleotide is converted to a peptide-nucleic acid bond, an oligonucleotide derivative in which a uracil in an oligonucleotide is replaced by a C-5 propynyl uracil, an oligonucleotide derivative in which a uracil in an oligonucleotide is replaced by a C-5 thiazole uracil, an oligonucleotide derivative in which a cytosine in an oligonucleotide is replaced by a C-5 propynyl cytosine, an oligonucleotide derivative in which a cytosine in an oligonucleotide is replaced by a phenoxazine-modified cytosine, an oligonucleotide derivative in which a ribose in DNA is replaced by a 2'-O-propylribose, and an oligonucleotide derivative in which a ribose in an oligonucleotide is replaced by a 2'-methoxyethoxyribose. Unless otherwise indicated, it is contemplated that a specific nucleotide sequence also encompasses an explicit sequence as well as its conservatively modified modification (e.g., degenerate codon substitution) and complementary sequences thereof. Note that, a sequence of a nucleic acid is also referred to as a base sequence, a nucleic acid sequence, a nucleotide sequence, etc., but all have the same meaning. Specifically, the degenerate codon substitution can be achieved by producing a sequence in which the third positions of one or more selected (or all) codons are replaced by mixed bases and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). Depending on the context, the term "nucleic acid" herein is used interchangeably with the terms gene, DNA such as cDNA, RNA such as mRNA, oligonucleotide, and polynucleotide. As used herein, the term "nucleotide" may refer to a naturally occurring nucleotide or a non-naturally occurring nucleotide. As used herein, the nucleic acid may be DNA or RNA.

As used herein, the term "gene" refers to an agent that determines a genetic trait, and the "gene" may also refer to "polynucleotide", "oligonucleotide", and "nucleic acid".

As used herein, the phrases "nucleic acid construct", "construct", and "gene construct" are used interchangeably and refer to a nucleic acid molecule containing a nucleic acid isolated from a naturally occurring gene or combined and juxtaposed in a non-naturally occurring manner, and a vector.

As used herein, the term "homology" of a gene refers to a degree of identity of two or more gene sequences with respect to each other, and the term "homologous" generally means a high degree of identity or similarity. The term "identity" refers to a degree of correspondence of a sequence in terms of identical amino acids, and the term "similarity" refers to a degree of correspondence of a sequence in terms of identical amino acids and amino acids having a similar property. Thus, the higher the homology between two genes, the higher the identity or the similarity between their sequences. Whether two genes are homologous or not can be determined by a direct comparison of their sequences or, in the case of nucleic acids, by a hybridization method under a stringent condition. When sequences of two genes are directly compared, if DNA sequences are representatively at least 50% identical, preferably at least 70% identical, more preferably at least 80%, 90%, 95%, 96%, 97%, 98% or 99% identical between the sequences, then the two genes are homologous. Thus, as used herein, the term "homologue" or "homologous gene product" means a protein in another species, preferably a mammal, that exhibits the same biological function as a protein component of a complex described further herein. It is understood that such a homologue also encompasses an "orthologous gene product" and a "paralogous gene product" and that such a homologue, homologous gene product, orthologous gene product, paralogous gene product, etc. may also be used as long as they are consistent with the purpose of the present disclosure.

An amino acid may be referred to herein either by its commonly known three-letter code or by one-letter code recommended by the IUPAC-IUB Biochemical Nomenclature Commission. Similarly, a nucleotide can also be referred to by a commonly recognized one-letter code. Here, a comparison of similarity, identity, or homology between amino acid sequences or base sequences is calculated using BLAST, a tool for sequence analysis, with default parameters. The identity can be searched, for example, using BLAST 2.2.28 by the NCBI (published April 2, 2013) (Proc. Natl. Acad. Sci. USA 90:5873-5877, 1993). A value of identity herein usually refers to a value determined when aligned using the BLAST under default conditions. However, if a change in parameters results in a higher value, the highest value shall be the value of identity. When the identity is evaluated in a plurality of regions, the highest value shall be the value of identity. Similarity refers to a numerical value calculated taking similar amino acids into account in addition to identity. When amino acid sequences are compared by BLAST, Blastp can be used as an algorithm with a default setting. Measurement results are converted into numerical values as Positives or Identities. Homology between amino acid sequences or base sequences can be determined by the algorithm BLAST by Karlin and Altschul. Based on this algorithm, programs called BLASTN and BLASTX have been developed (Altschul et al. J. Mol. Biol. 215: 403-410, 1990). If a base sequence is analyzed by BLASTN based on BLAST, parameters are, for example, set as follows: score = 100 and word length = 12. If an amino acid sequence is analyzed by BLASTX based on BLAST, parameters are, for example, set as follows: score = 50 and word length = 3. When BLAST and Gapped BLAST programs are used, default parameters for each program are used. Specific procedures for these analyses have been well known (http://www.ncbi.nlm.nih.gov.).

A nucleic acid or a protein to be used in the present disclosure can include a sequence in which one or a plurality of amino acids or nucleotides are substituted, deleted, and/or added in an amino acid or base sequence of interest. As used herein, the phrase "one or a plurality of" in the context of a full-length amino acid sequence of a chimeric protein usually means within 50 amino acids, preferably within 30 amino acids, and further preferably within 10 amino acids (e.g., within 5 amino acids, within 3 amino acids, 1 amino acid). In the context of an amino acid sequence of a domain, "one or a plurality of" usually means within 6 amino acids, preferably within 5 amino acids, and further preferably within 4 amino acids (e.g., within 3 amino acids, within 2 amino acids, or 1 amino acid). If a biological activity of a chimeric protein of the present disclosure is to be retained, an amino acid residue is desirably mutated to another amino acid that preserves a property of a side chain of the amino acid residue. For the property of the side chain of the amino acid residue, for example, a hydrophobic amino acid (A, I, L, M, F, P, W, Y, V), a hydrophilic amino acid (R, D, N, C, E, Q, G, H, K, S, T), an amino acid having an aliphatic side chain (G, A, V, L, I, P), an amino acid having a hydroxy group-containing side chain (S, T, Y), an amino acid having a sulfur atom-containing side chain (C, M), an amino acid having carboxylic acid and amide-containing side chains (D, N, E, Q), an amino acid having a base-containing side chain (R, K, H), and an amino acid having an aromatic-containing side chain (H, F, Y, W) (all in parentheses represent one letter codes of amino acids) may be exemplified. These are also referred to herein as "conservative substitution". Note that, it has been known that a protein having an amino acid sequence in which one or a plurality of amino acid residues are modified by deletion, addition, and/or substitution with another amino acid residue preserves its biological activity (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). Therefore, in one embodiment of the present disclosure, the term "several" may refer to, for example, 10, 8, 6, 5, 4, 3, or 2, or may also refer to a value equal to or less than any of these values. A chimeric protein with a deletion, etc. can be produced by, for example, site-directed mutagenesis, random mutagenesis, or biopanning using an antibody phage library. For the site-specific mutagenesis, for example, the KOD-Plus-Mutagenesis Kit (TOYOBO CO., LTD.) can be used. An antibody with an equivalent activity to that of a wild type can be selected from mutant antibodies with a deletion, etc. by performing various characterizations such as a FACS analysis and ELISA.

In one embodiment of the present disclosure, an amino acid sequence and a nucleic acid sequence of a modified protein and/or chimeric protein of the present disclosure may be 70% or more, 80% or more, or 90% or more identical or similar to the reference sequence. In the context of an amino acid sequence or a base sequence herein, the phrase "70% or more" may refer to, for example, 70, 75, 80, 85, 90, 95, 96, 97, 98, 99% or more, the phrase "80% or more" may refer to, for example, 80, 85, 90, 95, 96, 97, 98, 99% or more, and the phrase "90 % or more" may refer to, for example, 90, 95, 96, 97, 98, 99% or more, or these phrases may also refer to be within a range of any two of the above-described values. "Homology" may be calculated as a percentage in the number of homologous amino acids between two or a plurality of amino acid sequences according to a method known in the art. Before calculating the percentage, amino acid sequences in a group of amino acid sequences to be compared are aligned, and a gap is introduced in a portion of the amino acid sequences if necessary to maximize a percentage of identical amino acids. Methods for alignment, calculation of the percentage, and comparison, and computer programs related thereto are conventionally well-known in the art (e.g., BLAST, GENETYX, etc.). For "identity", a percentage of identical amino acids is calculated and for "similarity", a percentage of similar amino acids is calculated. A similar amino acid may include, but is not limited to, an amino acid for which conservative substitution is possible.

As used herein, the phrase "polynucleotide hybridizing under a stringent condition" refers to a well-known condition customarily used in the art. Such a polynucleotide can be obtained by using a colony hybridization method, a plaque hybridization method, or a southern blot hybridization method using a polynucleotide selected from the polynucleotides of the present disclosure as a probe. Specifically, the phrase means a polynucleotide that can be identified by hybridization at 65°C in the presence of 0.7 to 1.0 M NaCl using a filter on which a colony- or plaque-derived DNA is immobilized, followed by washing the filter at 65°C using a 0.1 to 2 x SSC (saline-sodium citrate) solution (a composition of a 1 x SSC solution is 150 mM sodium chloride and 15 mM sodium citrate). For example, the following conditions can be employed as the "stringent condition": (1) use of low ionic strength and a high temperature for washing (e.g., 0.015 M sodium chloride/0.0015 M sodium citrate/0.1% sodium dodecyl sulfate at 50°C), (2) use of a denaturing agent such as formamide during hybridization (e.g., 50% (v/v) formamide, 0.1% bovine serum albumin/0.1% Ficoll/0.1% polyvinyl pyrrolidone/50 mM sodium phosphate buffer at pH 6.5, and 750 mM sodium chloride, 75 mM sodium citrate at 42°C), or (3) incubation in a solution containing 20% formamide, 5 x SSC, 50 mM sodium phosphate (pH 7.6), 5 x Denhard's solution, 10% dextran sulfate, and 20 mg/mL denatured, sheared salmon sperm DNA overnight at 37°C, followed by washing the filter with 1 x SSC at about 37 to 50°C. Note that, the formamide may have a concentration of 50% or higher. A time for the washing may be 5, 15, 30, 60, or 120 minutes or longer. A plurality of factors is believed to affect stringency of a hybridization reaction, such as temperature, salt concentration, etc. For details, see Ausubel et al., Current Protocols in Molecular Biology, Wiley Interscience Publishers, (1995). A "highly stringent condition" is, for example, 0.0015 M sodium chloride and 0.0015 M sodium citrate at 65 to 68°C, or 0.015 M sodium chloride, 0.0015 M sodium citrate, and 50% formamide at 42°C. Hybridization can be performed according to the procedures described in experimental books such as Molecular Cloning 2nd ed., Current Protocols in Molecular Biology, Supplement 1-38, DNA Cloning 1: Core Techniques, A Practical Approach, Second Edition, Oxford University Press(1995). Here, a sequence containing only a A sequence or only a T sequence is preferably excluded from a sequence that hybridizes under the stringent condition. A moderately stringent condition can be readily determined by those skilled in the art based on, for example, a length of DNA, is described in Sambrook et al., Molecular Cloning: A Laboratory Manual, Number 3, Vol. 1, 7.42-7.45 Cold Spring Harbor Laboratory Press,2001, and includes, in the case of using a nitrocellulose filter, use of a prewashing solution of 5 x SSC, 0.5% SDS, and 1.0 mM EDTA (pH 8.0), a hybridization condition of about 50% formamide and 2 x SSC to 6 x SSC at about 40 to 50°C (or another similar hybridization solution such as a Stark's solution in about 50% formamide at about 42°C), and a wash condition of 0.5 x SSC and 0.1% SDS at about 60°C. Thus, a polypeptide to be used in the present disclosure also encompasses a polypeptide encoded by a nucleic acid molecule that hybridizes under the highly or moderately stringent condition to a nucleic acid molecule encoding a polypeptide particularly described in the present disclosure.

As used herein, a "purified" substance or biological agent (e.g., a nucleic acid or a protein) refers to one from which at least a portion of an agent naturally associated with the substance or the biological agent has been removed. Thus, in the purified biological agent, the biological agent usually has a higher purity (i.e., enriched) than that in a state in which the biological agent is naturally present. As used herein, the term "purified" means that preferably at least 75% by weight, more preferably at least 85% by weight, further more preferably at least 95% by weight, and most preferably at least 98% by weight of the same type of biological agent is present. A substance or a biological agent to be used in the present disclosure is preferably a "purified" substance. As used herein, an "isolated" substance or biological agent (e.g., a nucleic acid or a protein) is one from which an agent naturally associated with the substance or the biological agent has been substantially removed. As used herein, the term "isolated" needs not necessarily to be expressed in terms of purity since this varies depending on the purpose, but, if necessary, means that preferably at least 75% by weight, more preferably at least 85% by weight, further more preferably at least 95% by weight, and most preferably at least 98% by weight of the same type of biological agent is present. A substance to be used in the present disclosure is preferably an "isolated" substance or biological agent.

As used herein, a "corresponding" amino acid or nucleic acid or portion refers to an amino acid or nucleotide that has or is expected to have an equivalent effect in a polypeptide or polynucleotide molecule (e.g., rhodopsin) to that of a predetermined amino acid or nucleotide or portion in a reference polypeptide or polynucleotide to be compared, in particular, in the case of an enzyme molecule, an amino acid that is present at a similar position in an active site and equivalently contributes to a catalytic activity, and in the case of a complex molecule, a corresponding portion (e.g., heparan sulfate, etc.). For example, in the case of an antisense molecule, it could be a similar portion in an ortholog corresponding to a specific portion of the antisense molecule. A corresponding amino acid may be a specific amino acid that undergoes, for example, cysteinylation, glutathionylation, formation of an S-S bond, oxidation (e.g., oxidation of a methionine side chain), formylation, acetylation, phosphorylation, glycosylation, myristylation, etc. Alternatively, the corresponding amino acid can be an amino acid responsible for dimerization. Such a "corresponding" amino acid or nucleic acid may be a region or domain spanning a certain range. Thus, such a region or domain is referred to herein as a "corresponding" region or domain. Such a corresponding region or domain is useful when designing a complex molecule in the present disclosure.

As used herein, a "corresponding" gene (in this case, may be a polynucleotide sequence or molecule encoding opsin, etc.) refers to a gene (in this case, may be a polynucleotide sequence or molecule encoding opsin, etc.) that, in one species, has or is predicted to have an equivalent effect to that of a predetermined gene in a reference species to be compared, and if there are a plurality of genes with such an effect, refers to one having the same evolutionary origin. Therefore, a gene corresponding to a gene may be an ortholog of that gene. Thus, for each human opsin, a corresponding opsin can be found in another animal (especially a mammal). Such a corresponding gene can be identified using a technique well-known in the art. Thus, for example, a corresponding gene in an animal (e.g., mouse) can be found by searching a database containing sequences for the animal using as a query sequence a specific sequence in a reference gene for the corresponding gene (e.g., opsin).

As used herein, the term "portion," "fragment" or "a part" refers to a polypeptide or polynucleotide having a sequence length of 1 to n-1 relative to a full-length polypeptide or polynucleotide (with a length of n). A length of the fragment can be changed as appropriate for the purpose, for example, a lower limit of the length is 3, 4, 5, 6, 7, 8, 9, 10, 15, 20, 25, 30, 40, 50, and more amino acids in the case of a polypeptide and a length expressed by an integer that is not specifically listed (e.g., 11) may also be appropriate as the lower limit. In the case of a polynucleotide, the lower limit of the length may be 5, 6, 7, 8, 9, 10, 15, 20, 25, 30, 40, 50, 75, 100, and more nucleotides, and a length expressed by an integer that is not specifically listed (e.g., 11) may also be appropriate as the lower limit. It is understood herein that if a full-length polypeptide or polynucleotide functions as a marker or a target molecule, a fragment thereof falls within the scope of the present disclosure as long as the fragment itself also functions as the marker or the target molecule.

In accordance with the present disclosure, the term "activity" refers to a function of a molecule in its broadest sense. Without intended being as a limitation, the activity generally includes a biological, biochemical, physical, or chemical function of the molecule. The activity includes, for example, an enzymatic activity, an ability to interact with another molecule, and an ability to activate, promote, stabilize, inhibit, suppress, or destabilize a function of another molecule, stability, and an ability to localize to a specific subcellular location. When applicable, this term also relates to a function of a protein complex in its broadest sense. As used herein, the phrase "biological activity" includes, for example, activation of a light reaction.

As used herein, the phrase "functional equivalent" refers to any entity that has the same intended function as but a different structure from, that of an original entity of interest. Thus, it is understood that a functional equivalent of "opsin" or a chimera thereof is not opsin or a chimera thereof itself, but encompasses a mutant or modification (e.g., amino acid sequence modification, etc.) of the opsin or the chimera thereof that has a biological effect of the opsin or the chimera thereof, and one can be transformed into the opsin or an antibody thereof itself or into the mutant or modification of the opsin or the chimera thereof at the time of exerting the effect (e.g., a nucleic acid encoding the opsin or the chimera thereof or the mutant or modification of the opsin or the chimera thereof, and a vector or a cell containing such a nucleic acid, etc.). A functional equivalent of the present disclosure may be an amino acid sequence in which one or a plurality of amino acids are inserted, substituted, and/or deleted, or added to one or both ends thereof. As used herein, the phrase "amino acid sequence in which one or a plurality of amino acids are inserted, substituted and/or deleted, or added to one or both ends thereof" means that an amino acid sequence in which a plurality of amino acids has been modified, for example, substituted by a well-known technical method such as site-directed mutagenesis or by natural mutation to the extent that it may occur naturally. A modified amino acid sequence may be, for example, one in which 1 to 30, preferably 1 to 20, more preferably 1 to 9, furthermore preferably 1 to 5, and particularly preferably 1 to 2 amino acids are inserted, substituted, or deleted, or added to one or both ends thereof. The modified amino acid sequence may also preferably be an amino acid sequence having one or a plurality of (preferably one or several, or one, two, three, or four) conservative substitutions in an amino acid sequence of an opsin.

As used herein the terms "agent", "agent", and "factor" (all equivalent to "agent" in English) are used interchangeably in a broad sense and may be any substance or another element (e.g., an energy such as light, radioactivity, heat, electricity) as long as the intended purpose can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA and genomic DNA, RNA such as mRNA), a polysaccharide, an oligosaccharide, a lipid, an organic small molecule (e.g., a hormone, a ligand, a transmitter, an organic small molecule, a molecule synthesized in combinatorial chemistry, a small molecule that can be used as a medicament (e.g., a small molecule ligand), and a complex molecule thereof.

For oral administration, the agent may be formulated in various dosage forms such as a tablet, a granule, a subtle granule, a powder, a capsule, etc., and may contain an additive commonly used in the formulation such as a binding agent, an inclusion agent, an excipient, a lubricant, a disintegrant, and a wetting agent. In addition thereto, in the case of oral administration, the agent may be formulated as a liquid state, such as a solution for internal use, suspension, emulsion, or syrup, etc., or as a dry state that is redissolved for use.

For parenteral administration, the agent may be formulated into a formulation contained in a unit-dose ampule or a multi-dose container, or a tube, and may also contain an additive such as a stabilizer, a buffer, a preservative, a tonicity agent, etc. In the case of parenteral administration, the agent may also be formulated into a powder that can be redissolved in a suitable carrier (such as sterile water) for use.

Examples of the parenteral administration include intraocular administration such as intravitreal, subretinal, subchoroidal, and intra-anterior chamber administration, as well as extraocular administration such as subconjunctival, sub-Tenon's capsule, and ophthalmic administration, with the intravitreal administration being preferred. A composition of the present disclosure can be used for treatment, prevention, and inhibition of progression by administering to human as described above.

As used herein, the term "treatment" refers to prevention of aggravation, preferably maintenance of the status quo, more preferably alleviation, and further preferably elimination of a disease or disorder (e.g., vesicular transport disorder or apoptosis) when the disease or disorder is developed, and includes exertion of a symptomatic ameliorative or preventive effect on a disease or one or more symptoms associated with the disease in a patient. Prior diagnosis for appropriate treatment is called a "companion therapy" and a diagnostic agent therefor is sometimes called a "companion diagnostic drug". The present disclosure targets a genetic disorder, so a patient may be pre-tested for a gene and then treated.

As used herein, the phrase "therapeutic drug (agent)" refers to any agent capable of treating a condition of interest (e.g., retinal degenerative disease) in the broad sense. In one embodiment of the present disclosure, the "therapeutic drug" may be a pharmaceutical composition containing an active ingredient and one or more pharmacologically acceptable carriers. The pharmaceutical composition can be produced, for example, by mixing the active ingredient with the above carriers and using any method known in the art of formulation. A usage form of the therapeutic drug is not limited as long as it is used for treatment, and may be the active ingredient alone or a mixture of the active ingredient and any component. A form of the above carriers is not limited and may be, for example, in a solid or liquid form (e.g., buffer solution).

As used herein, the term "prevention" refers to prevention of development of a disease or disorder (e.g., retinal degenerative disease) before the disease or disorder is developed. The agent of the present disclosure can be used to diagnose and, if necessary, prevent or take measures to prevent, for example, a retinal degenerative disease. As used herein, the term "prophylactic drug (agent)" refers to any agent capable of preventing a condition of interest (e.g., vesicular transport disorder, apoptosis, etc.) in the broad sense.

As used herein, the term "kit" refers to a unit in which parts to be provided (e.g., a nucleic acid, a nucleic acid construct, a cell that has been transfected with a nucleic acid of interest, a test drug, a diagnostic drug, a therapeutic drug, an antibody, a label, an instruction, etc.) are provided usually in two or more compartments. A combination of a diagnostic drug and a therapeutic agent may be provided as a kit when it is preferable to administer a specific medicament (e.g., nucleic acid medicament) to only an appropriate patient after a patient to be administered is identified using a reagent to determine a property of a patient, for example, in the case of a companion diagnostic agent. Alternatively, such a kit is preferred for the purpose of providing a composition that should not be provided in a mixture for a stability reason, for example, in the case of a specific unstable medicament, and is preferably mixed immediately prior to use. It is advantageous that such a kit preferably includes a direction or instruction that describes how to use the parts to be provided (e.g., a nucleic acid, a nucleic acid construct, a cell that has been transfected with a nucleic acid of interest, a test drug, a diagnostic agent, a therapeutic agent) or how a reagent should be processed. When a kit is used herein as a reagent kit, the kit typically includes an instruction describing how to use a test drug, a diagnostic drug, a therapeutic drug, an antibody, etc.

As used herein, the phrase "active ingredient" refers to an ingredient contained in an amount necessary to achieve a therapeutic, prophylactic, or progression-inhibiting effect that a composition of the present disclosure targets and another ingredient may also be included as long as the effect is not impaired to less than a desired level. A medicament or composition of the present disclosure may also be formulated. A route of administration for the medicament or composition of the present disclosure may be either oral or parenteral, and may be set as appropriate depending on a form of formulation.

As used herein, the term "instruction" (including an attachment and a label utilized by the U.S. FDA) is an instruction to a physician or another user on how to use the present disclosure. The instruction includes wording that instructs a detection method according to the present disclosure, how to use a diagnostic drug, or administration of a medicament, etc. The instruction may also include wording that instructs oral or retinal administration (e.g., by injection) with respect to a site of administration. This instruction is prepared in accordance with the format prescribed by the regulatory authority of the country where the present disclosure is to be implemented (e.g., Ministry of Health, Labour and Welfare in Japan, Food and Drug Administration (FDA) in the United States, etc.) and clearly states that it has been approved by that regulatory authority. The instruction is a so-called package insert or label, which is usually provided in paper form, but is not limited thereto, and can also be provided in an electronic form (e.g., a website provided on the Internet, e-mail).

### (Preferred Embodiment)

Preferred embodiments of the present disclosure will be described. It is understood that the embodiments provided below are provided for a better understanding of the present disclosure and the scope of the present disclosure should not be limited to the following description. Therefore, it is clear that those skilled in the art may make a modification as appropriate within the scope of the present disclosure, taking into account the description herein. It is understood that the following embodiments of the present disclosure may also be used alone or in combination.

In one aspect, the present disclosure provides a protein including an amino acid sequence of an opsin, the protein including a modification at an amino acid corresponding to position 188 when aligned with SEQ ID NO: 1 in the amino acid sequence of the opsin.

Opsin is a photosensitive G protein-coupled receptor that is ubiquitous in animals. All opsins have a common structural element including seven-transmembrane domains and bind to a light-absorbing chromophore retinal via a Schiff base bond to Lys296 (based on the bovine rhodopsin numbering system) of the opsin. The opsin functions in both visual and non-visual photoreception and is classified into several groups based on an amino acid sequence (Shichida and Matsuyama, 2009; Koyanagi and Terakita, 2014). Bovine rhodopsin is the most studied opsin (Yau and Hardie, 2009), functions as a visual photoreceptive protein in the retina, and is bound to 11-cis retinal in the dark. Photoisomerization of retinal to an all-trans form produces a Meta II intermediate of rhodopsin, which binds to a G protein. Meta II is in a metastable active state and is spontaneously converted to Meta III (Heck et al., 2003). When the Meta II is irradiated with light, formation of Meta III is induced instead of an original dark state (Bartl et al., 2001; Ritter et al., 2008). In other words, the Meta II in an active state very inefficiently returns to its original dark state by a light reaction or a thermal reaction. These indicate that vertebrate opsin is specialized for photoactivation and is characterized as monostable opsin. On the other hand, molluscan and arthropod opsins form an acid meta-state, a stable active state, by photoisomerization from 11-cis to all-trans retinal, and the active state can return to an original dark state containing 11-cis retinal by a light reaction (Koyanagi and Terakita, 2014; Yau and Hardie, 2009). In other words, these opsins are known as bistable opsins since they have two stable states, a dark state and an active state, that can be interconverted by light. Recent accumulation of knowledge on a molecular property of an opsin has revealed that many members in various opsin groups are bistable opsins, suggesting that vertebrate opsins evolved as monostable opsins from their ancestral bistable opsins (Shichida and Matsuyama, 2009).

The only opsin in animals with a photocycle property (Opn5L1) has a cysteine residue at position 188, which is known to underlie a photocycle reaction of an opsin (Sato et al., 2018). The Opn5L1 binds to all-trans retinal rather than 11-cis retinal in the dark to form an active state. Light irradiation causes photoisomerization of retinal into an 11-cis form to thereby suppress a G-protein activating ability of the Opn5L1. A covalent bond is then formed between retinal and Cys188 of an opsin to convert a C11=C12 double bond in the retinal to a single bond. Then, thermal rotation of the C11-C12 single bond in the retinal dissociates a Cys188-retinal adduct to regenerate an original dark state. Therefore, it can be said that the G-protein activating ability of the Opn5L1 is regulated by a combination of photoisomerization and thermal isomerization of the retinal and thus the Opn5L1 is the first animal opsin of which activity is regulated by a photocycle reaction.

A comparison of amino acid sequences among opsins shows that a cysteine residue at position 188 is well conserved in an Opn5L1 group but rarely found in other opsin groups, indicating that Cys188 is important for a photocycle reaction unique to Opn5L1. On the other hand, vertebrate and pyramidal opsins, which are monostable opsins, each has a glycine residue at this position (FIG. 9). In the present disclosure, it has been found that a mutation at position 188 can confer a photocycle property in bovine rhodopsin. The present disclosure has revealed that a G188C mutant converts to an active state, Meta II, upon light irradiation and thermally returns to its original dark state. The Meta II of the G188C mutant can also return to an original dark state upon light irradiation. Thus, the G188C mutant of bovine rhodopsin exhibits a photocycle property and photoreversibility, indicating that the residue at position 188 regulates recovery from the active state to the original dark state in vertebrate opsins.

In one embodiment of the present disclosure, an amino acid at position 188 when aligned with SEQ ID NO: 1 in an amino acid sequence of an opsin of the present disclosure may be an amino acid corresponding to G, T, S, or E. A modified opsin of the present disclosure is one in which any of these amino acids is modified.

One embodiment of the present disclosure can include modification to cysteine of an amino acid corresponding to position G188 when aligned with SEQ ID NO: 1. An amino acid corresponding to position G188 of an opsin to be used in the present disclosure may be an amino acid corresponding to G, T, S, or E, as described above, any of which can be modified to cysteine to obtain a photocycle property and photoreversibility. Thus, in one embodiment of the present disclosure, a protein of the present disclosure can be inactivated without releasing a photoreceptive factor after being activated by photostimulation.

In one embodiment, any opsin can be used as opsin to be used in the present disclosure, including but not limited to a G protein-coupled receptor rhodopsin, rod opsin, cone opsin (blue opsin, green opsin, red opsin, melanopsin. encephalopsin, OPN5 panopsin, RGR, and peropsin. In one embodiment, the opsin to be used in the present disclosure can include, but is not limited to, those derived from a vertebrate, an invertebrate, or a microorganism, or a chimera derived therefrom. Examples of the vertebrate may include, but are not limited to, a mammal, a bird, a reptile, an amphibia, a fish (teleost and elasmobranch), or an agnatha. Examples of the invertebrate may also include, but are not limited to, a mollusk, an arthropod, or a cnidaria. Examples of the microorganism may include, but are not limited to, an eubacterium, an archaeon, or a fungus. In one embodiment, a mammal-derived opsin may be used as opsin of the present disclosure. Examples of the G protein-coupled receptor rhodopsin may include, but are not limited to, one derived from a mammal including rhodopsin derived from a rodent, an artiodactyl, a perissodactyl, a primate, a carnivore, etc., for example, rhodopsin from an artiodactyl or a primate, with primate rhodopsin being advantageous. Opsin that may be used may be, for example, one derived from cattle, human, mouse, rat, cat, dog, pig, sheep, horse, etc. Among these, one derived from cattle or human is particularly preferred.

In one embodiment, specific amino acid sequences of an opsin of the present disclosure may include, but are not limited to, sequences of SEQ ID NOs: 1 to 34 listed in Table 1.

**[Table 1-1]**

| |
|---|
| >Homo sapiens rhodopsin NP_001372054.1 (SEQ ID NO.1) |
| * |
| >Mus musculus rhodopsin NP_663358.1 (SEQ ID NO.2) |
| MNGTEGPNFYVPFSNVTGVVRSPFEQPQYYLAEPWQFSMLAAYMFLLIVLGFPIN FLTLYVTVQHKKLRTPLNYILLNLAVADLFMVFGGFTTTLYTSLHGYFVFGPTGCN LEGFFATLGGEIALWSLVVLAIERYVVVCKPMSNFRFGENHAIMGVVFTWIMALA CAAPPLVGWSRYIPEGMQCSCGIDYYTLKPEVNNESFVIYMFVVHFTIPMIVIFFC YGQLVFTVKEAAAQQQESATTQKAEKEVTRMVIIMVIFFLICWLPYASVAFYIFTH QGSNFGPIFMTLPAFFAKSSSIYNPVIYIMLNKQFRNCMLTTLCCGKNPLGDDDAS ATASKTETSQVAPA* |
| >Canis familiaris rhodopsin CAA50502.1 (SEQ ID NO.3) |
| MNGTEGPNFYVPFSNKTGVVRSPFEYPQYYLAEPWQFSMLAAYMFLLIVLGFPIN FLTLYVTVQHKKLRTPLNYILLNLAVADLFMVFGGFTTTLYTSLHGYFVFGPTGCN VEGFFATLGGEIALWSLVVLAIERYVVVCKPMSNFRFGENHAIMGVAFTWVMALA CAAPPLAGWSRYIPEGMQCSCGIDYYTLKPEINNESFVIYMFVVHFAIPMIVIFFCY GQLVFTVKEAAAQQQESATTQKAEKEVTRMVIIMVIAFLICWVPYASVAFYIFTHQ GSDFGPIFMTLPAFFAKSSSIYNPVIYIMMNKQFRNCMITTLCCGKNPLGDDEASA SASKTETSQVAPA* |
| >Gallus gallus rhodopsin NP_001884426.1 (SEQ ID NO.4) |
| MNGTEGQDFYVPMSNKTGVVRSPFEYPQYYLAEPWKFSALAAYMFMLILLGFPV NFLTLYVTIQHKKLRTPLNYILLNLAVADLFMVFGGFTTTMYTSMNGYFVFGVTG CYIEGFFATLGGEIALWSLVVLAVERYVVVCKPMSNFRFGENHAIMGVAFSWIMA MACAAPPLFGWSRYIPEGMQCSCGIDYYTLKPEINNESFVIYMFVVHFMIPLAVIF FCYGNLVCTVKEAAAQQQESATTQKAEKEVTRMVIIMVIAFLICWVPYASVAFYIF TNQGSDFGPIFMTIPAFFAKSSAIYNPVIYIVMNKQFRNCMITTLCCGKNPLGDED TSAGKTETSSVSTSQVSPA* |
| >Oryzias latipes rhodopsin BAD99136.1 (SEQ ID NO.5) |
| MNGTEGPYFYVPMVNTTGIVRSPYEYPQYYLVNPAAYAALGAYMFFLILVGFPINF LTLYVTLEHKKLRTPLNYILLNLAVADLFMVFGGFTTTMYTSMHGYFVLGRLGCN LEGFFATLGGEIGLWSLVVLAIERWVVVCKPISNFRFGENHAIMGLVFTWIMAASC |

**[Table 1-2]**

| |
|---|
| AVPPLVGWSRYIPEGMQCSCGVDYYTRAEGFNNESFVVYMFVCHFLIPLIVVFFC YGRLLCAVKEAAAAQQESETTQRAEREVTRMVVIMVIGFLVCWLPYASVAWYIFT NQGSEFGPLFMTIPAFFAKSSSIYNPAIYICMNKQFRNCMITTLCCGKNPFEEEEG ASTTASKTEASSVSSSSVSPA* |
| >Homo sapiens blue cone opsin NP_001372054.1 (SEQ ID NO.6) |
| MRKMSEEEFYLFKNISSVGPWDGPQYHIAPVWAFYLQAAFMGTVFLIGFPLNAM VLVATLRYKKLRQPLNYILVNVSFGGFLLCIFSVFPVFVASCNGYFVFGRHVCALE GFLGTVAGLVTGWSLAFLAFERYIVICKPFGNFRFSSKHALTVVLATWTIGIGVSIP PFFGWSRFIPEGLQCSCGPDWYTVGTKYRSESYTWFLFIFCFIVPLSLICFSYTQLL RALKAVAAQQQESATTQKAEREVSRMVVVMVGSFCVCYVPYAAFAMYMVNNRN HGLDLRLVTIPSFFSKSACIYNPIIYCFMNKQFQACIMKMVCGKAMTDESDTCSSQ KTEVSTVSSTQVGPN* |
| >Homo sapiens red cone opsin NP_064445.2 (SEQ ID NO.7) |
| * |
| >Homo sapiens green cone opsin NP_000504.1 (SEQ ID NO.8) |
| MAQQWSLQRLAGRHPQDSYEDSTQSSIFTYTNSNSTRGPFEGPNYHIAPRWVYH LTSVWMIFVVIASVFTNGLVLAATMKFKKLRHPLNWILVNLAVADLAETVIASTISV VNQVYGYFVLGHPMCVLEGYTVSLCGITGLWSLAIISWERWMVVCKPFGNVRFD AKLAIVGIAFSWIWAAVWTAPPIFGWSRYWPHGLKTSCGPDVFSGSSYPGVQSYMI VLMVTCCITPLSIIVLCYLQVWLAIRAVAKQQKESESTQKAEKEVTRMVVVMVLAF CFCWGPYAFFACFAAANPGYPFHPLMAALPAFFAKSATIYNPVIYVFMNRQFRNCI LQLFGKKVDDGSELSSASKTEVSSVSSVSPA* |
| >Mus musculus UV cone opsin AAG17989.1 (SEQ ID NO.9) |
| MSGEDDFYLFQNISSVGPWDGPQYHLAPVWAFRLQAAFMGFVFFVGTPLNAIVL VATLHYKKLRQPLNYILVNVSLGGFLFCIFSVFTVFIASCHGYFLFGRHVCALEAF LGSVAGLVTGWSLAFLAFERYVVICKPFGSIRFNSKHALMVVLATWIIGIGVSIPPF FGWSRFIPEGLQCSCGPDWYTVGTKYRSEYYTWFLFIFCFIIPLSLICFSYSQLLRT LRAVAAQQQESATTQKAEREVSHMVVVMVGSFCLCYVPYAALAMYMVNNRNHG LDLRLVTIPAFFSKSSCVYNPIIYCFMNKQFRACILEMVCRKPMADESDVSGSQKT EVSTVSSSKVGPH* |

**[Table 1-3]**

| |
|---|
| >Mus musculus green cone opsin AAB64302.1 (SEQ ID NO.10) |
| MAQRLTGEQTLDHYEDSTHASIFTYTNSNSTKGPFEGPNYHIAPRWVYHLTSTW MILVVVASVFTNGLVLAATMRFKKLRHPLNWILVNLAVADLAETIIASTISVVNQIY GYFVLGHPLCVIEGYIVSLCGITGLWSLAIISWERWLVVCKPFGNVRFDAKLATVG IVFSWVWAAIWTAPPIFGWSRYWPYGLKTSCGPDVFSGTSYPGVQSYMMVLMVT CCIFPLSIIVLCYLQVWLAIRAVAKQQKESESTQKAEKEVTRMVVVMVFAYCLCWG PYTFFACFATAHPGYAFHPLVASLPSYFAKSATIYNPIIYVFMNRQFRNCILHLFGK KVDDSSELSSTSKTEVSSVSSVSPA* |
| >Gallus gallus green cone opsin AAA48786.1 (SEQ ID NO.11) |
| MNGTEGINFYVPMSNKTGVVRSPFEYPQYYLAEPWKYRLVCCYIFFLISTGLPINL LTLLVTFKHKKLRQPLNYILVNLAVADLFMACFGFTVTFYTAWNGYFVFGPVGCA VEGFFATLGGQVALWSLVVLAIERYIVVCKPMGNFRFSATHAMMGIAFTWVMAFS CAAPPLFGWSRYMPEGMQCSCGPDYYTHNPDYHNESYVLYMFVIHFIIPVVVIFF SYGRLICKVREAAAQQQESATTQKAEKEVTRMVILMVLGFMLAWTPYAVVAFWIF TNKGADFTATLMAVPAFFSKSSSLYNPIIYVLMNKQFRNCMITTICCGKNPFGDED VSSTVSQSKTEVSSVSSSQVSPA* |
| >Gallus gallus blue cone opsin AAA48633.1 (SEQ ID NO.12) |
| MHPPRPTTDLPEDFYIPMALDAPNITALSPFLVPQTHLGSPGLFRAMAAFMFLLIA LGVPINTLTIFCTARFRKLRSHLNYILVNLALANLLVILVGSTTACYSFSQMYFALG PTACKIEGFAATLGGMVSLWSLAVVAFERFLVICKPLGNFTFRGSHAVLGCVATWV LGFVASAPPLFGWSRYIPEGLQCSCGPDWYTTDNKWHNESYVLFLFTFCFGVPLA IIVFSYGRLLITLRAVARQQEQSATTQKADREVTKMVVVMVLGFLVCWAPYTAFAL WVVTHRGRSFEVGLASIPSVFSKSSTVYNPVIYVLMNKQFRSCMLKLLFCGRSPF GDDEDVSGSSQATQVSSVSSSHVAPA* |
| >Gallus gallus violet cone opsin AAA49141.1 (SEQ ID NO.13) |
| MSSDDDFYLFTNGSVPGPWDGPQYHIAPPWAFYLQTAFMGIVFAVGTPLNAVVLW VTVRYKRLRQPLNYILVNISASGFVSCVLSVFVVFVASARGYFVFGKRVCELEAFV GTHGGLVTGWSLAFLAFERYIVICKPFGNFRFSSRHALLVVVATWLIGVGVGLPPF FGWSRYMPEGLQCSCGPDWYTVGTKYRSEYYTWFLFIFCFIVPLSLIIFSYSQLLS ALRAVAAQQQESATTQKAEREVSRMVVVMVGSFCLCYVPYAALAMYMVNNRDH GLDLRLVTIPAFFSKSACVYNPIIYCFMNKQFRACIMETVCGKPLTDDSDASTSAQ RTEVSSVSSSQVGPT* |
| >Gallus gallus red cone opsin CAA40727.1 (SEQ ID NO.14) |
| MAAWEAAFAARRRHEEEDTTRDSVFTYTNSNNTRGPFEGPNYHIAPRWVYNLTS LWMIFVVAASVFTNGLVLVATWKFKKLRHPLNWILVNLAVADLGETVIASTISVIN QISGYFILGHPMCVVEGYTVSACGITALWSLAIISWERWFVVCKPFGNIKFDGKLA |

**[Table 1-4]**

| |
|---|
| VAGILFSWLWSCAWTAPPIFGWSRYWPHGLKTSCGPDVFSGSSDPGVQSYMVVL MVTCCFFPLAIIILCYLQVSLAIRAVAAQQKESESTQKAEKEVSRMVVVMIVAYCFC WGPYTFFACFAAANPGYAFHPLAAALPAYFAKSATIYNPIIYVFMNRQFRNCILQLF GKKVDDGSEVSTSRTEVSSVSNSSVSPA* |
| >Gallus gallus pinopsin AAA64223.1 (SEQ ID NO.15) |
| MSSNSSQAPPNGTPGPFDGPQWPYQAPQSTYVGVAVLMGTVVACASVVNGLVIVV SICYKKLRSPLNYILVNLAVADLLVTLCGSSVSLSNNINGFFVFGRRMCELEGFMV SLTGIVGLWSLAILALERYVVVCRPLGDFQFQRRHAVSGCAFTWGWALLWSTPPL LGWSSYVPEGLRTSCGPNWYTGGSNNNSYILSLFVTCFVLPLSLILFSYTNLLLTL RAAAAQQKEADTTQRAEREVTRMVIVMVMAFLLCWLPYSTFALVVATHKGIIQP VLASLPSYFSKTATVYNPIIYVFMNKQFQSCLLEMLCCGYQPQRTGKASPGTPGP HADVTAAGLRNKVMPAHPV* |
| >Homo sapiens Opn3 AAH36773.1 (SEQ ID NO.16) |
| MYSGNRSGGHGYWDGGGAAGAEGPAPAGTLSPAPLFSPGTYERLALLLGSIGLLG VGNNLLVLVLYYKFQRLRTPTHLLLVNISLSDLLVSLFGVTFTFVSCLRNGWVWDI VGCVWDGFSGSLFGIVSIATLTVLAYERYIRVVHARVINFSWAWRAITYIWLYSLAW AGAPLLGWNRYILDVHGLGCTVDWKSKDANDSSFVLFLFLGCLVVPLGVIAHCY GHILYSIRMLRCVEDLQTIQVIKILKYEKKLAKMCFLMIFTFLVCWMPYIVICFLVV NGHGHLVTPTISIVSYLFAKSNTVYNPVIYVFMIRKFRRSLLQLLCLRLLRCQRPAK DLPAAGSEMQIRPIVMSQKDGDRPKKKVTFNSSSIIFIITSDESLSVDDSDKTNGSK VDVIQVRPL* |
| >Homo sapiens Opn4 AAI13559.1 (SEQ ID NO.17) |
| MNPPSGPRVLPSPTQEPSCMATPAPPSWWDSSQSSISSLGRLPSISPTAPGTWAAA WVPLPTVDVPDHAHYTLGTVILLVGLTGMLGNLTVIYTFCRSRSLRTPANMFIINL AVSDFLMSFTQAPVFFTSSLYKQWLFGETGCEFYAFCGALFGISSMITLTAIALDRY LVITRPLATFGVASKRRAAFVLLGVWLYALAWSLPPFFGWSAYVPEGLLTSCSWDY MSFTPAVRAYTMLLCCFVFFLPLLIIIYCYIFIFRAIRETGRALQTFGACKGNGESL WQRQRLQSECKMAKIMLLVILLFVLSWAPYSAVALVAFAGYAHVLTPYMSSVPAVI AKASAIHNPIIYAITHPKYRVAIAQHLPCLGVLLGVSRRHSRPYPSYRSTHRSTLTS HTSNLSWISIRRRQESLGSESEVGWTHMEAAAVWGAAQQANGRSLYGQGLEDLE AKAPPRPQGHEAETPGKTKGLIPSQDPRM* |
| >Homo sapiens Opn5 AAR21109.1 (SEQ ID NO.18) |
| MALNHTALPQDERLPHYLRDGDPFASKLSWEADLVAGFYLTIIGILSTFGNGYVLY MSSRRKKKLRPAEIMTINLAVCDLGISVVGKPFTIISCFCHRWVFGWIGCRWYGW AGFFFGCGSLITMTAVSLDRYLKICYLSYGVWLKRKHAYICLAAIWAYASFWTTMP LVGLGDYVPEPFGTSCTLDWWLAQASVGGQVFILNILFFCLLLPTAVIVFSYVKIIA |

**[Table 1-5]**

| |
|---|
| KVKSSSKEVAHFDSRIHSSHVLEMKLTKVAMLICAGFLIAWIPYAVVSVWSAFGRP DSIPIQLSVVPTLLAKSAAMYNPIIYQVIDYKFACCQTGGLKATKKKSLEGFRLHTV TTVRKSSAVLEIHEEWE* |
| >Homo sapiens Rgr AAA56748.1 (SEQ ID NO.19) |
| MAETSALPTGFGELEVLAVGMVLLVEALSGLSLNTLTIFSFCKTPELRTPCHLLVL SLALADSGISLNALVAATSSLLRRWPYGSDGCQAHGFQGFVTALASICSSAAIAWG RYHHYCTRSQLAWNSAVSLVLFVWLSSAFWAALPLLGWGHYDYEPLGTCCTLDY SKGDRNFTSFLFTMSFFNFAMPLFITITSYSLMEQKLGKSGHLQVNTTLPARTLLLL GWGPYAILYLYAVIADVTSISPKLQMVPALIAKMVPTINAINYALGNEMVCRGIWQC LSPQKREKDRTK* |
| >Homo sapiens Rrh AAC51757.1 (SEQ ID NO.20) |
| MLRNNLGNSSDSKNEDGSVFSQTEHNIVATYLIMAGMISIISNIIVLGIFIKYKELR TPTNAIIINLAVTDIGVSSIGYPMSAASDLYGSWKFGYAGCQVYAGLNIFFGMASIG LLTVVAVDRYLTICLPDVGRRMTTNTYIGLILGAWINGLFWALMPIIGWASYAPDPT GATCTINWRKNDRSFVSYTMTVIAINFIVPLTVMFYCYYHVTLSIKHHTTSDCTES LNRDWSDQIDVTKMSVIMICMFLVAWSPYSIVCLWASFGDPKKIPPPMAIIAPLFA KSSTFYNPCIYVVANKKFRRAMLAMFKCQTHQTMPVTSILPMDVSQNPLASGRI* |
| >Mus musculus Opn3 AAD32670.1 (SEQ ID NO.21) |
| MYSGNRSGDQGYWEDGAGAEGAAPAGTRSPAPLFSPTAYERLALLLGCLALLGV GGNLLVLLLYSKFPRLRTPTHLFLVNLSLGDLLVSLFGVTFTFASCLRNGWVWDA VGCAWDGFSGSLFGFVSITTLTVLAYERYIRVVHARVINFSWAWRAITYIWLYSLAW AGAPLLGWNRYILDIHGLGCTVDWRSKDANDSSFVLFLFLGCLVVPVGIIAHCYG HILYSVRMLRCVEDLQTIQVIKMLRYEKKVAKMCFLMAFVFLTCWMPYIVTRFLV VNGYGHLVTPTVSIVSYLFAKSSTVYNPVIYIFMNRKFRRSLLQLLCFRLLRCQRPA KNLPAAESEMHIRPIVMSQKDGDRPKKKVTFNSSSIIFIITSDESLSVEDSDRSSAS KVDVIQVRPL* |
| >Mus musculus Opn4 AAF24979.1 (SEQ ID NO.22) |
| MDSPSGPRVLSSLTQDPSFTTSPALQGIWNGTQNVSVRAQLLSVSPTTSAHQAAA WVPFPTVDVPDHAHYTLGTVILLVGLTGMLGNLTVIYTFCRNRGLRTPANMFIINL AVSDFLMSVTQAPVFFASSLYKKWLFGETGCEFYAFCGAVFGITSMITLTAIAMDR YLVITRPLATIGRGSKRRTALVLLGVWLYALAWSLPPFFGWSAYVPEGLLTSCSWD YMTFTPQVRAYTMLLFCFVFFLPLLIIIFCYIFIFRAIRETGRACEGCGESPLRQRRQ WQRLQSEWKMAKVALIVILLFVLSWAPYSTVALVAFAGYSHILTPYMSSVPAVIAKA SAIHNPIIYAITHPKYRVAIAQHLPCLGVLLGVSGQRSHPSLSYRSTHRSTLSSQSSD LSWISGRKRQESLGSESEVGWTDTETTAAWGAAQQASGQSFCSQNLEDGELKAS SSPQVQRSKTPKVPGPSTCRPMKGQGARPSSLRGDQKGRLAVCTGLSECPHPHTS |

**[Table 1-6]**

| |
|---|
| QFPLAFLEDDVTLRHL* |
| >Mus musculus Opn5 AAR08201.1 (SEQ ID NO.23) |
| MALNHTALPQDERLPHYLRDEDPFASKLSWEADLVAGFYLTIIGILSTFGNGYVLY MSSRRKKKLRPAEIMTINLAVCDLGISVVGKPFTIISCFCHRWVFGWFGCRWYGW AGFFFGCGSLITMTAVSLDRYLKICYLSYGVWLKRKHAYICLAVIWAYASFWTTMP LVGLGDYAPEPFGTSCTLDWWLAQASGGGQVFILSILFFCLLLPTAVIVFSYAKIIA KVKSSSKEVAHFDSRIHSSHVLEVKLTKVAMLICAGFLIAWIPYAVVSVWSAFGRP DSIPIQLSVVPTLLAKSAAMYNPIIYQVIDYRFACCQAGGLRGTKKKSLEDFRLHT VTAVRKSSAVLEIHPESSSRFTSAHVMDGESHSNDGDCGKK* |
| >Mus musculus Rgr AAC69836.1 (SEQ ID NO.24) |
| MAATRALPAGLGELEVLAVGTVLLMEALSGISLNGLTIFSFCKTPDLRTPSNLLVL SLALADTGISLNALVAAVSSLLRRWPHGSEGCQVHGFQGFATALASICGSAAVAWG RYHHYCTRRQLAWDTAIPLVLFVWMSSAFWASLPLMGWGHYDYEPVGTCCTLDY SRGDRNFISFLFTMAFFNFLVPLFITHTSYRFMEQKFSRSGHLPVNTTLPGRMLLL GWGPYALLYLYAALADVSFISPKLQMVPALIAKTMPTINAINYALHREMVCRGTWQ CLSPQKSKKDRTQ* |
| >Mus musculus Rrh AAC53344.1 (SEQ ID NO.25) |
| MLSEASDFNSSGSRSEGSVFSRTEHSVIAAYLIVAGITSILSNVVVLGIFIKYKELRT PTNAVIINLAFTDIGVSSIGYPMSAASDLHGSWKFGHAGCQIYAGLNIFFGMVSIGL LTVVAMDRYLTISCPDVGRRMTTNTYLSMILGAWINGLFWALMPIIGWASYAPDPT GATCTINWRNNDTSFVSYTMMVIVVNFIVPLTVMFYCYYHVSRSLRLYAASDCTA HLHRDWADQADVTKMSVIMILMFLLAWSPYSIVCLWACFGNPKKIPPSMAIIAPLF AKSSTFYNPCIYVAAHKKFRKAMLAMFKCQPHLAVPEPSTLPMDMPQSSLAPVRI * |
| >Gallus gallus VAL opsin ACX32474.1 (SEQ ID NO.26) |
| MDVFRALGNESLLSNSSGPARWDPFHHPLDSIQPWHFRLVAAVMFVVTSLSLAEN LAVILVTFKFKQLRQPVNYVIVNLSVADFLVSLTGGTISFLANLKGYFYMGHWACV LEGFAVTFFGIVALWSLALLAFERYIVICRPVGNMRLRGKHAAQGIAFVWTFSFIW TIPPTMGWSSYTTSKIGTTCEPNWYSGAYNDRSYIIAFFTTCFIVPLLVILVSYGKLL QKLRKVSNTQGRLRTARKPERQVTRMVVVMIIAFLICWMPYAVFSILATAYPSIEL DPHLAAIPAFFSKTATVYNPIIYVFMNKQFRMCLIQMFKCSAIETAESNMNPTSER ATLTQDKRDSQLSVMAVRSTIS* |
| >Gallus gallus Opn3 BAV92607.1 (SEQ ID NO.27) |
| MHSGNGTGATSRPQLAAAGHEVPGERPLFSAGTYELLALLIATIGTLGVCNNLLV LVLYYKFKRLRTPTNLFLVNISLSDLLVSVCGVSLTFMSCLRSRWVWDAAGCVWD |

**[Table 1-7]**

| |
|---|
| GFSNSLFGIVSIMTLTVLAYERYIRVVHAKVIDFSWSWRAITYIWLYSLAWTGAPLL GWNRYTLEIHGLGCSMDWKSKDPNDTSFVLLFFLGCLVAPVVIMAYCYGHILYAV RMLRCVEDFQTSQVIKLLKYEKKVAKMCFLMISTFLICWMPYAVVSLLVTYGYSN LVTPTVAIIPSFFAKSSTAYNPVIYIFMSRKFRQCLLQLLCFRLMRFQRIMKEPSGA GNVKPIRPIVMSQKVGDRPKKKVTFSSSSIIFIIASDDTQQIDDNSKHNGTKVNVIQ VKPL* |
| >Gallus gallus TMT opsin BAV93805.1 (SEQ ID NO.28) |
| MNHTWTYNLSFGAPTDPVEPRAGLSRNGHTVVAVFLGFILFFGFLNNLIVLILFCK FKTLRNPVNMLLLNISISDMLVCISGTTLSFASNIHGKWIGGEHGCRWYGFVNSC FGIVSLISLAVLSYERYSTLTLCNKRSDDYRKALLAVGGSWVYSLLWTVPPLLGWS SYGIEGAGTSCSVRWSSETAESTSYIICLFIFCLVIPVMVMMYCYGRLLYAVKQVGK IHKNTARKREYHVLFMVITTVICYLVCWIPYGVIALLATFGKPGVVTPVASIIPSILA KSSTVCNPIIYILMNKQFYKCFRQLFHCQPPSSTDGEPTCHSKVTVIQLNQKTDGG KLCNNKPRPETDNKVTSLLHPEPGLEPAAKTVPPM* |
| >Gallus gallus Opn4xABX10830.1 (SEQ ID NO.29) |
| MDLNMGTQPHSVTKSEIPDHVLYTVGTCVLVIGSIGIIGNLLVLYAFYSNKKLRTP QNFFIMNLAVSDFLMSASQAPICFINSLHREWILGDIGCDLYAFCGALFGITSMMT LLAISVDRYLVITKPLRSIQWTSKKRTIQIIAVWLYSLGWSVAPLLGWSSYVPEGL MISCTWDYVTYSPANRSYTMILCCCVFFIPLIIILHCYLFMFLAIRSTGRDVQKLGS CSRKSFLSQSMKNEWKLAKIAFVVIIVYVLSWSPYACVTLIAWAGRGNTLTPYSKS VPAVIAKASAIYNPIIYAIIHPRYRKTIHNAVPCLRFLIRISKNDLLRGSINESSFRTSL SSHQSLAGRTKNTCVSSVSTGEANWSDVELDTVEPAHEKLQPRRSHSFSSSLRQK RDLLPDSYSCSEETEEKVSLSSSYLEKVLGRSAFPSSPVALVTSSLRAASLPVGLNS SSASRGAGSDISQMKTEESHNNGGLDSIVSNTVPQIIIIPTSETNLFQEEPEEEETE LFHFHDKKNNLLDLEGLSSSTEFLEAVEKFLS* |
| >Gallus gallus Opn4m BAL14786.1 (SEQ ID NO.30) |
| MDLPPRAPTKMTVKDVRGAFPTVDVPDHAHYTIGTVILIVGITGTLGNFLVIYAFC RSRTLQKPANIFIINLAVSDFLMSITQSPVFFTNSLHKRWIFGEKGCELYAFCGALF GITSMITLMVIALDRYFVITKPLASVRVMSKKKALIIILVGVWLYSLAWSLPPFFGWS AYVPEGLLTSCSWDYMTFTPSVRAYTMLLFCFVFFIPLIAIIYSYVFIFEAIKKANKS VQTFGCKHGNRELQKQYHRMRNEWKLAKIALIVILLYVISWSPYSVVALVAFAGYS HVLTPFMNSVPAVIAKASAIHNPIIYAITHPKYRTAIATYVPCLGFLLRVSPKESRSF SSYPSSRRTTITSQSSETSGLQKGKRRLSSISDSESGCTDTETDITSMISRPASSQVS YEMGEDTTQTSDLGGKPKVKSHDSGIFRKTVVDADEIPMVEINDTEHSATSTCKT SEKCNVEEIQRSESLSGIGLREGESRHRTSASQIPSIIITYSNVQGVELPSGYSAGFL HPKNKSHKQNKSSNS* |

**[Table 1-8]**

| |
|---|
| >Gallus gallus Opn5m BAG65738.1 (SEQ ID NO.31) |
| MSGMASDCNSSSQEEYLPHYVQQEDPFASKLSREADIIAGFYLTVIGILSTLGNGY VIFMSSKRKKKLRPAEIMTVNLAVCDLGISVVGKPFSIISFFSHRWIFGWMGCRWY GWAGFFFGCGSLITMTAVSLDRYLKICHLAYGTWLKRHHAFICLALIWAYATFWAT VPFAGVGSYAPEPFGTSCTLDWWLAQASVAGQAFVLSILFFCLLFPTAVIVFSYVKI ILKVKSSTKEVAHYDTRIQNSHILEMKLTKVAMLICAGFLIAWIPYAVVSVWSAFG QPDSVPIQFSVVPTLLAKSAAMYNPIIYQVIDCKFACCRSGGPKTLQKKSSLKESR MYTLSSHRDSAALSGTQLEV* |
| >Gallus gallus Opn5L2 BAG65739.2 (SEQ ID NO.32) |
| MEEQYISKLHPVVDYGAGVFLLIIAILTILGNSAVLATAVKRSSLLKSPELLTVNLAV ADIGMAISMYPLAIASAWNHAWLGGDASCIYYALMGFLFGVCSMMTLCAMAVIRF LVTNSSKSNSNKISKNTVHILITFIWLYSLLWAILPLVGWGYYGPEPFGISCTIAWS KFHSSSNGFSFILSMFLLCTVLPALTIVACYLGIAWKVHKAYQEIQNINRIPHAAKL EKKLTLMAVLISVGFLSAWTPYAAASFWSIFNSSDSLQPIVTLLPCLFAKSSTAYNP FIYYIFSKTFRHEIKQLQCCWGWRVHFFSADNSAENSVSMMWSGRDNIRLSPTAK VESQGAARH* |
| >Gallus gallus RrhAAR02098.1 (SEQ ID NO.33) |
| MHWNDSANSSESDAEAHSVFTQTEHNIVAAYLITAGVISIFSNIVVLGIFVKYKEFR TATNAIINLAFTDIGVSGIGYPMSAASDLHGSWKFGYTGCQIYAALNIFFGMASIG LLTVVAVDRYLTICRPDIGRRMTTRNYAALILAAWINAVFWASMPTVGWAGYASDP TGATCTANWRKNDVPFVSYTMSVIAVNFVVPLTVMFYCYYNVSRTMKQYTSSNC LESINMDWSDQVDVTKMSVVMIVMFLVAWSPYSIVCLWSSFGDPKKISPAMAIIAP LFAKSSTFYNPCIYVIANKKFRRAILAMVRCQTRQEITISNALPMTVSLSALTS* |
| >Gallus gallus Rgr AAR02099.1 (SEQ ID NO.34) |
| MVTSHPLPEGFTEIEVFAIGTALLVEALLGFCLNGLTIISFRKIKELRTPSNLLVLSI ALADCGICINAFIAAFSSFLRYWPYGSEGCQIHGFQGFLTALASISSSAAVAWDRYH HYCTRSKLQWSTAISMMVFAWLFAAFWATMPLLGWGEYDYEPLRTCCTLDYSKG DRNYITFLFALSIFNFMIPGFIMMTAYQSIHQKFKKSGHYKFNTGLPLKTLVICWG PYCLLSFYAAIENVMFISPKYRMIPAIIAKTVPTVDSFVYALGNENYRGGIWQFLTG QKIEKAEVDSKTK* |

In one embodiment, specific nucleic acid sequences of an opsin of the present disclosure may include, but are not limited to, sequences of SEQ ID NOs: 35 to 68 listed in Table 2.

**[Table 2-1]**

| |
|---|
| >Homo sapiens rhodopsin NM_000589.3 (SEQ ID NO.35) |
| ATGAATGGCACAGAAGGCCCTAACTTCTACGTGCCCTTCTCCAATGCGACGGGT GTGGTACGCAGCCCCTTCGAGTACCCACAGTACTACCTGGCTGAGCCATGGCA GTTCTCCATGCTGGCCGCCTACATGTTTCTGCTGATCGTGCTGGGCTTCCCCAT CAACTTCCTCACGCTCTACGTCACCGTCCAGCACAAGAAGCTGCGCACGCCTCT CAACTACATCCTGCTCAACCTAGCCGTGGCTGACCTCTTCATGGTCCTAGGTGG CTTCACCAGCACCCTCTACACCTCTCTGCATGGATACTTCGTCTTCGGGCCCAC AGGATGCAATTTGGAGGGCTTCTTTGCCACCCTGGGCGGTGAAATTGCCCTGT GGTCCTTGGTGGTCCTGGCCATCGAGCGGTACGTGGTGGTGTGTAAGCCCATG AGCAACTTCCGCTTCGGGGAGAACCATGCCATCATGGGCGTTGCCTTCACCTG GGTCATGGCGCTGGCCTGCGCCGCACCCCCACTCGCCGGCTGGTCCAGGTACA TCCCCGAGGGCCTGCAGTGCTCGTGTGGAATCGACTACTACACGCTCAAGCCG GAGGTCAACAACGAGTCTTTTGTCATCTACATGTTCGTGGTCCACTTCACCATC CCCATGATTATCATCTTTTTCTGCTATGGGCAGCTCGTCTTCACCGTCAAGGAG GCCGCTGCCCAGCAGCAGGAGTCAGCCACCACACAGAAGGCAGAGAAGGAGG TCACCCGCATGGTCATCATCATGGTCATCGCTTTCCTGATCTGCTGGGTGCCCT ACGCCAGCGTGGCATTCTACATCTTCACCCACCAGGGCTCCAACTTCGGTCCCA TCTTCATGACCATCCCAGCGTTCTTTGCCAAGAGCGCCGCCATCTACAACCCTG TCATCTATATCATGATGAACAAGCAGTTCCGGAACTGCATGCTCACCACCATCT GCTGCGGCAAGAACCCACTGGGTGACGATGAGGCCTCTGCTACCGTGTCCAAG ACGGAGACGAGCCAGGTGGCCCCGGCCTAA |
| >Mus musculus rhodopsin NM_145383.2 (SEQ ID NO.36) |
| ATGAACGGCACAGAGGGCCCCAATTTTTATGTGCCCTTCTCCAACGTCACAGGC GTGGTGCGGAGCCCCTTCGAGCAGCCGCAGTACTACCTGGCGGAACCATGGCA GTTCTCCATGCTGGCAGCGTACATGTTCCTGCTCATCGTGCTGGGCTTCCCCAT CAACTTCCTCACGCTCTACGTCACCGTACAGCACAAGAAGCTGCGCACACCCCT CAACTACATCCTGCTCAACTTGGCCGTGGCTGACCTCTTCATGGTCTTCGGAGG ATTCACCACCACCCTCTACACATCACTCCATGGCTACTTCGTCTTTGGGCCCAC AGGCTGTAATCTCGAGGGCTTCTTTGCCACACTTGGAGGTGAAATCGCCCTGTG GTCCCTGGTGGTCCTGGCCATTGAGCGCTACGTGGTGGTCTGCAAGCCGATGA GCAACTTCCGCTTCGGGGAGAATCACGCTATCATGGGTGTGGTCTTCACCTGGA TCATGGCGTTGGCCTGTGCTGCTCCCCCACTCGTTGGCTGGTCCAGGTACATCC CTGAGGGCATGCAATGTTCATGCGGGATTGACTACTACACACTCAAGCCTGAGG TCAACAACGAATCCTTTGTCATCTACATGTTCGTGGTCCACTTCACCATTCCTAT GATCGTCATCTTCTTCTGCTATGGGCAGCTGGTCTTCACAGTCAAGGAGGCGGC TGCCCAGCAGCAGGAGTCAGCCACCACTCAGAAGGCAGAGAAGGAAGTCACCC GCATGGTTATCATCATGGTCATCTTCTTCCTGATCTGCTGGCTTCCCTACGCCAG TGTGGCCTTCTACATCTTCACCCACCAGGGCTCCAACTTCGGCCCCATCTTCAT GACTCTGCCAGCTTTCTTTGCTAAGAGCTCTTCCATCTATAACCCGGTCATCTAC ATCATGTTGAACAAGCAGTTCCGGAACTGTATGCTCACCACGCTGTGCTGCGGC |

**[Table 2-2]**

| |
|---|
| AAGAATCCACTGGGAGATGACGACGCCTCTGCCACCGCTTCCAAGACGGAGAC CAGCCAGGTGGCTCCAGCCTAA |
| >Canis familiaris rhodopsin X71380.1 (SEQ ID NO.37) |
| ATGAACGGGACGGAGGGCCCGAACTTCTACGTGCCCTTCTCCAACAAGACGGG TGTGGTGCGCAGCCCCTTCGAGTACCCACAGTACTACCTGGCTGAGCCATGGC AGTTCTCCATGCTGGCTGCCTACATGTTTCTGCTGATCGTGCTCGGCTTCCCCA TCAACTTCCTCACGCTCTACGTCACAGTCCAGCACAAGAAGCTGCGTACACCTC TCAACTACATCCTGCTCAACCTGGCTGTGGCTGACCTCTTCATGGTCTTCGGTG GCTTCACCACCACCCTCTACACCTCTCTGCATGGATACTTTGTCTTCGGGCCCA CAGGATGCAATGTGGAGGGCTTCTTTGCCACACTGGGCGGTGAAATTGCCCTG TGGTCTTTGGTGGTCCTGGCCATTGAGCGGTACGTGGTGTGTAAGCCCAT GAGCAACTTCCGTTTTGGGGAGAACCATGCCATCATGGGCGTCGCCTTCACCT GGGTCATGGCGCTGGCCTGTGCGGCACCCCCCCTCGCTGGCTGGTCCAGGTAC ATCCCAGAGGGCATGCAGTGCTCATGTGGGATCGACTACTACACACTCAAGCCA GAAATCAACAATGAGTCCTTCGTCATCTACATGTTCGTGGTCCACTTCGCCATC CCCATGATTGTCATATTCTTCTGCTATGGACAGCTCGTCTTCACAGTCAAGGAG GCAGCTGCCCAGCAGCAGGAATCGGCCACCACCCAGAAGGCTGAAAAGGAGG TCACCCGCATGGTCATCATCATGGTCATCGCTTTCCTGATCTGCTGGGTGCCCT ATGCCAGTGTGGCATTCTACATCTTCACCCACCAGGGCTCCGACTTTGGCCCCA TCTTCATGACCCTCCCAGCGTTCTTCGCCAAGTCCTCCTCCATCTACAACCCTG TCATCTATATCATGATGAACAAGCAGTTCCGGAACTGCATGATCACCACCCTCT GCTGCGGCAAGAACCCACTGGGTGACGACGAGGCCTCTGCCAGCGCCTCCAAG ACGGAGACCAGCCAGGTGGCACCGGCCTAA |
| >Gallus gallus rhodopsin NM_001397497.1 (SEQ ID NO.38) |
| ATGAACGGGACGGAAGGCCAAGACTTCTACGTGCCCATGTCCAACAAGACCGG GGTGGTGCGGAGCCCCTTCGAGTACCCCCAGTACTACCTGGCTGAGCCCTGGA AGTTCTCGGCGCTGGCTGCCTACATGTTCATGCTGATCCTGCTCGGCTTCCCCG TCAACTTCCTCACGCTGTACGTCACCATCCAGCACAAGAAACTCCGGACGCCTC TAAACTACATCCTCCTGAACCTGGCGGTCGCCGACCTCTTTATGGTCTTCGGAG GCTTCACGACCACCATGTACACCTCGATGAACGGGTACTTTGTCTTTGGAGTAA CAGGGTGCTACATCGAGGGCTTCTTTGCTACGCTGGGCGGTGAAATTGCTCTCT GGTCACTCGTCGTCCTGGCCGTGGAACGATACGTGGTGGTCTGTAAGCCCATG AGCAACTTCCGCTTCGGGGAGAACCACGCCATCATGGGGGTCGCGTTCTCCTG GATCATGGCCATGGCCTGCGCAGCCCCCCCGCTGTTCGGCTGGTCACGGTACA TCCCCGAGGGCATGCAGTGCTCGTGTGGGATCGACTACTACACGCTGAAGCCG GAGATCAACAACGAATCCTTTGTCATCTACATGTTCGTGGTCCACTTCATGATCC CACTGGCCGTCATCTTCTTCTGCTATGGGAACCTGGTCTGCACTGTGAAGGAGG CTGCCGCCCAGCAGCAGGAGTCTGCCACCACCCAGAAGGCAGAGAAGGAAGT |

**[Table 2-3]**

| |
|---|
| GACCCGCATGGTGATCATCATGGTCATCGCCTTCCTCATCTGCTGGGTTCCCTA CGCCAGCGTCGCTTTCTACATCTTCACCAACCAGGGCTCAGACTTTGGACCCAT CTTCATGACCATCCCGGCATTCTTTGCCAAGAGCTCTGCCATCTACAATCCTGT GATCTACATCGTAATGAACAAACAGTTCCGTAACTGCATGATCACAACCCTCTG CTGCGGCAAGAACCCGCTGGGCGATGAGGACACGTCTGCTGGAAAGACAGAG ACCTCCTCCGTCTCCACCAGCCAGGTGTCCCCTGCATAG |
| >Oryzias latipes rhodopsin AB180742.1 (SEQ ID NO.39) |
| ATGAATGGCACAGAGGGACCATATTTTTATGTCCCTATGGTGAACACCACCGGC ATTGTCCGGAGTCCTTATGAATATCCTCAGTATTACCTTGTCAACCCAGCAGCCT ATGCTGCTCTGGGCGCCTACATGTTTTTTCTCATCCTGGTTGGCTTCCCCATCA ACTTCCTCACTCTCTACGTCACCCTCGAACACAAGAAGCTGCGAACCCCTCTAA ACTACATCCTGCTAAACCTCGCGGTGGCCGACCTCTTCATGGTGTTCGGAGGAT TCACTACAACGATGTACACCTCCATGCACGGCTACTTTGTCCTTGGACGTCTCG GCTGCAATCTGGAAGGATTCTTTGCTACCCTTGGTGGTGAAATTGGTCTCTGGT CACTGGTTGTCCTGGCTATTGAGAGATGGGTTGTTGTCTGCAAGCCCATCAGCA ACTTCCGCTTTGGTGAGAATCATGCTATTATGGGTTTGGTCTTCACCTGGATCAT GGCTGCCTCCTGTGCCGTGCCCCCTCTCGTCGGCTGGTCTCGTTACATCCCAGA GGGCATGCAGTGCTCATGTGGAGTCGACTACTACACCCGTGCAGAGGGCTTCA ACAATGAGTCCTTTGTTGTCTACATGTTCGTCTGCCACTTCCTAATTCCACTGAT CGTTGTGTTCTTCTGCTACGGCCGTCTGCTCTGTGCTGTCAAGGAGGCTGCTGC TGCCCAGCAGGAGTCTGAGACCACCCAGAGAGCTGAGAGGGAAGTCACCCGC ATGGTTGTCATCATGGTCATCGGCTTCCTGGTATGTTGGCTTCCCTATGCCAGT GTGGCCTGGTACATCTTCACAAATCAGGGCTCTGAATTCGGGCCACTCTTCATG ACCATCCCAGCCTTCTTTGCCAAGAGTTCTTCCATCTACAACCCAGCAATCTAC ATCTGCATGAACAAGCAGTTCCGCAACTGCATGATCACCACCCTGTGCTGTGGG AAGAATCCCTTTGAAGAGGAGGAGGGAGCCTCCACTACTGCTTCCAAGACCGA GGCCTCATCTGTCTCCTCAAGCTCTGTGTCCCCTGCATAA |
| >Homo sapiens blue cone opsin NM_001385125.1 (SEQ ID NO.40) |
| ATGAGAAAAATGTCGGAGGAAGAGTTTTATCTGTTCAAAAATATCTCTTCAGTG GGGCCGTGGGATGGGCCTCAGTACCACATTGCCCCTGTCTGGGCCTTCTACCT CCAGGCAGCTTTCATGGGCACTGTCTTCCTTATAGGGTTCCCACTCAATGCCAT GGTGCTGGTGGCCACACTGCGCTACAAAAAGTTGCGGCAGCCCCTCAACTACA TTCTGGTCAACGTGTCCTTCGGAGGCTTCCTCCTCTGCATCTTCTCTGTCTTCC CTGTCTTCGTCGCCAGCTGTAACGGATACTTCGTCTTCGGTCGCCATGTTTGTG CTTTGGAGGGCTTCCTGGGCACTGTAGCAGGTCTGGTTACAGGATGGTCACTG GCCTTCCTGGCCTTTGAGCGCTACATTGTCATCTGTAAGCCCTTCGGCAACTTC CGCTTCAGCTCCAAGCATGCACTGACGGTGGTCCTGGCTACCTGGACCATTGG TATTGGCGTCTCCATCCCACCCTTCTTTGGCTGGAGCCGGTTCATCCCTGAGGG |

**[Table 2-4]**

| |
|---|
| CCTGCAGTGTTCCTGTGGCCCTGACTGGTACACCGTGGGCACCAAATACCGCA GCGAGTCCTATACGTGGTTCCTCTTCATCTTCTGCTTCATTGTGCCTCTCTCCCT CATCTGCTTCTCCTACACTCAGCTGCTGAGGGCCCTGAAAGCTGTTGCAGCTCA GCAGCAGGAGTCAGCTACGACCCAGAAGGCTGAACGGGAGGTGAGCCGCATG GTGGTTGTGATGGTAGGATCCTTCTGTGTCTGCTACGTGCCCTACGCGGCCTTC GCCATGTACATGGTCAACAACCGTAACCATGGGCTGGACTTACGGCTTGTCACC ATTCCTTCATTCTTCTCCAAGAGTGCTTGCATCTACAATCCCATCATCTACTGCT TCATGAATAAGCAGTTCCAAGCTTGCATCATGAAGATGGTGTGTGGGAAGGCCA TGACAGATGAATCCGACACATGCAGCTCCCAGAAAACAGAAGTTTCTACTGTCT CGTCTACCCAAGTTGGCCCCAACTGA |
| >Homo sapiens red cone opsin NM_020061.6 (SEQ ID NO.41) |
| ATGGCCCAGCAGTGGAGCCTCCAAAGGCTCGCAGGCCGCCATCCGCAGGACAG CTATGAGGACAGCACCCAGTCCAGCATCTTCACCTACACCAACAGCAACTCCAC CAGAGGCCCCTTCGAAGGCCCGAATTACCACATCGCTCCCAGATGGGTGTACC ACCTCACCAGTGTCTGGATGATCTTTGTGGTCACTGCATCCGTCTTCACAAATG GGCTTGTGCTGGCGGCCACCATGAAGTTCAAGAAGCTGCGCCACCCGCTGAAC TGGATCCTGGTGAACCTGGCGGTCGCTGACCTAGCAGAGACCGTCATCGCCAG CACTATCAGCATTGTGAACCAGGTCTCTGGCTACTTCGTGCTGGGCCACCCTAT GTGTGTCCTGGAGGGCTACACCGTCTCCCTGTGTGGGATCACAGGTCTCTGGT CTCTGGCCATCATTTCCTGGGAGAGATGGATGGTGGTCTGCAAGCCCTTTGGCA ATGTGAGATTTGATGCCAAGCTGGCCATCGTGGGCATTGCCTTCTCCTGGATCT GGGCTGCTGTGTGGACAGCCCCGCCCATCTTTGGTTGGAGCAGGTACTGGCCC CACGGCCTGAAGACTTCATGCGGCCCAGACGTGTTCAGCGGCAGCTCGTACCC CGGGGTGCAGTCTTACATGATTGTCCTCATGGTCACCTGCTGCATCATCCCACT CGCTATCATCATGCTCTGCTACCTCCAAGTGTGGCTGGCCATCCGAGCGGTGGC AAAGCAGCAGAAAGAGTCTGAATCCACCCAGAAGGCAGAGAAGGAAGTGACGC GCATGGTGGTGGTGATGATCTTTGCGTACTGCGTCTGCTGGGGACCCTACACCT TCTTCGCATGCTTTGCTGCTGCCAACCCTGGTTACGCCTTCCACCCTTTGATGG CTGCCCTGCCGGCCTACTTTGCCAAAAGTGCCACTATCTACAACCCCGTTATCT ATGTCTTTATGAACCGGCAGTTTCGAAACTGCATCTTGCAGCTTTTCGGGAAGA AGGTTGACGATGGCTCTGAACTCTCCAGCGCCTCCAAAACGGAGGTCTCATCT GTGTCCTCGGTATCGCCTGCATGA |
| >Homo sapiens green cone opsin NM_000513.2 (SEQ ID NO.42) |
| ATGGCCCAGCAGTGGAGCCTCCAAAGGCTCGCAGGCCGCCATCCGCAGGACAG CTATGAGGACAGCACCCAGTCCAGCATCTTCACCTACACCAACAGCAACTCCAC CAGAGGCCCCTTCGAAGGCCCGAATTACCACATCGCTCCCAGATGGGTGTACC ACCTCACCAGTGTCTGGATGATCTTTGTGGTCATTGCATCCGTCTTCACAAATG GGCTTGTGCTGGCGGCCACCATGAAGTTCAAGAAGCTGCGCCACCCGCTGAAC |

**[Table 2-5]**

| |
|---|
| TGGATCCTGGTGAACCTGGCGGTCGCTGACCTGGCAGAGACCGTCATCGCCAG CACTATCAGCGTTGTGAACCAGGTCTATGGCTACTTCGTGCTGGGCCACCCTAT GTGTGTCCTGGAGGGCTACACCGTCTCCCTGTGTGGGATCACAGGTCTCTGGT CTCTGGCCATCATTTCCTGGGAGAGATGGATGGTGGTCTGCAAGCCCTTTGGCA ATGTGAGATTTGATGCCAAGCTGGCCATCGTGGGCATTGCCTTCTCCTGGATCT GGGCTGCTGTGTGGACAGCCCCGCCCATCTTTGGTTGGAGCAGGTACTGGCCC CACGGCCTGAAGACTTCATGCGGCCCAGACGTGTTCAGCGGCAGCTCGTACCC CGGGGTGCAGTCTTACATGATTGTCCTCATGGTCACCTGCTGCATCACCCCACT CAGCATCATCGTGCTCTGCTACCTCCAAGTGTGGCTGGCCATCCGAGCGGTGG CAAAGCAGCAGAAAGAGTCTGAATCCACCCAGAAGGCAGAGAAGGAAGTGACG CGCATGGTGGTGGTGATGGTCCTGGCATTCTGCTTCTGCTGGGGACCATACGC CTTCTTCGCATGCTTTGCTGCTGCCAACCCTGGCTACCCCTTCCACCCTTTGAT GGCTGCCCTGCCGGCCTTCTTTGCCAAAAGTGCCACTATCTACAACCCCGTTAT CTATGTCTTTATGAACCGGCAGTTTCGAAACTGCATCTTGCAGCTTTTCGGGAA GAAGGTTGACGATGGCTCTGAACTCTCCAGCGCCTCCAAAACGGAGGTCTCAT CTGTGTCCTCGGTATCGCCTGCATGA |
| >Mus musculus UV cone opsin AF190670.1 (SEQ ID NO.43) |
| ATGTCAGGAGAGGATGACTTTTACCTGTTTCAGAATATCTCTTCGGTGGGGCCC TGGGATGGGCCTCAGTACCACCTTGCTCCTGTCTGGGCCTTCCGCCTCCAGGC AGCCTTCATGGGATTTGTCTTCTTTGTGGGGACCCCACTCAATGCCATAGTGCT GGTGGCCACACTGCATTACAAAAAGTTGCGACAGCCCCTCAACTATATTCTGGT CAATGTATCCCTCGGGGGCTTCCTCTTCTGCATCTTCTCTGTCTTCACAGTCTTC ATCGCCAGCTGTCACGGATACTTCCTCTTTGGTCGCCATGTTTGTGCTCTGGAG GCCTTCTTGGGCTCTGTAGCAGGTCTAGTGACAGGATGGTCATTGGCTTTCCTG GCTTTTGAACGCTACGTTGTCATCTGTAAACCCTTCGGCAGCATCCGCTTCAAC TCCAAGCATGCACTGATGGTGGTCCTGGCTACTTGGATTATTGGTATCGGGGTG TCCATCCCACCCTTTTTTGGCTGGAGCAGGTTCATCCCTGAGGGCCTGCAGTGC TCCTGTGGCCCAGACTGGTACACTGTGGGCACCAAGTATCGAAGCGAGTACTA CACCTGGTTCCTCTTCATCTTCTGTTTCATCATTCCTCTTTCCCTCATCTGCTTC TCCTACTCCCAGTTGCTGAGGACTCTCAGAGCTGTGGCAGCTCAGCAGCAAGA GTCTGCTACGACACAAAAGGCTGAACGGGAGGTGAGTCATATGGTGGTGGTGA TGGTGGGATCCTTCTGTCTCTGCTACGTGCCCTATGCTGCCCTGGCCATGTACA TGGTCAACAATCGGAACCACGGGCTGGACTTACGGCTTGTCACCATCCCCGCC TTCTTTTCCAAGAGCTCCTGTGTCTACAACCCCATCATCTACTGCTTCATGAATA AGCAGTTCCGGGCTTGCATTCTGGAGATGGTGTGCAGGAAGCCCATGGCAGAC GAATCTGACGTGTCTGGCTCTCAGAAAACAGAAGTTTCTACTGTCTCTTCTAGC AAAGTTGGCCCTCACTGA |
| >Mus musculus green cone opsin AF011389.1 (SEQ ID NO.44) |

**[Table 2-6]**

| |
|---|
| ATGGCCCAAAGGCTTACAGGTGAACAGACACTGGACCACTATGAGGATAGCAC CCATGCAAGCATCTTCACCTATACCAACAGCAACAGCACCAAAGGTCCCTTTGA AGGCCCCAATTATCACATTGCTCCCAGGTGGGTGTACCACCTCACCAGCACCTG GATGATTCTTGTGGTCGTTGCATCTGTCTTCACTAATGGACTTGTGCTGGCAGC CACCATGAGATTCAAGAAGCTGCGCCATCCACTGAACTGGATTCTGGTGAACTT GGCAGTTGCTGACCTAGCAGAGACCATTATTGCCAGCACTATCAGTGTTGTGAA CCAAATCTATGGCTACTTCGTTCTGGGACACCCTCTGTGTGTCATTGAAGGCTA CATTGTCTCATTGTGTGGAATCACAGGCCTCTGGTCCCTGGCCATCATTTCCTG GGAGAGATGGCTGGTGGTCTGCAAGCCCTTTGGCAATGTGAGATTTGATGCTA AGCTGGCCACTGTGGGAATCGTCTTCTCCTGGGTCTGGGCTGCTATATGGACG GCCCCACCAATCTTTGGTTGGAGCAGGTACTGGCCTTATGGCCTGAAGACATCC TGTGGCCCAGACGTGTTCAGCGGTACCTCGTACCCCGGGGTTCAGTCTTATATG ATGGTCCTCATGGTCACGTGCTGCATCTTCCCACTCAGCATCATCGTGCTCTGC TACCTCCAAGTGTGGCTGGCCATCCGAGCAGTGGCAAAGCAACAGAAAGAATC TGAGTCCACTCAGAAGGCCGAGAAGGAGGTGACACGCATGGTGGTGGTGATGG TCTTCGCATACTGCCTCTGCTGGGGACCCTATACTTTCTTTGCATGCTTTGCTAC TGCCCACCCTGGCTATGCCTTCCACCCTCTTGTGGCCTCCCTACCATCCTACTTT GCCAAAAGTGCCACTATCTACAACCCCATTATCTATGTCTTTATGAACCGGCAGT TTCGAAACTGCATCTTACATCTCTTTGGAAAGAAGGTTGATGATAGCTCTGAAC TTTCCAGCACCTCCAAGACAGAAGTCTCATCTGTCTCTTCAGTGTCACCTGCAT AA |
| >Gallus gallus green cone opsin M92038.1 (SEQ ID NO.45) |
| ATGAATGGGACAGAAGGTATCAATTTTTATGTGCCTATGTCCAACAAGACAGGG GTGGTGCGAAGCCCCTTCGAGTACCCCCAGTATTACCTAGCCGAGCCCTGGAA ATACCGCCTTGTGTGTTGCTACATCTTCTTCCTCATCTCCACTGGTCTGCCCATC AACCTCCTCACCCTCCTGGTCACCTTCAAACACAAGAAGCTCCGGCAGCCGCT CAACTACATCCTGGTCAACCTGGCGGTGGCTGACCTCTTCATGGCCTGCTTCGG CTTCACCGTCACTTTCTACACAGCCTGGAATGGATATTTCGTCTTCGGTCCCGT TGGCTGTGCTGTGGAGGGCTTCTTCGCCACTCTGGGAGGCCAGGTTGCCCTCT GGTCCCTGGTGGTCTTGGCCATAGAGCGTTACATCGTTGTCTGCAAGCCCATGG GAAACTTCCGCTTCTCTGCCACCCACGCCATGATGGGCATAGCTTTCACCTGGG TCATGGCCTTCTCCTGTGCTGCTCCACCCCTCTTTGGCTGGTCCAGGTACATGC CGGAGGGGATGCAGTGCTCCTGTGGCCCTGACTATTACACCCACAACCCTGAC TACCACAACGAGTCCTACGTCCTCTACATGTTTGTCATCCACTTCATCATCCCGG TCGTGGTCATCTTCTTCTCCTATGGGCGCCTCATCTGCAAAGTCCGAGAGGCAG CCGCGCAGCAGCAGGAGTCAGCCACCACCCAGAAGGCAGAGAAGGAGGTGAC GCGGATGGTGATCCTGATGGTGCTGGGCTTCATGCTGGCCTGGACACCCTATG CTGTAGTGGCATTCTGGATCTTCACCAACAAGGGAGCAGACTTCACCGCCACAC TGATGGCAGTGCCTGCCTTCTTCTCCAAGAGCTCCTCTCTCTACAACCCCATCA TCTATGTCCTCATGAACAAGCAGTTCCGTAATTGCATGATCACCACTATCTGCTG |

**[Table 2-7]**

| |
|---|
| TGGCAAGAACCCTTTTGGTGACGAAGATGTCTCTTCCACCGTATCCCAGAGCAA GACTGAGGTCTCCTCTGTCTCCTCCAGCCAAGTTTCACCTGCATAG |
| >Gallus gallus blue cone opsin M92037.1 (SEQ ID NO.46) |
| ATGCACCCCCCCCGCCCCACCACGGACCTCCCGGAGGATTTCTACATCCCCATG GCTTTGGACGCCCCCAACATTACGGCGCTCAGCCCCTTCCTGGTCCCCCAAACC CACCTGGGCAGCCCGGGGCTGTTCAGAGCTATGGCGGCCTTTATGTTCCTCCTC ATCGCTTTGGGGGTCCCCATCAACACGTTGACCATTTTTTGCACCGCCCGTTTC CGGAAGCTCCGTTCCCACCTTAATTACATCCTGGTTAATTTGGCCTTGGCCAAC CTGCTGGTGATCCTGGTGGGCTCCACCACGGCCTGTTACAGCTTCTCGCAGAT GTACTTCGCCCTCGGACCCACGGCGTGCAAAATCGAGGGCTTCGCTGCCACGC TGGGAGGCATGGTGAGTCTGTGGTCGTTGGCCGTGGTGGCGTTCGAGCGCTTC CTCGTCATCTGCAAACCTTTGGGCAACTTCACGTTCCGCGGCAGCCACGCCGT GTTGGGCTGCGTGGCCACGTGGGTGCTCGGATTCGTCGCCTCCGCGCCGCCGC TCTTCGGATGGAGTCGGTACATCCCGGAGGGGCTGCAGTGCTCGTGCGGGCCG GACTGGTACACGACGGACAACAAGTGGCACAACGAGAGCTACGTGCTCTTCCT GTTCACCTTCTGCTTCGGGGTCCCTCTCGCCATCATCGTCTTCTCCTACGGCCG CCTCCTCATCACACTCAGAGCGGTTGCCCGGCAGCAGGAGCAGTCAGCGACGA CGCAGAAGGCGGACCGGGAGGTGACGAAGATGGTGGTGGTGATGGTGCTGGG CTTCCTGGTGTGCTGGGCGCCATACACGGCCTTCGCGCTGTGGGTGGTGACGC ACCGCGGGCGCAGCTTCGAGGTGGGATTGGCCTCCATCCCCTCCGTCTTCTCC AAGTCCTCCACCGTCTACAACCCCGTCATCTACGTCCTCATGAACAAGCAGTTC CGCTCGTGCATGCTGAAGCTGCTGTTCTGCGGCCGCAGTCCGTTCGGGGACGA CGAGGACGTGTCGGGCTCCTCGCAGGCCACGCAGGTCTCCTCCGTCTCCTCCA GCCACGTCGCCCCCGCCTAA |
| >Gallus gallus violet cone opsin M92039.1 (SEQ ID NO.47) |
| ATGTCATCGGACGACGACTTCTACCTCTTCACCAACGGCTCAGTGCCGGGCCC GTGGGACGGCCCCCAATACCACATCGCCCCCCCGTGGGCCTTCTACCTACAGA CGGCCTTCATGGGGATCGTATTCGCCGTGGGGACCCCACTGAACGCCGTGGTG CTGTGGGTCACCGTTCGTTACAAACGACTCCGGCAGCCCCTTAATTACATCCTG GTCAACATCTCGGCCAGCGGATTCGTCTCCTGCGTGCTCAGCGTCTTCGTCGTC TTCGTGGCCAGCGCCCGCGGATACTTCGTCTTCGGGAAGAGGGTCTGCGAGTT GGAGGCCTTCGTGGGCACCCACGGGGGGCTGGTGACGGGATGGTCCCTGGCC TTTTTGGCCTTCGAGCGTTACATCGTTATCTGCAAACCGTTCGGCAACTTCCGG TTCAGCTCCCGGCACGCCCTGCTGGTCGTCGTGGCCACGTGGCTCATCGGCGT CGGCGTCGGACTGCCCCCCTTCTTCGGGTGGAGCCGGTACATGCCTGAGGGTC TGCAGTGCTCGTGTGGCCCCGACTGGTACACGGTGGGCACGAAATACCGCAGC GAGTACTACACGTGGTTCCTCTTCATCTTCTGCTTCATCGTCCCCCTCTCCCTCA TCATCTTCTCCTACTCCCAACTGCTGAGCGCCCTGCGAGCCGTGGCGGCGCAG |

**[Table 2-8]**

| |
|---|
| CAGCAGGAGTCGGCCACGACGCAGAAGGCGGAGCGGGAGGTGTCGCGGATGG TGGTGGTGATGGTGGGGTCGTTCTGCCTCTGCTACGTCCCCTACGCCGCCCTC GCCATGTATATGGTCAACAACCGCGACCACGGCTTGGACCTCCGCCTCGTCACC ATCCCCGCCTTCTTCTCCAAGAGCGCCTGCGTCTACAACCCCATCATCTACTGC TTCATGAACAAGCAGTTCCGCGCCTGCATCATGGAGACGGTTTGCGGGAAGCC GCTGACGGACGATTCGGACGCGTCGACTTCAGCGCAGAGGACGGAGGTGTCCT CCGTGTCCTCCAGCCAAGTCGGCCCCACTTAG |
| >Gallus gallus red cone opsin X57490.1 (SEQ ID NO.48) |
| ATGGCGGCGTGGGAGGCGGCGTTCGCCGCTCGGAGGCGGCACGAAGAGGAGG ACACCACGCGCGACAGCGTCTTCACCTACACCAACAGCAACAACACCCGCGGA CCATTTGAGGGCCCCAACTACCACATAGCGCCGCGCTGGGTGTACAACCTGAC GTCGCTGTGGATGATCTTCGTGGTGGCGGCGTCGGTGTTCACCAACGGGTTGG TGCTCGTGGCCACCTGGAAGTTCAAGAAGCTGCGCCACCCCCTCAACTGGATC CTGGTGAACCTCGCCGTGGCCGACCTGGGCGAGACCGTCATCGCCTCCACCAT CAGCGTCATCAACCAGATCTCGGGGTACTTCATCCTCGGGCACCCCATGTGCGT CGTCGAGGGATACACCGTGTCTGCATGCGGCATTACGGCGCTGTGGTCGCTGG CGATCATTTCGTGGGAGCGGTGGTTCGTGGTGTGCAAACCGTTCGGGAACATC AAATTCGACGGGAAGTTGGCGGTGGCGGGAATCCTCTTCTCCTGGCTGTGGTC CTGCGCGTGGACCGCGCCGCCCATCTTCGGGTGGAGCCGGTATTGGCCCCACG GCCTGAAGACGTCGTGCGGCCCGGATGTGTTCAGCGGCAGCTCCGACCCGGG GGTGCAGTCCTACATGGTGGTGCTGATGGTCACGTGCTGCTTCTTCCCCCTCGC CATCATCATCCTCTGCTACCTGCAAGTCTCGCTCGCCATCCGCGCCGTGGCGGC TCAGCAGAAGGAGTCGGAGTCGACGCAGAAGGCGGAGAAGGAGGTGTCCCGC ATGGTGGTGGTGATGATCGTGGCCTACTGCTTCTGCTGGGGGCCCTACACCTTC TTCGCCTGCTTCGCCGCCGCCAACCCCGGCTACGCCTTCCACCCCCTGGCGGC CGCCCTGCCCGCCTACTTCGCCAAGTCGGCCACCATCTACAACCCCATCATCTA CGTCTTCATGAACCGACAGTTCCGGAACTGCATCCTGCAGCTCTTCGGCAAAAA GGTGGACGACGGCTCCGAGGTGTCCACGTCGCGCACGGAGGTCTCCTCCGTCT CCAACTCCTCCGTCTCGCCCGCCTAA |
| >Gallus gallus pinopsin U15762.1 (SEQ ID NO.49) |
| ATGTCCTCCAACAGCTCCCAGGCACCTCCCAATGGGACCCCAGGGCCTTTTGAT GGCCCCCAGTGGCCCTACCAGGCCCCACAGAGCACATATGTGGGGGTGGCCGT GCTGATGGGCACGGTGGTCGCCTGTGCCTCGGTGGTGAATGGGCTGGTCATCG TGGTGTCCATCTGCTACAAAAAGCTCCGCTCCCCACTGAATTACATCCTGGTGA ACCTGGCCGTGGCTGACCTGCTGGTGACGCTGTGCGGCAGCTCCGTCAGCCTC TCCAACAACATCAATGGCTTCTTCGTGTTTGGCAGGAGGATGTGTGAGCTGGA GGGCTTCATGGTCTCCTTGACAGGCATCGTGGGGCTGTGGTCGCTGGCCATCC TGGCCCTGGAGCGCTACGTTGTGGTCTGCAGACCCCTGGGAGATTTCCAGTTC |

**[Table 2-9]**

| |
|---|
| CAACGTCGGCACGCGGTGAGCGGCTGCGCCTTCACGTGGGGCTGGGCGCTGC TCTGGAGCACCCCACCACTGCTGGGCTGGAGCAGCTACGTGCCTGAAGGGTTG AGGACATCGTGCGGGCCTAACTGGTACACGGGTGGCAGCAACAACAACAGCTA CATCCTGTCCCTGTTTGTCACCTGCTTCGTCCTGCCGCTCAGCCTCATCCTCTT CTCCTACACAAACCTGCTGCTGACCCTGCGGGCGGCTGCAGCACAGCAGAAGG AAGCTGACACCACGCAGCGGGCGGAGCGGGAGGTGACGCGGATGGTGATCGT GATGGTGATGGCCTTCCTGCTCTGCTGGCTGCCCTACAGCACGTTTGCCTTGGT GGTGGCCACCCATAAGGGCATCATCATCCAGCCGGTCCTCGCCTCCTTGCCTTC CTATTTCTCCAAGACAGCCACCGTGTACAATCCCATCATCTACGTCTTCATGAAC AAACAGTTCCAGAGCTGCCTGCTGGAAATGCTGTGCTGTGGTTACCAGCCCCA GCGGACAGGAAAAGCCTCACCGGGGACACCTGGGCCCCATGCAGATGTCACTG CAGCGGGGCTGAGGAACAAGGTGATGCCAGCACACCCCGTGTGA |
| >Homo sapiens Opn3 BC036773.1 (SEQ ID NO.50) |
| ATGTACTCGGGGAACCGCAGCGGCGGCCACGGCTACTGGGACGGCGGCGGGG CCGCGGGCGCTGAGGGGCCGGCGCCGGCGGGGACACTGAGCCCCGCGCCCCT CTTCAGCCCCGGCACCTACGAGCGCCTGGCGCTGCTGCTGGGCTCCATTGGGC TGCTGGGCGTCGGCAACAACCTGCTGGTGCTCGTCCTCTACTACAAGTTCCAG CGGCTCCGCACTCCCACTCACCTCCTCCTGGTCAACATCAGCCTCAGCGACCTG CTGGTGTCCCTCTTCGGGGTCACCTTTACCTTCGTGTCCTGCCTGAGGAACGGC TGGGTGTGGGACACCGTGGGCTGCGTGTGGGACGGGTTTAGCGGCAGCCTCTT CGGGATTGTTTCCATTGCCACCCTAACCGTGCTGGCCTATGAACGTTACATTCG CGTGGTCCATGCCAGAGTGATCAATTTTTCCTGGGCCTGGAGGGCCATTACCTA CATCTGGCTCTACTCACTGGCGTGGGCAGGAGCACCTCTCCTGGGATGGAACA GGTACATCCTGGACGTACACGGACTAGGCTGCACTGTGGACTGGAAATCCAAG GATGCCAACGATTCCTCCTTTGTGCTTTTCTTATTTCTTGGCTGCCTGGTGGTG CCCCTGGGTGTCATAGCCCATTGCTATGGCCATATTCTATATTCCATTCGAATGC TTCGTTGTGTGGAAGATCTTCAGACAATTCAAGTGATCAAGATTTTAAAATATGA AAAGAAACTGGCCAAAATGTGCTTTTTAATGATATTCACCTTCCTGGTCTGTTG GATGCCTTATATCGTGATCTGCTTCTTGGTGGTTAATGGTCATGGTCACCTGGTC ACTCCAACAATATCTATTGTTTCGTACCTCTTTGCTAAATCGAACACTGTATACA ATCCAGTGATTTATGTCTTCATGATCAGAAAGTTTCGAAGATCCCTTTTGCAGCT TCTGTGCCTCCGACTGCTGAGGTGCCAGAGGCCTGCTAAAGACCTACCAGCAG CTGGAAGTGAAATGCAGATCAGACCCATTGTGATGTCACAGAAAGATGGGGAC AGGCCAAAGAAAAAAGTGACTTTCAACTCTTCTTCCATCATTTTTATCATCACCA GTGATGAATCACTGTCAGTTGACGACAGCGACAAAACCAATGGGTCCAAAGTT GATGTAATCCAAGTTCGTCCTTTGTAG |
| >Homo sapiens Opn4 BC113558.1 (SEQ ID NO.51) |
| ATGAACCCTCCTTCGGGGCCAAGAGTCCTGCCCAGCCCAACCCAAGAGCCCAG |

**[Table 2-10]**

| |
|---|
| CTGCATGGCCACCCCAGCACCACCCAGCTGGTGGGACAGCTCCCAGAGCAGCA TCTCCAGCCTGGGCCGGCTTCCATCCATCAGTCCCACAGCACCTGGGACTTGG GCTGCTGCCTGGGTCCCCCTCCCCACGGTTGATGTTCCAGACCATGCCCACTAT ACCCTGGGCACAGTGATCTTGCTGGTGGGACTCACGGGGATGCTGGGCAACCT GACGGTCATCTATACCTTCTGCAGGAGCAGAAGCCTCCGGACACCTGCCAACAT GTTCATTATCAACCTCGCGGTCAGCGACTTCCTCATGTCCTTCACCCAGGCCCC TGTCTTCTTCACCAGTAGCCTCTATAAGCAGTGGCTCTTTGGGGAGACAGGCTG CGAGTTCTATGCCTTCTGTGGAGCTCTCTTTGGCATTTCCTCCATGATCACCCT GACGGCCATCGCCCTGGACCGCTACCTGGTAATCACACGCCCGCTGGCCACCT TTGGTGTGGCGTCCAAGAGGCGTGCGGCATTTGTCCTGCTGGGCGTTTGGCTC TATGCCCTGGCCTGGAGTCTGCCACCCTTCTTCGGCTGGAGCGCCTACGTGCC CGAGGGGTTGCTGACATCCTGCTCCTGGGACTACATGAGCTTCACGCCGGCCG TGCGTGCCTACACCATGCTTCTCTGCTGCTTCGTGTTCTTCCTCCCTCTGCTTAT CATCATCTACTGCTACATCTTCATCTTCAGGGCCATCCGGGAGACAGGACGGGC TCTCCAGACCTTCGGGGCCTGCAAGGGCAATGGCGAGTCCCTGTGGCAGCGGC AGCGGCTGCAGAGCGAGTGCAAGATGGCCAAGATCATGCTGCTGGTCATCCTC CTCTTCGTGCTCTCCTGGGCTCCCTATTCCGCTGTGGCCCTGGTGGCCTTTGCT GGGTACGCACACGTCCTGACACCCTACATGAGCTCGGTGCCAGCCGTCATCGC CAAGGCCTCTGCAATCCACAACCCCATCATTTACGCCATCACCCACCCCAAGTA CAGGGTGGCCATTGCCCAGCACCTGCCCTGCCTGGGGGTGCTGCTGGGTGTAT CACGCCGGCACAGTCGCCCCTACCCCAGCTACCGCTCCACCCACCGCTCCACG CTGACCAGCCACACCTCCAACCTCAGCTGGATCTCCATACGGAGGCGCCAGGA GTCCCTGGGCTCGGAGAGTGAGGTGGGCTGGACACACATGGAGGCAGCAGCT GTGTGGGGAGCTGCCCAGCAAGCAAATGGGCGGTCCCTCTACGGTCAGGGTCT GGAGGACTTGGAAGCCAAGGCACCCCCCAGACCCCAGGGACACGAAGCAGAG ACTCCAGGGAAGACCAAGGGGCTGATCCCCAGCCAGGACCCCAGGATGTAG |
| >Homo sapiens Opn5 AY377391.1 (SEQ ID NO.52) |
| ATGGCGTTAAATCACACTGCCCTGCCTCAGGACGAGCGCCTGCCCCATTACCTT CGAGATGGGGATCCTTTTGCTTCCAAACTTTCTTGGGAAGCGGATTTAGTGGCT GGCTTTTACCTAACAATAATTGGGATTCTGTCCACATTTGGAAATGGATATGTCC TTTACATGTCTTCTAGACGAAAGAAGAAGCTGAGACCCGCTGAAATAATGACTA TCAATTTAGCAGTCTGTGATCTGGGGATTTCAGTTGTAGGCAAGCCGTTCACCA TCATCTCTTGCTTTTGTCACCGCTGGGTGTTTGGCTGGATCGGCTGCCGCTGGT ATGGATGGGCTGGATTTTTCTTTGGCTGTGGAAGCCTTATCACCATGACTGCTG TCAGCCTGGATCGATATTTGAAAATCTGCTATTTATCTTATGGGGTTTGGCTGAA AAGAAAGCACGCCTACATCTGCCTGGCAGCCATCTGGGCCTATGCTTCCTTCTG GACCACCATGCCCTTGGTAGGTCTGGGGGACTACGTACCTGAGCCCTTCGGAA CCTCGTGCACCCTGGACTGGTGGCTGGCCCAGGCCTCGGTAGGGGGCCAGGTT TTCATCCTGAACATCCTCTTCTTCTGCCTCTTGCTCCCAACGGCTGTGATCGTG TTCTCCTACGTAAAGATCATTGCCAAGGTTAAGTCCTCTTCCAAAGAAGTAGCT |

**[Table 2-11]**

| |
|---|
| CATTTCGACAGTCGGATCCATAGCAGCCATGTGCTGGAAATGAAACTGACAAAG GTAGCGATGTTGATTTGTGCTGGATTCCTGATTGCCTGGATTCCTTATGCAGTG GTGTCTGTGTGGTCAGCTTTTGGAAGGCCAGACTCCATTCCCATACAGCTCTCT GTGGTGCCAACCCTACTTGCAAAATCTGCAGCGATGTACAATCCCATCATTTAC CAAGTTATTGATTACAAATTTGCCTGTTGCCAAACTGGTGGTTTGAAAGCAACC AAGAAGAAGTCTCTGGAAGGCTTCAGGCTGCACACCGTAACCACAGTCAGGAA GTCTTCTGCTGTGCTGGAAATTCATGAAGAGTGGGAATAA |
| >Homo sapiens Rgr U14910.1 (SEQ ID NO.53) |
| ATGGCAGAGACCAGTGCCCTGCCCACTGGCTTCGGGGAGCTCGAGGTGCTGGC TGTGGGGATGGTGCTACTGGTGGAAGCTCTCTCCGGTCTCAGCCTCAATACCCT GACCATCTTCTCTTTCTGCAAGACCCCGGAGCTGCGGACTCCCTGCCACCTACT GGTGCTGAGCTTGGCTCTTGCGGACAGTGGGATCAGCCTGAATGCCCTCGTTG CAGCCACATCCAGCCTTCTCCGGCGCTGGCCCTACGGCTCGGACGGCTGCCAG GCTCACGGCTTCCAGGGCTTTGTGACAGCGTTGGCCAGCATCTGCAGCAGTGC AGCCATCGCATGGGGGCGTTATCACCACTACTGCACCCGTAGCCAGCTGGCCT GGAACTCAGCCGTCTCTCTGGTGCTCTTCGTGTGGCTGTCTTCTGCCTTCTGGG CAGCTCTGCCCCTTCTGGGTTGGGGTCACTATGACTATGAGCCACTGGGGACAT GCTGCACCCTGGACTACTCCAAGGGGGACAGAAACTTCACCAGCTTCCTCTTC ACCATGTCCTTCTTCAACTTCGCCATGCCCCTCTTCATCACGATCACTTCCTACA GTCTCATGGAGCAGAAACTGGGGAAGAGTGGCCATCTCCAGGTAAACACCACT CTGCCAGCAAGGACGCTGCTGCTCGGCTGGGGCCCCTATGCCATCCTGTATCTA TACGCAGTCATCGCAGACGTGACTTCCATCTCCCCCAAACTGCAGATGGTGCCC GCCCTCATTGCCAAAATGGTGCCCACGATCAATGCCATCAACTATGCCCTGGGC AATGAGATGGTCTGCAGGGGAATCTGGCAGTGCCTCTCACCGCAGAAGAGGGA GAAGGACCGAACCAAGTGA |
| >Homo sapiens Rrh AF012270.1 (SEQ ID NO.54) |
| ATGCTAAGAAATAATTTAGGCAACAGTTCAGACTCTAAAAATGAAGATGGCTCG GTCTTTTCACAGACTGAACACAATATTGTTGCAACTTACTTGATTATGGCAGGTA TGATAAGTATTATCAGCAACATAATAGTTCTGGGCATCTTCATTAAGTACAAGGA ACTTCGGACACCCACAAATGCAATTATTATTAACCTGGCTGTTACTGATATAGGG GTCAGTAGCATTGGCTATCCCATGTCTGCTGCCTCAGATCTGTATGGAAGTTGG AAATTTGGATACGCAGGCTGTCAGGTTTATGCTGGATTGAATATTTTTTTTGGAA TGGCAAGCATTGGATTACTCACGGTCGTGGCTGTGGACCGATACCTGACCATCT GCCTTCCTGACGTAGGGAGAAGAATGACCACCAACACTTACATCGGCTTGATTC TGGGAGCCTGGATCAATGGCCTGTTTTGGGCTTTGATGCCTATCATAGGGTGGG CTAGTTATGCCCCAGATCCTACTGGTGCTACGTGTACCATAAACTGGAGGAAAA ATGATAGATCTTTTGTGTCTTACACCATGACAGTTATTGCGATAAATTTTATTGT GCCCTTGACAGTGATGTTTTACTGCTATTACCATGTCACGCTATCCATTAAACAT |

**[Table 2-12]**

| |
|---|
| CACACTACCAGTGACTGCACTGAGTCCCTCAACAGAGACTGGTCAGATCAGATA GATGTAACAAAGATGTCTGTGATCATGATCTGCATGTTTCTGGTGGCATGGTCC CCTTATTCCATCGTGTGCTTATGGGCTTCTTTTGGTGACCCAAAGAAGATTCCTC CCCCCATGGCCATCATAGCTCCACTGTTTGCAAAATCTTCTACATTCTATAACCC CTGCATTTATGTGGTTGCTAATAAAAAGTTTCGGAGGGCAATGCTTGCCATGTT CAAATGTCAGACTCACCAAACAATGCCTGTGACAAGTATTTTACCCATGGATGT ATCTCAAAACCCATTGGCTTCTGGAAGAATCTGA |
| >Mus musculus Opn3 AF140241.1 (SEQ ID NO.55) |
| ATGTACTCGGGGAACCGTAGTGGCGACCAGGGCTACTGGGAGGACGGGGCGG GCGCCGAGGGCGCAGCACCGGCGGGCACGCGGAGCCCCGCGCCTCTCTTCAG CCCCACCGCGTACGAGCGCCTGGCGCTGCTACTCGGCTGCCTCGCGCTGCTGG GCGTCGGCGGCAACCTGCTGGTGCTGCTTCTCTACTCCAAGTTCCCGAGACTG CGCACGCCCACCCACCTCTTCCTGGTCAACCTGAGCCTGGGCGACCTGCTGGT ATCCCTGTTCGGAGTCACCTTCACCTTCGCGTCGTGCCTGCGGAACGGCTGGG TGTGGGACGCCGTGGGCTGCGCGTGGGACGGGTTTAGCGGCAGCCTCTTTGG GTTCGTTTCCATTACCACCCTCACTGTGCTGGCCTATGAACGTTATATCCGTGTG GTACATGCCAGAGTGATCAACTTTTCCTGGGCCTGGAGGGCCATTACCTATATC TGGCTCTACTCCTTGGCATGGGCAGGAGCACCTCTCCTGGGCTGGAACAGGTA CATCCTAGACATACATGGACTGGGCTGTACCGTGGACTGGAGATCCAAGGATGC CAACGACTCTTCCTTTGTGCTCTTCCTGTTTCTCGGCTGCCTGGTGGTGCCCGT GGGCATCATAGCCCATTGCTACGGCCACATTCTCTATTCTGTTCGAATGCTTCGC TGTGTTGAAGATCTTCAGACCATTCAAGTGATCAAGATGCTAAGATATGAAAAG AAAGTAGCAAAGATGTGCTTTTTGATGGCCTTTGTCTTTCTCACCTGCTGGATG CCTTACATTGTGACCCGCTTCTTGGTGGTCAATGGCTATGGACACCTGGTCACC CCAACTGTGTCTATTGTTTCTTATCTCTTTGCTAAATCGAGCACTGTGTACAACC CAGTTATCTACATCTTCATGAACAGAAAGTTTCGGAGGTCCCTTCTGCAGCTCC TATGCTTCCGCCTGCTGAGATGCCAGCGGCCTGCTAAAAACCTCCCAGCGGCT GAGAGTGAAATGCACATCAGGCCCATCGTGATGTCACAGAAAGATGGGGACAG GCCAAAGAAGAAAGTGACCTTTAACTCTTCCTCTATCATCTTTATCATCACCAGT GATGAGTCCCTGTCAGTCGAGGACAGTGACAGAAGCAGCGCATCTAAGGTCGA TGTCATCCAAGTGCGTCCTCTATAA |
| >Mus musculus Opn4 AF147789.1 (SEQ ID NO.56) |
| ATGGACTCTCCTTCAGGACCAAGAGTCTTGTCAAGCTTAACTCAGGATCCCAGC TTCACAACCAGTCCTGCCCTGCAAGGCATTTGGAACGGCACTCAGAACGTCTC CGTAAGAGCCCAGCTTCTCTCTGTTAGCCCCACGACATCTGCACATCAGGCTGC TGCCTGGGTCCCCTTCCCCACAGTCGATGTCCCAGACCATGCTCACTATACCCT AGGCACGGTGATCCTGCTGGTGGGACTCACAGGGATGCTGGGCAATCTGACGG TCATCTACACCTTCTGCAGGAACAGAGGCCTGCGGACACCAGCAAACATGTTCA |

**[Table 2-13]**

| |
|---|
| TCATCAACCTCGCAGTCAGCGACTTCCTCATGTCAGTCACTCAGGCCCCGGTCT TCTTTGCCAGCAGCCTCTACAAGAAGTGGCTCTTTGGGGAGACAGGTTGCGAG TTCTATGCCTTCTGCGGGGCTGTCTTTGGCATCACTTCCATGATCACCCTGACA GCCATAGCCATGGACCGCTATCTGGTGATCACACGTCCACTGGCCACCATCGGC AGGGGATCCAAAAGACGAACGGCACTCGTCCTGCTAGGCGTCTGGCTTTATGC CCTGGCCTGGAGTCTGCCACCTTTCTTTGGTTGGAGTGCCTACGTGCCCGAGG GGCTGCTGACATCCTGCTCCTGGGACTACATGACCTTCACACCCCAGGTGCGT GCCTACACCATGCTGCTCTTCTGCTTTGTCTTCTTCCTCCCCCTGCTCATCATCA TCTTCTGCTACATCTTCATCTTCAGGGCCATCCGAGAGACAGGCCGGGCCTGTG AGGGCTGCGGTGAGTCCCCTCTGCGGCAGAGGCGGCAGTGGCAGCGGCTGCA GAGTGAGTGGAAGATGGCCAAGGTCGCACTGATTGTCATTCTTCTCTTCGTGCT GTCCTGGGCTCCCTACTCCACTGTGGCTCTGGTGGCCTTTGCTGGATACTCGCA CATCCTGACGCCCTACATGAGCTCGGTGCCAGCCGTCATCGCCAAGGCTTCTGC CATCCACAATCCCATTATCTACGCCATCACTCACCCCAAGTACAGGGTGGCCAT TGCCCAGCACCTGCCTTGCCTTGGGGTGCTTCTCGGTGTATCAGGCCAGCGCA GCCACCCCTCCCTCAGCTACCGCTCTACCCACCGCTCCACATTGAGCAGCCAGT CCTCAGACCTCAGCTGGATCTCTGGACGGAAGCGTCAAGAGTCCCTGGGTTCT GAGAGTGAAGTGGGCTGGACAGACACAGAAACAACCGCTGCATGGGGAGCTG CCCAGCAAGCAAGTGGACAGTCCTTCTGCAGTCAGAACCTAGAAGATGGAGAA CTCAAGGCCTCTTCCAGCCCCCAGGTACAGAGATCTAAGACTCCCAAGGTGCCT GGACCCAGTACCTGCCGCCCTATGAAAGGACAGGGAGCCAGGCCAAGTAGCCT AAGGGGTGACCAGAAAGGCAGGCTTGCTGTGTGCACAGGCCTCTCAGAGTGTC CCCATCCCCATACATCCCAGTTTCCCCTTGCTTTCCTAGAGGATGATGTGACTCT CAGACATCTGTAG |
| >Mus musculus Opn5 AY318865.1 (SEQ ID NO.57) |
| ATGGCCTTGAACCACACTGCCCTACCTCAGGATGAGCGCCTGCCCCACTATCTT CGAGACGAGGACCCTTTTGCTTCCAAACTTTCCTGGGAAGCGGATTTAGTGGCT GGCTTTTACCTAACAATAATCGGGATTCTCTCTACATTTGGAAATGGGTATGTCC TTTATATGTCTTCTAGACGCAAGAAGAAGCTGAGACCTGCGGAAATAATGACTA TCAATTTAGCAGTCTGTGATCTGGGGATATCAGTTGTAGGCAAGCCGTTCACCA TCATCTCTTGCTTCTGCCACCGCTGGGTGTTTGGCTGGTTTGGCTGCCGCTGGT ATGGCTGGGCTGGATTTTTCTTTGGCTGTGGAAGCCTGATTACCATGACTGCTG TCAGCCTGGACCGCTATCTGAAGATCTGTTATCTGTCTTATGGGGTCTGGCTGA AGAGAAAGCATGCCTACATCTGCCTGGCAGTCATCTGGGCTTATGCTTCCTTCT GGACCACCATGCCCTTGGTGGGCCTGGGGGACTATGCACCTGAGCCCTTCGGA ACCTCATGCACCCTGGACTGGTGGCTGGCCCAGGCTTCAGGTGGGGGTCAGGT GTTCATCCTGAGCATCCTCTTCTTCTGCCTCCTGCTGCCAACGGCTGTGATTGT TTTCTCATATGCTAAGATCATCGCCAAGGTGAAGTCTTCTTCTAAAGAGGTAGC CCATTTCGACAGTCGAATCCATAGCAGCCATGTACTTGAGGTGAAGCTGACCAA GGTGGCAATGCTGATTTGTGCTGGTTTCCTGATTGCCTGGATTCCTTATGCGGT |

**[Table 2-14]**

| |
|---|
| CGTCTCTGTGTGGTCAGCCTTTGGAAGGCCAGACTCCATCCCCATACAGCTCTC CGTGGTGCCCACACTCCTTGCAAAATCAGCAGCGATGTACAATCCAATCATCTA CCAGGTCATTGATTACAGATTTGCCTGTTGCCAGGCTGGTGGTTTGAGAGGAAC GAAGAAGAAATCTTTGGAAGACTTTAGGCTGCATACTGTAACCGCAGTCAGGAA GTCTTCTGCTGTGCTGGAGATCCATCCAGAGAGCAGTTCCAGATTTACTAGTGC CCATGTTATGGATGGAGAGAGTCACAGTAATGATGGTGACTGTGGCAAGAAATA A |
| >Mus musculus Rgr AF076930.1 (SEQ ID NO.58) |
| ATGGCAGCAACCAGGGCCCTGCCTGCTGGTTTGGGGGAACTTGAGGTGCTGGC TGTAGGAACAGTCCTGCTGATGGAAGCGCTCTCTGGCATCAGCCTCAATGGCCT GACCATCTTCTCTTTCTGCAAGACTCCAGATCTGCGGACTCCCAGCAACCTGCT GGTACTGAGCCTGGCCCTGGCAGACACTGGGATCAGCCTGAATGCCCTTGTTG CAGCGGTATCCAGCCTCCTCCGGCGCTGGCCACATGGTTCAGAGGGCTGCCAG GTTCATGGTTTCCAGGGATTTGCAACAGCACTGGCCAGCATCTGTGGCAGTGC AGCCGTTGCCTGGGGACGTTATCACCACTACTGCACCCGCAGACAGTTGGCAT GGGACACGGCCATCCCTCTGGTGCTGTTTGTGTGGATGTCATCTGCCTTCTGGG CATCCCTGCCCCTGATGGGCTGGGGCCACTATGACTATGAGCCTGTGGGGACAT GCTGTACACTGGACTATTCGAGGGGTGACAGAAACTTCATCAGTTTTCTCTTCA CCATGGCTTTTTTCAACTTCCTCGTACCCCTGTTCATCACACACACTTCATACCG GTTCATGGAGCAGAAATTCTCCAGGAGTGGCCATCTCCCGGTGAATACTACTCT TCCAGGCAGAATGCTGCTGCTTGGCTGGGGCCCCTATGCCCTCCTGTACCTATA CGCAGCCATCGCAGATGTGAGCTTCATCTCTCCTAAACTACAGATGGTGCCTGC TCTCATCGCCAAAACCATGCCCACAATCAACGCCATCAACTATGCGTTGCACAG GGAGATGGTCTGTAGAGGAACCTGGCAGTGTCTGTCTCCACAAAAAAGCAAGA AGGACCGAACCCAGTGA |
| >Mus musculus Rrh AF012271.1 (SEQ ID NO.59) |
| ATGCTGAGTGAGGCTTCGGATTTTAACAGCTCGGGCTCTAGGAGCGAAGGTTC TGTCTTTTCAAGGACTGAGCACAGCGTCATAGCGGCTTACCTGATTGTGGCAGG TATCACGAGCATTCTCAGCAACGTAGTAGTCCTGGGTATCTTCATCAAGTACAA GGAGCTGCGGACACCCACCAATGCAGTGATTATCAACCTGGCTTTCACGGATAT AGGGGTCAGTAGCATCGGCTATCCCATGTCTGCGGCTTCAGATCTGCATGGAAG TTGGAAATTTGGACATGCAGGCTGTCAGATTTATGCAGGACTGAATATCTTCTTT GGGATGGTGAGCATTGGCTTACTCACGGTTGTAGCTATGGATCGGTACCTGACC ATCAGTTGCCCTGATGTAGGACGAAGAATGACCACCAACACTTACCTCAGCATG ATCCTGGGCGCCTGGATCAATGGCCTGTTTTGGGCTTTGATGCCCATCATAGGG TGGGCTAGTTATGCCCCGGATCCTACAGGGGCCACGTGTACCATAAACTGGCGG AATAATGATACCTCGTTCGTGTCTTACACCATGATGGTTATCGTGGTGAACTTCA TTGTGCCCTTGACAGTGATGTTTTACTGTTACTACCACGTCAGTCGGTCCCTGA |

**[Table 2-15]**

| |
|---|
| GACTCTACGCCGCTAGTGACTGCACTGCACACCTCCACAGAGACTGGGCAGAC CAGGCAGATGTAACAAAGATGTCTGTGATAATGATACTGATGTTTCTGCTGGCA TGGTCCCCTTATTCCATCGTGTGCTTGTGGGCTTGTTTTGGAAACCCCAAAAAG ATTCCTCCTTCAATGGCTATCATAGCTCCACTGTTTGCAAAATCCTCTACGTTCT ACAACCCCTGTATTTACGTGGCTGCGCATAAGAAGTTTCGGAAGGCCATGTTGG CCATGTTTAAATGCCAACCTCACCTAGCAGTGCCTGAGCCAAGTACTCTACCAA TGGATATGCCTCAGAGCTCATTGGCTCCTGTGAGGATCTGA |
| >Gallus gallus VAL opsin GQ280390.1 (SEQ ID NO.60) |
| ATGGATGTATTCAGAGCACTTGGAAATGAGTCGCTGTTGTCGAACTCCTCTGGT CCTGCTCGATGGGATCCCTTCCACCATCCCTTGGACTCCATCCAGCCCTGGCAC TTCAGGCTTGTGGCAGCAGTAATGTTCGTGGTGACCTCCCTGTCTCTTGCGGAG AACCTGGCTGTGATCCTGGTAACTTTTAAGTTCAAGCAGCTGAGACAACCTGTC AATTATGTTATAGTGAATTTATCTGTGGCTGATTTCCTGGTCTCTTTGACTGGTG GCACCATCAGCTTTTTAGCCAACCTAAAAGGCTATTTCTATATGGGGCACTGGG CTTGTGTACTGGAAGGATTTGCTGTCACGTTTTTTGGCATTGTGGCTCTCTGGT CTCTTGCTCTTTTGGCTTTTGAGCGGTACATTGTGATCTGCCGCCCAGTGGGAA ATATGCGCTTGAGAGGGAAGCATGCTGCCCAAGGTATTGCTTTTGTGTGGACCT TTTCCTTCATTTGGACCATTCCACCAACCATGGGTTGGAGCAGCTACACCACCA GTAAGATTGGAACTACTTGTGAGCCTAATTGGTATTCAGGAGCTTATAATGATCG TTCCTACATTATTGCATTCTTCACCACCTGTTTTATAGTGCCTTTATTGGTAATTC TGGTGTCGTATGGGAAACTGCTGCAGAAGCTAAGAAAGGTGTCAAATACACAA GGCAGGTTGAGAACTGCCAGGAAACCTGAAAGACAAGTGACTAGAATGGTGGT TGTTATGATCATTGCTTTTCTAATCTGCTGGATGCCATATGCCGTCTTTTCTATCC TAGCCACTGCATACCCTTCCATTGAACTGGATCCTCACCTGGCAGCAATTCCAG CTTTCTTTTCCAAAACAGCTACTGTTTATAATCCAATTATTTATGTTTTTATGAAC AAACAGTTCAGGATGTGTCTGATTCAGATGTTCAAGTGTAGTGCCATAGAGACT GCGGAGTCCAACATGAACCCAACTTCAGAGAGAGCAACACTCACCCAAGACAA AAGAGACAGTCAGTTGTCTGTCATGGCAGTACGAAGCACCATTTCTTAG |
| >Gallus gallus Opn3 AB436160.1 (SEQ ID NO.61) |
| ATGCACTCGGGGAACGGCACGGGGGCGACGAGCCGCCCGCAGCTCGCGGCGG CGGGGCACGAGGTGCCGGGCGAGCGTCCGCTCTTCAGCGCCGGCACCTACGA GCTGCTGGCGCTGCTCATCGCCACCATCGGCACGCTGGGCGTGTGCAACAACT TGCTGGTGCTGGTGCTCTACTACAAGTTCAAGCGGCTCCGCACGCCCACCAAC CTGTTCCTCGTCAACATCAGCCTCAGCGACCTGCTGGTGTCCGTCTGCGGCGT CAGCCTCACCTTCATGTCCTGCCTGCGGAGCCGCTGGGTGTGGGACGCCGCCG GCTGCGTCTGGGACGGCTTCAGCAACAGCCTCTTCGGGATCGTTTCAATTATGA CTCTCACTGTTCTTGCCTATGAACGCTACATTCGAGTAGTCCATGCAAAAGTGA TTGACTTCTCTTGGTCTTGGCGGGCTATCACATACATTTGGCTTTATTCGTTAGC |

**[Table 2-16]**

| |
|---|
| CTGGACAGGAGCACCTCTTTTGGGCTGGAACAGATACACACTGGAAATTCATG GATTAGGTTGCTCAATGGACTGGAAGTCAAAAGATCCCAATGATACCTCCTTTG TGCTACTTTTCTTCCTTGGCTGTCTGGTAGCACCTGTTGTGATCATGGCCTATTG CTACGGCCATATCTTATACGCAGTAAGAATGCTTCGATGTGTGGAAGATTTTCAG ACTTCTCAAGTGATTAAACTTCTAAAATATGAAAAGAAAGTGGCTAAAATGTGTT TCTTAATGATCTCTACCTTCCTTATTTGTTGGATGCCTTATGCAGTCGTCTCCCT CCTAGTAACATATGGCTATAGCAACCTTGTAACTCCCACAGTAGCTATAATCCCA TCTTTCTTTGCCAAATCAAGTACTGCTTATAATCCAGTCATCTACATCTTCATGA GTAGAAAGTTTCGACAATGCCTTTTGCAACTCCTGTGCTTTCGCCTGATGAGAT TCCAGAGGATCATGAAAGAGCCCTCAGGAGCAGGGAATGTCAAACCGATACGT CCGATAGTAATGTCTCAGAAAGTAGGGGACAGGCCAAAGAAGAAAGTGACATT CAGCTCTTCCTCCATCATTTTTATTATCGCCAGTGATGACACTCAGCAAATAGAT GACAACAGCAAACACAATGGGACAAAAGTAAATGTCATCCAAGTAAAACCACTA TAG |
| >Gallus gallus TMT opsin AB519059.1 (SEQ ID NO.62) |
| ATGAACCACACTTGGACATATAACCTGAGCTTTGGTGCACCTACAGATCCTGTT GAGCCAAGAGCTGGACTGTCACGAAATGGGCACACAGTAGTGGCTGTTTTTCT TGGATTTATCTTGTTTTTTGGTTTCCTGAATAACTTGATTGTCCTCATCCTCTTCT GCAAGTTTAAAACCCTCCGTAACCCTGTCAACATGCTTCTCCTGAACATCAGTA TCAGTGACATGTTAGTGTGCATCTCAGGCACTACCCTGAGTTTTGCATCTAACA TCCATGGCAAGTGGATTGGAGGGGAGCATGGTTGTAGGTGGTATGGCTTTGTC AATTCCTGCTTTGGCATCGTGTCCCTGATCTCCCTGGCTGTCCTGTCCTACGAA CGCTACAGCACCCTTACCCTGTGTAACAAGCGCAGCGATGACTATCGGAAAGCG CTGCTGGCAGTTGGCGGTTCTTGGGTTTATTCCTTGCTCTGGACTGTGCCACCT CTGCTTGGATGGAGCAGTTATGGGATAGAAGGTGCAGGCACCAGCTGTTCAGT GCGCTGGTCCTCTGAGACAGCTGAGTCCACGTCCTATATCATCTGTCTCTTCAT ATTCTGCTTGGTCATCCCTGTAATGGTCATGATGTACTGCTATGGTCGGCTCCTT TATGCTGTCAAACAGGTTGGAAAAATCCACAAGAACACTGCTCGCAAAAGAGA ATACCACGTACTGTTCATGGTGATCACTACAGTTATCTGTTATCTTGTATGTTGG ATTCCCTATGGAGTTATAGCACTACTTGCAACATTTGGTAAGCCAGGTGTGGTG ACTCCAGTTGCAAGCATCATACCTTCCATCTTAGCAAAAAGCAGCACTGTTTGC AATCCCATTATCTACATACTTATGAATAAGCAGTTTTACAAATGCTTTCGCCAGC TTTTCCACTGTCAACCTCCTTCCTCAACTGATGGAGAGCCCACCTGCCACTCCA AGGTAACCGTTATCCAGCTGAATCAGAAAACAGATGGTGGAAAGTTGTGCAATA ATAAACCACGTCCAGAAACAGACAATAAAGTCACTTCTCTTCTGCACCCAGAAC CAGGCCTGGAGCCAGCTGCAAAAACTGTACCACCAATGTAA |
| >Gallus gallus Opn4x EU124630.1 (SEQ ID NO.63) |
| ATGGATTTGAACATGGGCACCCAGCCACATTCTGTGACCAAGTCAGAGATCCCA |

**[Table 2-17]**

| |
|---|
| GACCATGTTCTTTACACGGTGGGGACGTGTGTTCTTGTTATTGGCTCCATTGGC ATCATAGGAAACCTCCTAGTTCTCTATGCATTTTACAGCAACAAGAAGCTGAGG ACACCCCAGAACTTCTTTATAATGAATTTGGCTGTGAGTGACTTCCTGATGTCA GCTTCTCAGGCCCCCATATGCTTTATCAACAGCTTGCACAGAGAGTGGATCCTT GGAGATATAGGTTGTGACTTGTATGCTTTCTGCGGGGCGCTCTTTGGAATAACC TCGATGATGACTCTGTTAGCTATTTCTGTTGACCGCTATTTGGTGATCACCAAGC CCCTGCGCTCCATCCAGTGGACCTCCAAGAAGCGCACCATACAGATTATTGCTG CTGTGTGGCTGTACTCGCTGGGGTGGAGCGTAGCCCCACTCCTTGGGTGGAGT TCCTACGTGCCTGAGGGCTTGATGATATCCTGTACATGGGACTATGTAACCTACT CCCCTGCAAATAGAAGTTACACCATGATTTTATGCTGCTGCGTATTTTTTATTCC CCTGATAATAATACTCCATTGCTATTTATTTATGTTCTTGGCCATAAGAAGTACTG GCAGAGATGTCCAAAAGCTTGGGTCCTGCAGCCGCAAATCCTTCCTCTCTCAGT CCATGAAGAATGAATGGAAACTGGCAAAAATTGCGTTTGTGGTCATCATTGTGT ATGTCTTGTCCTGGTCTCCATATGCTTGTGTCACCTTGATTGCCTGGGCAGGCC GAGGAAACACTCTAACGCCGTATTCCAAATCTGTGCCAGCTGTTATTGCTAAAG CCTCTGCAATCTACAACCCCATCATATATGCAATAATTCACCCGAGATACAGAAA AACCATCCACAATGCTGTGCCCTGCTTAAGGTTCCTAATACGAATATCAAAGAAT GATCTCCTGAGAGGCTCCATAAATGAATCATCATTCAGGACCTCTCTCTCCAGC CACCAGTCTCTTGCTGGAAGGACCAAGAACACTTGTGTTTCATCAGTTTCCACT GGAGAAGCTAACTGGAGTGATGTAGAGCTTGACACTGTAGAACCAGCTCATGA GAAACTGCAGCCTCGCCGAAGTCATTCCTTCTCATCAAGTCTGAGACAGAAGA GAGACCTGCTTCCAGACAGCTATAGCTGCAGTGAAGAAACTGAAGAAAAGGTT TCGCTGTCATCCAGCTACCTGGAGAAGGTGCTGGGGCGATCAGCCTTCCCCAG CTCCCCTGTAGCACTTGTGACCAGCTCCCTCCGAGCAGCCTCTCTTCCCGTTGG TTTGAACAGCAGCAGTGCCAGCAGAGGAGCAGGCTCAGATATCTCACAGATGA AAACTGAAGAAAGTCATAATAATGGAGGCCTGGACTCCATTGTTAGTAATACAG TCCCTCAAATCATTATCATTCCTACCTCAGAGACAAACCTATTTCAAGAGGAACC TGAAGAGGAAGAGACTGAATTATTCCACTTCCATGACAAAAAGAACAATCTGCT GGATTTGGAAGGGCTTAGCTCATCTACAGAGTTCCTCGAAGCTGTTGAGAAATT TCTATCATAA |
| >Gallus gallus Opn4m AB295599.1 (SEQ ID NO.64) |
| ATGGACCTGCCTCCTAGAGCCCCAACAAAGATGACAGTGAAAGATGTTCGTGG TGCTTTTCCTACGGTGGACGTCCCAGACCATGCCCATTATACAATTGGAACAGT CATTCTTATCGTGGGGATCACAGGAACTCTGGGTAATTTCCTGGTCATCTATGCT TTCTGCAGGAGTAGGACCCTTCAGAAACCAGCCAATATATTCATCATCAATCTAG CTGTTAGTGACTTCCTTATGTCCATTACACAGTCTCCAGTATTTTTCACCAACAG TCTCCATAAACGTTGGATTTTTGGTGAGAAAGGCTGTGAGCTGTATGCCTTCTG CGGAGCTCTTTTTGGCATTACATCCATGATCACTTTGATGGTGATTGCCTTGGA CAGATACTTTGTCATCACAAAACCTCTGGCTTCTGTTCGAGTGATGTCAAAGAA GAAGGCCCTTATAATCCTGGTAGGCGTCTGGCTGTACTCTTTGGCTTGGAGCCT |

**[Table 2-18]**

| |
|---|
| CCCACCCTTCTTTGGATGGAGTGCCTATGTTCCTGAAGGCCTGCTGACTTCCTG TTCCTGGGATTACATGACTTTCACACCATCAGTCCGTGCCTACACAATGCTGCT TTTCTGCTTTGTCTTCTTCATTCCTTTGATTGCTATCATATACAGCTATGTCTTTA TATTTGAGGCTATCAAGAAGGCCAACAAGTCTGTACAGACATTTGGTTGCAAAC ATGGAAACAGAGAGCTCCAGAAACAGTATCATAGGATGAGGAATGAGTGGAAG TTGGCCAAGATTGCACTGATCGTCATCTTGCTTTATGTCATTTCCTGGTCACCAT ACTCTGTTGTTGCCCTGGTAGCTTTTGCTGGGTATTCTCATGTTCTAACACCTTT CATGAATTCAGTACCAGCTGTGATTGCCAAAGCTTCTGCCATCCATAACCCCATT ATTTATGCCATCACTCACCCTAAATATAGAACAGCCATTGCAACATATGTACCCT GCCTTGGATTCCTGCTGAGAGTTTCTCCTAAAGAGTCACGATCCTTTAGCAGTT ATCCCTCCTCCAGACGAACCACCATAACCAGCCAGTCTTCTGAGACAAGTGGGC TGCAGAAAGGAAAAAGGCGACTATCTTCCATCTCTGATAGTGAATCGGGCTGTA CCGATACAGAAACTGATATCACCAGTATGATCTCCAGACCTGCTAGCAGCCAGG TTTCCTATGAAATGGGGGAAGATACAACCCAAACTAGTGACCTAGGAGGCAAAC CTAAAGTGAAAAGCCATGATTCTGGGATTTTCAGGAAGACAGTTGTAGATGCAG ATGAAATACCCATGGTGGAAATCAATGACACAGAGCATTCTGCTACATCTACTT GCAAAACATCTGAAAAATGCAATGTGGAGGAAATTCAGAGAAGTGAGAGCCTG AGTGGTATTGGACTAAGAGAAGGAGAGTCTCGCCATAGAACATCCGCATCTCAG ATACCCAGCATTATAATAACATACAGCAACGTACAAGGAGTAGAGCTGCCTTCA GGATACAGTGCTGGCTTCCTGCACCCAAAGAACAAAAGCCACAAACAGAACAA GAGCTCCAACAGCTGA |
| >Gallus gallus Opn5m AB368182.1 (SEQ ID NO.65) |
| ATGAGTGGGATGGCATCGGACTGCAACTCCTCCTCTCAGGAAGAATACCTCCCA CACTACGTGCAGCAGGAAGACCCCTTTGCATCAAAGCTGTCTAGAGAAGCTGA CATCATAGCGGGGTTTTATTTGACAGTAATTGGGATTCTTTCAACTCTGGGAAA CGGGTATGTTATCTTTATGTCCTCAAAGCGGAAAAAGAAGTTAAGACCCGCTGA GATAATGACTGTTAATTTAGCGGTGTGTGATCTGGGCATCTCAGTTGTAGGAAA ACCCTTCAGTATCATTTCCTTCTTTTCTCACCGCTGGATCTTTGGATGGATGGGC TGCCGCTGGTACGGATGGGCTGGCTTCTTCTTTGGCTGTGGGAGCCTTATTACC ATGACAGCCGTCAGCCTGGATAGATACTTAAAAATCTGTCACTTGGCTTATGGT ACCTGGCTTAAGAGACACCATGCCTTTATCTGCCTGGCACTAATCTGGGCCTAT GCGACGTTCTGGGCTACAGTGCCTTTTGCTGGTGTGGGGAGCTACGCTCCTGA GCCGTTTGGGACCTCGTGCACCCTGGACTGGTGGCTGGCGCAGGCTTCAGTGG CTGGGCAGGCTTTTGTTCTCAGCATTCTTTTCTTTTGCCTCCTGTTTCCAACTGC AGTGATTGTGTTCTCCTATGTTAAAATTATTTAAAGTCAAGTCGTCCACCAAA GAAGTTGCTCACTATGACACCAGGATCCAGAACAGCCACATACTTGAAATGAAG CTGACCAAGGTGGCAATGCTGATCTGTGCCGGATTCCTCATTGCTTGGATCCCC TACGCCGTGGTCTCTGTGTGGTCAGCCTTTGGACAGCCGGATTCCGTTCCCATT CAATTTTCGGTGGTACCAACTCTGCTTGCCAAGTCGGCTGCCATGTACAACCCA ATTATTTACCAGGTCATTGATTGCAAATTTGCCTGCTGCCGGTCAGGTGGGCCG |

**[Table 2-19]**

| |
|---|
| AAGACACTGCAGAAGAAGAGCTCTTTGAAGGAATCGAGGATGTACACCTTATCT TCACACAGAGATTCAGCGGCACTGAGCGGAACCCAACTGGAAGTCTGA |
| >Gallus gallus Opn5L2 AB368183.3 (SEQ ID NO.66) |
| ATGGAGGAGCAGTACATTTCCAAACTGCACCCGGTAGTGGACTACGGAGCTGG GGTCTTTCTTCTGATCATAGCCATCCTGACAATCCTTGGAAATTCCGCTGTCCTT GCTACAGCAGTGAAACGCTCTTCCCTCCTGAAGTCACCAGAGCTGCTTACAGTC AACCTGGCAGTAGCAGATATTGGGATGGCAATCAGCATGTACCCGCTGGCAATT GCATCTGCCTGGAACCACGCTTGGCTGGGAGGAGATGCGTCCTGCATATATTAC GCCCTGATGGGTTTCCTTTTTGGTGTCTGCAGCATGATGACGCTGTGTGCCATG GCTGTGATTCGATTCCTTGTTACCAATTCATCCAAATCTAACAGTAACAAAATCT CTAAGAATACTGTTCACATCCTGATTACTTTCATCTGGCTCTACTCCTTGCTCTG GGCCATTCTACCCTTGGTAGGCTGGGGCTACTATGGCCCCGAGCCATTTGGCAT CTCTTGTACGATAGCCTGGAGCAAGTTCCACAGCTCCTCCAATGGCTTTTCATT CATCCTGAGCATGTTCCTCCTGTGCACAGTCCTGCCTGCTCTGACCATCGTCGC CTGTTACTTGGGAATTGCCTGGAAGGTTCATAAAGCATACCAAGAGATCCAGAA TATCAACAGGATTCCTCACGCAGCTAAACTGGAGAAGAAGCTGACATTGATGGC TGTGCTCATCTCAGTTGGGTTCCTGAGCGCATGGACGCCGTATGCAGCAGCCA GCTTCTGGTCCATATTTAACTCCAGCGATTCCCTGCAGCCCATTGTCACGCTGC TGCCGTGTCTGTTTGCCAAATCTTCAACCGCGTATAACCCTTTTATTTACTACAT CTTCAGCAAAACTTTCCGTCATGAAATCAAACAGCTGCAGTGCTGCTGGGGCTG GCGAGTTCATTTCTTCAGCGCTGATAACTCTGCAGAAAATTCCGTCTCGATGAT GTGGAGTGGGAGAGATAACATACGTCTCTCCCCCACTGCAAAGGTGGAGAGCC AAGGAGCTGCAAGGCACTGA |
| >Gallus gallus RrhAY339626.1 (SEQ ID NO.67) |
| ATGCATTGGAATGATTCAGCCAACTCCTCCGAAAGTGATGCTGAAGCTCATTCT GTCTTTACACAGACTGAGCACAACATAGTCGCAGCTTACTTAATAACAGCAGGA GTGATAAGCATTTTCAGCAATATTGTTGTGTTAGGCATCTTTGTGAAGTACAAAG AGTTTCGGACAGCAACCAATGCAATTATTATCAACCTGGCTTTCACTGACATTG GTGTTAGTGGCATTGGCTACCCCATGTCTGCTGCTTCAGACCTGCATGGAAGCT GGAAGTTTGGATATACTGGATGCCAGATCTATGCTGCCTTAAATATATTTTTTGG GATGGCGAGTATTGGTTTGCTCACTGTTGTTGCAGTTGACCGTTACCTGACAAT CTGCAGGCCTGACATAGGAAGAAGAATGACTACCCGTAACTATGCCGCTCTAAT CCTGGCTGCGTGGATCAACGCAGTCTTTTGGGCGTCCATGCCTACTGTAGGCTG GGCTGGCTATGCTTCAGATCCGACTGGAGCAACTTGCACAGCCAATTGGAGGA AAAATGATGTGCCCTTTGTTTCCTACACAATGAGTGTAATTGCTGTTAATTTTGT TGTACCCTTAACAGTCATGTTTTACTGTTATTACAATGTTTCCCGGACAATGAAA CAATACACCAGCAGTAACTGCCTGGAGAGCATCAACATGGATTGGTCTGACCAA GTAGATGTAACAAAGATGTCTGTTGTGATGATTGTCATGTTCCTGGTGGCATGG |

**[Table 2-20]**

| |
|---|
| TCTCCCTATTCCATTGTGTGTCTATGGTCTTCCTTTGGAGACCCGAAGAAAATTT CTCCTGCAATGGCCATCATAGCTCCTTTATTTGCAAAATCCTCCACGTTCTATAA TCCCTGCATTTACGTCATTGCAAACAAAAAGTTTCGGAGAGCAATCCTGGCAAT GGTGCGATGTCAGACAAGACAAGAAATAACTATAAGCAATGCCTTGCCCATGAC TGTTTCTCTGAGTGCATTGACGTCATAA |
| >Gallus gallus Rgr AY339627.1 (SEQ ID NO.68) |
| ATGGTCACTTCACATCCTCTCCCTGAAGGCTTCACTGAAATAGAGGTGTTTGCC ATCGGCACTGCCCTACTGGTAGAAGCCCTGCTTGGTTTCTGTCTGAATGGCTTG ACGATTATTTCATTCCGAAAAATCAAAGAGCTCCGAACACCCAGCAACCTGCTG GTTCTCAGCATCGCACTGGCTGACTGCGGGATATGCATCAATGCATTCATCGCT GCCTTTTCCAGCTTCCTCAGGTACTGGCCCTATGGCTCCGAGGGCTGTCAGATT CATGGATTCCAGGGCTTCTTGACAGCACTAGCCAGCATCAGCTCCAGCGCAGC AGTTGCCTGGGACCGGTATCATCACTACTGTACAAGGAGCAAGCTGCAGTGGA GCACAGCCATCTCCATGATGGTGTTCGCGTGGCTGTTCGCTGCCTTCTGGGCCA CAATGCCCTTGCTGGGCTGGGGGGAGTATGACTATGAGCCCCTCCGAACCTGC TGCACCCTGGACTACAGCAAAGGGGACAGAAACTATATCACATTCCTTTTTGCT CTGTCCATTTTCAATTTCATGATCCCGGGCTTCATCATGATGACGGCCTATCAGT CCATACATCAGAAGTTCAAGAAAAGTGGGCACTATAAGTTTAACACTGGCTTAC CGTTGAAGACGCTGGTCATTTGCTGGGGTCCCTACTGCCTTCTGAGTTTCTATG CAGCAATTGAAAACGTGATGTTCATTTCACCAAAATACCGAATGATTCCTGCCAT TATTGCCAAGACAGTGCCAACCGTGGACAGCTTTGTGTATGCCCTGGGCAATGA GAACTACAGAGGAGGAATATGGCAGTTCCTCACAGGACAGAAGATTGAGAAAG CAGAGGTTGATAGCAAAACTAAGTAA |

In a certain embodiment, a protein of the present disclosure is a chimeric protein containing a portion of a G protein-coupled receptor rhodopsin and portions of another G protein-coupled receptor, a metabolic glutamate receptor, and an adrenergic receptor and has a seven-transmembrane structure.

In one embodiment of the present disclosure, an amino acid sequence encoding a protein of the present disclosure may include
1) an amino acid sequence including the above mentioned modification in an amino acid sequence represented by any one of SEQ ID NOs: 1 to 34;
2) an amino acid sequence including a sequence other than a site of the modification having an identity of at least about 80% to the sequence in 1), and encoding a protein having substantially the same biological activity as that of a protein obtained from the sequence in 1);
3) an amino acid sequence having one or more mutations in the sequence in 1) other than the site of the modification and encoding a protein having substantially the same biological activity as that of a protein obtained from the sequence in 1);
4) an amino acid sequence including the modification in an amino acid sequence encoded by a nucleic acid to which a nucleic acid encoding the sequence in 1) hybridizes; or
5) an amino acid sequence including the modification in an amino acid sequence encoded by an allelic variant of the nucleic acid encoding the sequence in 1). In one embodiment, the amino acid sequence encoding the protein of the present disclosure can be an amino acid sequence in which a sequence other than a site of the modification has an identity of at least about 80%, at least about 85%, at least about 90%, at least about 95%, at least about 98%, at least about 99%, or 100% to the sequence in 1) and which encodes a protein having substantially the same biological activity as that of a protein obtained from the sequence in 1).

In one embodiment of the present disclosure, a protein of the present disclosure can include a modification of an amino acid corresponding to position 122 when aligned with SEQ ID NO: 1 in an amino acid sequence of an opsin, and the amino acid corresponding to position 122 when aligned with SEQ ID NO: 1 can be, for example, an amino acid corresponding to E at position 122. A modified opsin of the present disclosure can be one in which such an amino acid has been modified.

One embodiment of the present disclosure can include a modification to glutamine of an amino acid corresponding to position 122 when aligned with SEQ ID NO: 1. An amino acid corresponding to position 122 of an opsin to be used in the present disclosure may be an amino acid corresponding to E as described above, which can be modified to glutamine to facilitate a decay of a Meta II and accelerate a photocycle reaction.

In one embodiment of the present disclosure, opsin of the present disclosure can include modifications of amino acids corresponding to an amino acid belonging to a portion of a N-terminal domain (positions 1 to 11) and an amino acid belonging to a portion of an extracellular third loop (positions 278 to 285) when aligned with SEQ ID NO: 1 in the amino acid sequence of the opsin. In one embodiment, amino acids corresponding to an amino acid belonging to a portion of a N-terminal domain (positions 1 to 11) and an amino acid belonging to a portion of an extracellular third loop (positions 278 to 285) when aligned with SEQ ID NO: 1 can be modified to cysteine. For example, the amino acids corresponding to an amino acid belonging to a portion of a N-terminal domain (positions 1 to 11) and an amino acid belonging to a portion of an extracellular third loop (positions 278 to 285) when aligned with SEQ ID NO: 1 can be amino acids corresponding to positions 2 and 282 in an amino acid sequence of an opsin when aligned in SEQ ID NO: 1 and preferably can include modification of these amino acids to cysteine. Such a modification can enhance a thermal stability of an opsin of the present disclosure. In another embodiment, opsin of the present disclosure can include modifications of amino acids corresponding to any one, two, three, four, five, six, seven, eight, nine, ten, or eleven of amino acids at position 1, 2, 3, 4, 5, 6, 7, 8, 9, 10, and 11 of an N-terminal domain when aligned with SEQ ID NO: 1 and amino acids corresponding to any one, two, three, four, five, six, seven, or eight of amino acids at positions 278, 279, 280, 281, 282, 283, 284, and 285 of an extracellular third loop when aligned with SEQ ID NO: 1 in an amino acid sequence, preferably include modifications of one or a plurality of such amino acids to a cysteine. In still another embodiment, a modified amino acid sequence may preferably be an amino acid sequence having one or a plurality of (preferably one or several, or one, two, three, four, five, six, seven, eight, nine, ten, or eleven) conservative substitutions in an amino acid sequence of an opsin.

A method for obtaining a nucleic acid such as DNA of the present disclosure is not particularly limited and examples thereof include known methods such as a method of obtaining cDNA by reverse transcription from mRNA (e.g., RT-PCR method), a method of preparing from genomic DNA, a method of synthesizing by chemical synthesis, an method of isolating from a genomic DNA library or cDNA library (see, for example, Japanese Laid-Open Publication No. H11-29599).

Herein, a chimeric protein can be prepared, for example, by using a transformant to which an expression vector containing a nucleic acid such as DNA encoding the above-described chimeric protein has been introduced. For example, the transformant is first cultured under an appropriate condition to synthesize a chimeric protein encoded by a nucleic acid such as the DNA. The thus-synthesized protein can then be collected from the transformant or a culture medium to obtain the chimeric protein of the present disclosure.

More specifically, the chimeric protein of the present disclosure can be produced by inserting DNA encoding the above-described chimeric protein into an appropriate expression vector. The "appropriate vector" may be any vector that is capable of retaining replication or self-reproducing in various types of prokaryotic and/or eukaryotic hosts, and can be selected as appropriate for the purpose of use. For example, a high-copy vector can be selected when a large amount of nucleic acid such as DNA is desired to be obtained, and an expression vector can be selected when a polypeptide (chimeric protein) is desired to be obtained. Specific examples thereof are not particularly limited, and include, for example, well-known vectors described in Japanese Laid-Open Publication No. H11-29599.

The expression vector can be used not only to synthesize a chimeric protein, but also in a composition of the present disclosure. In other words, a composition of the present disclosure may contain as an active ingredient an expression vector into which the above-described nucleic acid construct of the present disclosure has been incorporated. Direct introduction of such an expression vector into human enables treatment, prevention, and inhibition of progression of a retinal disease, disorder, or symptom. In this case, a vector that can be introduced into a human cell is used. For example, an adeno-associated virus vector (AAV vector) and a lentiviral vector are suitable as such a vector.

A method for introducing a vector can be selected according to a type of a vector and a host, etc. Specific examples thereof are not particularly limited and include, for example, a known method such as a protoplast method, a competent method when a bacterium is used as a host (see, for example, Japanese Laid-Open Publication No. H11-29599). When an expression vector is used as an active ingredient for a visual function regenerating agent or a visual function loss preventive agent of the present disclosure, it can be introduced, for example, by intraocular injection of the above-described AAV vector.

A host to which an expression vector is introduced can be any host that is compatible with the expression vector and can be transformed, and specific examples are not particularly limited and include a known natural cell or an artificially established cell such as a bacterial cell, a yeast cell, an animal cell, or an insect cell (see Japanese Laid-Open Publication No. H11-29599), or an animal such as human or mouse. A transformant can be cultured by selecting a nutrient medium from known nutrient media and adjusting a temperature, a pH of the nutrient medium, a culture time, etc. as appropriate, depending on, for example, a type of transformant so as to easily obtain a large amount of a chimeric protein (see, for example, Japanese Laid-Open Publication No. H11-29599).

In one preferred embodiment, opsin of the present disclosure is provided as a nucleic acid molecule or a nucleic acid construct containing the nucleic acid molecule, and is provided as a medicament that achieves a gene therapy. A nucleic acid molecule encoding opsin or a nucleic acid construct containing the nucleic acid molecule, which may be used in this embodiment, includes a nucleic acid molecule that encodes a protein including an amino acid sequence of an opsin and including a modification at an amino acid corresponding to position 188 when aligned with SEQ ID NO: 1 or a nucleic acid construct containing the nucleic acid molecule, and the opsin may be one described elsewhere in this specification. A nucleic acid molecule encoding opsin herein also includes, but is not limited to, a nucleic acid encoding melanopsin, encephalopsin, OPN5, RGR, and peropsin. In one embodiment, a nucleic acid molecule to be used in the present disclosure may be a nucleic acid molecule encoding a microbial opsin, an animal opsin, etc. More specifically, the animal opsin may also be a nucleic acid molecule encoding a vertebrate visual opsin, a vertebrate non-visual opsin, an invertebrate opsin, etc. A nucleic acid molecule that may be used in the present disclosure may be a nucleic acid molecule encoding a bistable opsin such as a vertebrate non-visual opsin and an invertebrate opsin. A nucleic acid encoding opsin to be used in the present disclosure may be advantageously used even if it does not strictly correspond to the microbial opsin, the vertebrate non-visual opsin, or the invertebrate opsin, as long as it is a nucleic acid molecule encoding a functional equivalent that has an equivalent function to that of the opsin that can be advantageously used.

A nucleic acid molecule to be used in the present disclosure may be, for example, a nucleic acid molecule encoding a G protein-coupled receptor rhodopsin or a chimeric opsin of a G protein-coupled receptor rhodopsin. When an animal-derived, preferably a mammal-derived, nucleic acid molecule is used as a nucleic acid encoding a G protein-coupled receptor rhodopsin that can be used herein, a high endogenous G-protein-mediated activity can be obtained while retaining an ability of a protein encoded thereby to repeatedly activate.

A method for isolating and purifying a chimeric protein is not particularly limited and includes known methods such as methods utilizing solubility, a difference in molecular weight, and charge (see, for example, Japanese Laid-Open Publication No. H11-29599).

### (Pharmaceutical use of opsins)

In another aspect, the present disclosure provides a composition, a compound, a medicament, or a method for regenerating vision or for treating, preventing, or inhibiting progression of a visual disorder or disease using a protein including an amino acid sequence of opsin, the protein including a modification at an amino acid corresponding to position 188 when aligned with SEQ ID NO: 1 in the amino acid sequence of the opsin, a nucleic acid molecule encoding the protein, or a nucleic acid construct including the nucleic acid molecule. The present disclosure may provide a method for regenerating vision in a subject or for treating, preventing, or inhibiting progression of a visual disorder or disease in a subject, the method including administering a protein of the present disclosure to the subject. The present disclosure may also provide a use of an opsin of the present disclosure or a nucleic acid molecule encoding the opsin to produce a composition or a medicament for regenerating vision or for treating, preventing, or inhibiting progression of a visual disorder or disease in a subject. The opsin of the present disclosure may have an activity of not releasing retinal upon photoreception.

In one embodiment, examples of a disease, a disorder, or a symptom that may be targeted by opsin of the present disclosure or a nucleic acid molecule encoding the protein, or a nucleic acid construct containing the nucleic acid molecule include, but are not limited to, a vesicular transport disorder, diabetes, diabetic retinopathy, myopia, macular degeneration (e.g., age-related macular degeneration), glaucoma, cataract, a viral infection, corneal dystrophy, retinoblastoma, Alzheimer's disease, Parkinson's disease, a lifestyle-related disease, stroke, hearing loss, arrhythmia, heart failure, motor paralysis, autonomic imbalance, depression, anxiety neurosis, dysuria and dyschezia, and rehabilitation.

In one embodiment, opsin, a nucleic acid molecule encoding the protein, or a nucleic acid construct containing the nucleic acid molecule of the present disclosure can be a medicament for a disease, disorder, or symptom in human, or for a disease, disorder, or symptom in an animal other than human.

In one embodiment, opsin, a protein including the opsin, a nucleic acid molecule encoding the opsin, and/or a nucleic acid construct containing the nucleic acid molecule of the present disclosure can be provided as a cell containing them and the cell can also be used as a medicament.

In one embodiment, a protein including an amino acid sequence of an opsin of the present disclosure, the protein including a modification at an amino acid corresponding to position 188 when aligned with SEQ ID NO: 1 in the amino acid sequence of the opsin can also be used for optogenetics and can be used to regulate only a specific neural activity in an animal of interest. Optogenetics is a technology by which a gene encoding a light-driven ion channel, pump, or enzyme is introduced to express a light-driven protein, thereby transforming a target cell into a light-regulatable form. This allows reversible, immediate, and bioorthogonal manipulation/regulation and analysis of a specific cell (group) on the millisecond timescale while an animal is still alive. For optogenetics, see, for example, Tye, K. M.; Deisseroth, K. "Optogenetic investigation of neural circuits underlying brain disease in animal models, "Nat. Rev. Neurosci. 2012, 13, 251. doi:10.1038/nrn3171; Deisseroth, K. "Optogenetics," Nat. Method 2011, 8, 26. doi:10.1038/nmeth.f.324, etc.

In another aspect of the present disclosure, opsin of the present disclosure or a nucleic acid molecule encoding it can also be provided as a kit in combination with a companion diagnostic drug. For example, a composition containing an active ingredient of a medicament of the present disclosure can exert a therapeutic effect by administering it to the subject when a disease or disorder such as a retinal-related disease is developed or when such a disease or disorder is expected to develop. Therefore, the composition of the present disclosure can be used for diagnosing or testing a genetic status of a subject or genes that the subject has in combination with a companion diagnostic drug for a prior diagnosis of a vision-related disorder, disease, or impairment and then administered only to a subject for whom the composition of the present disclosure is expected to be effective.

In one aspect of the present disclosure, a composition or a medicament of the present disclosure can also be provided as a nucleic acid medicament. In one embodiment, in the case of a gene therapy or a gene treatment using a nucleic acid medicament of the present disclosure, a polynucleotide can be introduced into a genome of a cell to restore or modify a gene and/or gene expression, for example, a therapeutic method in which a normal gene is introduced with a vector or another delivery system that can be introduced into a human cell, such as various viral vectors can be used. A method for introducing the vector can be selected as appropriate depending on a type of a vector or a host, etc. When an expression vector is used as an active ingredient of a composition of the present disclosure, for example, an AAV vector can be introduced by intraocular injection.

As used herein, the phrase "gene therapy" refers to insertion of a nucleic acid sequence (e.g., a transgene as defined herein) into a cell and/or a tissue of an individual to treat a disease. The transgene may be a functional mutant allele that replaces or supplements a defective allele. The gene therapy also includes insertion of a transgene that inhibits, reduces, or decreases an expression, activity, or function of an endogenous gene or protein such as a gene or protein that is inhibited in nature, i.e., undesirable or aberrant (e.g., pathogenic). Such a transgene may also be exogenous. Such an exogenous molecule or sequence is understood to be a molecule or sequence that is not normally present in a cell, a tissue, and/or an individual to be treated. Both acquired and congenital diseases are suitable for the gene therapy.

As used herein, the phrase "gene therapy vector" refers to any vector capable of delivering a polynucleotide encoding a therapeutic protein (e.g., opsin) to a host, such as a patient. In some embodiments, the gene therapy vector targets a specific host cell or organ for local delivery, e.g., for tissue-specific delivery. Typically, the local delivery requires a protein (e.g., therapeutic protein) encoded by mRNA which is translated and expressed primarily in and/or by an organ, e.g., the liver, thereby forming a depot, e.g., a liver depot for protein production (and secretion). In some embodiments, the gene therapy vector is configured to deliver a polynucleotide for a therapeutic protein to the eye of a patient. In some embodiments, the gene therapy vector delivers a polynucleotide encoding a therapeutic protein in a patient to another tissue. In some embodiments, the gene therapy vector delivers a polynucleotide encoding a therapeutic protein to an optic nerve in a patient.

A known or future-developed delivery vector for gene therapy, whether natural or engineered, may be used in the practice of the present disclosure. In some embodiments, the gene therapy vector is a viral vector, e.g., including a virus, a viral capsid, a viral genome, etc. In some embodiments, the gene therapy vector is a naked polynucleotide, e.g., an episome. In some embodiments, the gene therapy vector includes a polynucleotide complex. An exemplary and non-limiting polynucleotide complex for use as the gene therapy vector includes a lipoplex, a polymersome, a polipex, a dendrimer, inorganic nanoparticles (e.g., polynucleotide-coated gold, silica, iron oxide, calcium phosphate, etc.). In some embodiments, the gene therapy vector described herein includes a combination of a viral vector, a naked polynucleotide, and a polynucleotide complex.

In one embodiment, the gene therapy vector is a viral vector including a retrovirus, an adenovirus, a herpes simplex virus, a poxvirus, a vaccinia virus, a lentivirus, or an adeno-associated virus. In one embodiment, the gene therapy vector is an adeno-associated virus (AAV) including serotypes AAV1, AAV2, AAV3, AAV4, AAV5, AAV6, AAV7, AAV8, AAV9, AAV10, and AAV11, or an engineered or natural selected variant thereof. In one embodiment, the polynucleotide also contains a nucleic acid sequence of an adeno-associated virus (AAV). In one embodiment, the gene therapy vector is a chimeric adeno-associated virus containing genetic factors derived from two or more serotypes. For example, an AAV vector containing a rep gene derived from AAV1 and a cap gene derived from AAV2 (referred to as AAV1/2 or AAV RC1/2) may be used as a gene therapy vector for delivering a polynucleotide for a therapeutic protein of the present disclosure to a cell or a cell in a patient in need thereof. In one embodiment, the gene therapy vector is AAV1/2, AAV1/3, AAV1/4, AAV1/5, AAV1/6, AAV1/7, AAV1/8, AAV1/9, AAV1/10, AAV1/11, AAV2/1, AAV2/3, AAV2/4, AAV2/5, AAV2/6, AAV2/7, AAV2/8, AAV2/9, AAV2/10, AAV2/11, AAV3/1, AAV3/2, AAV3/4, AAV3/5, AAV3/6, AAV3/7, AAV3/8, AAV3/9, AAV3/10, AAV3/10, AAV4/1, AAV4/2, AAV4/3, AAV4/5, AAV4/6, AAV4/7, AAV4/8, AAV4/9, AAV4/10, AAV4/11, AAV5/1, AAV5/2, AAV5/3, AAV5/4, AAV5/6, AAV5/7, AAV5/8, AAV5/9, AAV5/10, AAV5/11, AAV6/1, AAV6/2, AAV6/3, AAV6/4, AAV6/5, AAV6/7, AAV6/8, AAV6/9, AAV6/10, AAV6/10, AAV7/1, AAV7/2, AAV7/3, AAV7/4, AAV7/5, AAV7/6, AAV7/8, AAV7/9, AAV7/10, AAV7/11, AAV8/1, AAV8/2, AAV8/3, AAV8/4, AAV8/5, AAV8/6, AAV8/7, AAV8/9, AAV8/10, AAV8/11, AAV9/1, AAV9/2, AAV9/3, AAV9/4, AAV9/5, AAV9/6, AAV9/7, AAV9/8, AAV9/10, AAV9/11, AAV10/1, AAV10/2, AAV10/3, AAV10/4, AAV10/5, AAV10/6, AAV10/7, AAV10/8, AAV10/9, AAV10/11, AAV11/1, AAV11/2, AAV11/3, AAV11/4 AAV11/5, AAV11/6, AAV11/7, AAV11/8, AAV11/9, AAV11/10, or a chimeric virion or derivative thereof. Gao et al., "Novel adeno-associated viruses from rhesus monkeys as vectors for human gene therapy", PNAS 99(18): 11854-11859, Sep. 3, 2002 is incorporated herein by reference for an AAV vector and a chimeric virion useful as the gene therapy vectors and their construction pair and use.

In some embodiments, the gene therapy vector is a viral vector that has been pseudotyped (e.g., engineered) to target a specific cell (e.g., a retinal cell). It can be summarized that many of advances in targeted gene therapy using a viral vector are a non-recombination-based (non-gene-mediated) or recombination-based (gene-mediated) modification of a viral vector and, as a result, pseudotyping, extension, and/or retargeting of natural tropism of the viral vector is achieved. (outlined in Nicklin and Baker (2002) Curr. Gene Ther. 2:273-93; Verheiji and Rottier (2012) Advances Virol 2012:1-15). A non-gene-mediated approach typically utilizes an adapter that recognizes both a wild-type (non-modified) viral surface protein and a target cell. A soluble pseudoreceptor (for a wild-type virus), a polymer such as polyethylene glycol, and an antibody or a portion thereof have been used as a virus-binding domain of the adapter, whereas a natural peptide or a vitamin ligand and an antibody or a portion thereof have been used as a cell-binding domain of the adapter. For example, retargeting of the viral vector to a target cell can be accomplished by binding a vector:adapter complex to a protein expressed on a surface of the target cell, such as a cell surface protein. Such an approach has been used for an AAV (Bartlett et al. (1999) Nat. Biotechnol. 74: 2777-2785), an adenovirus (Hemminki et al. (2001) Cancer Res. 61: 6377-81; van Beusechem et al. (2003) Gene Therapy 10:1982-1991; Einfeld, et al. (2001) J. Virol. 75:11284-91; Glasgow et al. (2009) PLOS One 4:e8355), a herpesvirus (Nakano et al. (2005) Mol. Ther. 11:617-24), and a paramyxovirus (Bian et al. (2005) Cancer Gene Ther. 12:295-303; Bian et al. (2005) Int. J. Oncol. 29:1359-69), and a coronavirus (Haijema et al. (2003) J. Virol. 77:4528-4538; Wurdinger et al. (2005) Gene Therapy 12:1394-1404).

Genetic modification by recombination of a viral capsid protein, hence genetic modification by recombination of a surface of a viral capsid is a common approach. In an indirect recombination approach, a viral capsid is modified by a heterologous "scaffold" and then linked to an adapter. The adapter binds to the scaffold and a target cell (see, Arnold et al. (2006) Mol. Ther. 5:125-132; Ponnazhagen et al. (2002) J. Virol. 76:12900-907; WO 97/05266). The scaffold such as (1) an Fc binding molecule that binds to Fc of an antibody adapter (e.g., Fc receptor, protein A, etc.), (2) (strept)avidin that binds to a biotinylated adapter, (3) biotin that binds to an adapter fused to (strept)avidin, and (4) a protein:protein binding pair that forms an isometric peptide bond, such as SpyCatcher that binds to a SpyTagged adapter, etc. has been incorporated in Ad (Pereboeva et al. (2007) Gene Therapy 14: 627-637; Park et al. (2008) Biochemical and Biophysical Research Communications 366: 769-774; Henning et al. (2002) Human Gene Therapy 13:1427-1439; Banerjee et al. (2011) Bioorganic and Medicinal Chemistry Letters 21:4985-4988), an AAV (Gigout et al. (2005) Molecular Therapy 11:856-865; Stachler et al. (2008) Molecular Therapy 16:1467-1473), and a togavirus (Quetglas et al. (2010) Virus Research 153:179-196; Ohno et al. (1997) Nature Biotechnology 15:763-767; Klimstra et al. (2005) Virology 338:9-21).

In a direct recombinant targeting approach, a targeting ligand is inserted or bound directly to a viral capsid, i.e., a protein viral capsid is modified to express a heterologous ligand. The ligand redirects, e.g., binds to, a receptor or a marker that is preferentially or exclusively expressed on a target cell. This has been used in a poxvirus (Guse et al. (2011) Expert Opinion on Biological Therapy 11:595-608; Galmiche et al. (1997) Journal of General Virology 78:3019-3027; Paul et al. (2007) Viral Immunology 20:664-671), a paramyxovirus (Nakamura and Russell (2004) Expert Opinion on Biological Therapy 4:1685-1692; Hammond et al. (2001) Journal of Virology 75:2087-2096; Galanis (2010) Clinical Pharmacology and Therapeutics 88:620-625; Blechacz and Russell (2008) Current Gene Therapy 8:162-175; Russell and Peng (2009) Current Topics in Microbiology and Immunology 330:213-241), and a herpes virus (Shah and Breakefield (2006) Current Gene Therapy 6:361-370; Campadelli-Fiume et al. (2011) Reviews in Medical Virology 21:213-226).

In some embodiments, a gene therapy vector described herein contains a naked polynucleotide. For example, in some embodiments, a polynucleotide encoding a therapeutic polypeptide may be injected, for example, near the eye, directly into an organ for depot formation, or intravenously. An additional well-known method for enhancing delivery of the naked polynucleotide includes, but is not limited to, electroporation, ultrasonic perforation, use of a gene gun to eject polynucleotide-coated gold particles, magnetic particles, and hydraulic delivery.

In some embodiments, the gene therapy vector described herein is a polynucleotide complex, for example, without limitation, nanoparticles (e.g., polynucleotide self-assembled nanoparticles, polymer-based self-assembled nanoparticles, inorganic nanoparticles, lipid nanoparticles, semiconductive/metallic nanoparticles), gel and hydrogel, a polynucleotide complex containing a cation and an anion, microparticles, and any combination thereof.

In some embodiments, the polynucleotide disclosed herein can be formulated as self-assembling nanoparticles. As a non-limiting example, a polynucleotide may be used to make nanoparticles that may be used in a delivery system for a polynucleotide (see, for example, International Patent Application Publication No. 2012125987 which is incorporated herein by reference in its entirety). In some embodiments, the polynucleotide self-assembled nanoparticles can include a core composed of the polynucleotide disclosed herein and a polymer shell. The polymer shell may be any of polymers described herein and is known in the art. In an additional embodiment, the polymer shell may be used to protect the polynucleotides in the core.

In some embodiments, these self-assembled nanoparticles may be microsponges formed of polynucleotide hairpins of long polymers, and the polynucleotide hairpins are formed into crystalline "folded" sheets and then self-assembled into microsponges. These microsponges are densely packed, sponge-like microparticles that may act as efficient carriers and deliver a cargo to a cell. The microsponges can be from 1 µm to 300 nm in diameter. The microsponges may be complexed with another drug known in the art to form even larger microsponges. As a non-limiting example, the microsponges may be complexed with a drug for forming an outer layer and facilitating uptake by a cell such as polycationic polyethyleneimine (PEI). This complex can form particles each of which has a diameter of 250 nm, which allows the particles to be stable at a high temperature (150°C) (Grabow and Jaegar, Nature Materials 2012, 11:269-269; incorporated herein by reference in its entirety). In addition, these microsponges may exhibit an exceptional degree of protection from degradation by a ribonuclease. In another embodiment, polymer-based self-assembling nanoparticles, for example, without limitation, microsponges may be fully programmable nanoparticles. Geometry, size, and stoichiometry of the nanoparticles can be precisely controlled to produce optimal nanoparticles for delivering a cargo, e.g., without limitation, a polynucleotide.

In some embodiments, the polynucleotide can be formulated into inorganic nanoparticles (U.S. Patent No. 8,257,745 which is incorporated herein by reference in its entirety). The inorganic nanoparticles can include, but are not limited to, a clay-like material that can be swollen with water. As a non-limiting example, the inorganic nanoparticles can include a synthetic smectite clay formed of a simple silicate (see, for example, U.S. Patent Nos. 5,585,108 and 8,257,745, each of which is incorporated herein by reference in their entirety).

In some embodiments, the polynucleotides may be formulated into water-dispersible nanoparticles containing a semiconducting material or a metallic material (U.S. Patent Application Publication No. 20120228565, which is incorporated herein by reference in its entirety) or formed into magnetic nanoparticles (U.S. Patent Application Publication No. U.S. Patent Application Publication Nos. 20120265001 and 20120283503, which are incorporated herein by reference in their entirety). The water-dispersible nanoparticles can be hydrophobic nanoparticles or hydrophilic nanoparticles.

In some embodiments, the polynucleotides disclosed herein can be encapsulated in any hydrogel known in the art that can form a gel when injected into a subject. The hydrogel is a network of a hydrophilic polymer chain and may be found as a colloidal gel in which water is a dispersion medium. The hydrogel can include a highly absorbent (capable of containing more than 99% of water) natural or synthetic polymer. The hydrogel also has flexibility very similar to a natural tissue due to its significantly high water content. The hydrogel described herein may be used to encapsulate biocompatible, biodegradable, and/or porous lipid nanoparticles.

As a non-limiting example, the hydrogel may be an aptamer-functionalized hydrogel. The aptamer-functionalized hydrogel may be programmed to release one or a plurality of polynucleotides using polynucleotide hybridization (Battig et al., J. Am. Chem. Society. 2012 134:12410-12413; which is incorporated herein by reference in its entirety). In some embodiments, the polynucleotide may be encapsulated within lipid nanoparticles, which may then be encapsulated within a hydrogel.

In some embodiments, the polynucleotide may be encapsulated within a fibrin gel, a fibrin hydrogel, or a fibrin glue. In another embodiment, the polynucleotide may be formulated into lipid nanoparticles or rapid-excluded lipid nanoparticles before being encapsulated within a fibrin gel, a fibrin hydrogel, or a fibrin glue. In yet another embodiment, the polynucleotide may be formulated as a lipoplex before being encapsulated in a fibrin gel, a hydrogel, or a fibrin glue. The fibrin gel, the hydrogel, and the glue include two components, that is, a fibrinogen solution and a calcium-rich thrombin solution (see, e.g., Spicer and Mikos, Journal of Controlled Release 2010. 148: 49-55; Kidd et al. Journal of Controlled Release 2012. 157:80-85, each of which is incorporated herein by reference in its entirety). Concentrations of the component of the fibrin gel, the hydrogel, and/or the glue can be varied to change a property, a mesh size of the network, and/or a degradation property of the gel, the hydrogel, and/or the glue, for example, without limitation, can change a release property of the fibrin gel, the hydrogel, and/or the glue (see, e.g., Spicer and Mikos, Journal of Controlled Release 2010. 148: 49-55; Kidd et al. Journal of Controlled Release 2012. 157:80-85; Catelas et al. Tissue Engineering 2008. 14:119-128, each of which is incorporated herein by reference in its entirety). This feature may be advantageous when being used to deliver the polynucleotide disclosed herein (see, for example, Kidd et al. Journal of Controlled Release 2012. 157:80-85; Catelas et al. Tissue Engineering 2008. 14:119-128, each of which is incorporated herein by reference in its entirety).

In some embodiments, the polynucleotide disclosed herein can contain a cation or an anion. In one embodiment, a formulation includes a metal cation, for example, without limitation, Zn2+, Ca2+, Cu2+, Mg+, and a combination thereof. As a non-limiting example, the formulation may include a polymer and a polynucleotide complexed with a metal cation (see, for example, U.S. Patent Nos. 6,265,389 and 6,555,525, each of which is incorporated herein by reference in their entirety).

In some embodiments, the polynucleotide can be formulated into nanoparticles and/or microparticles. These nanoparticles and/or microparticles can be molded into shape with any size and chemical. As an example, the nanoparticles and/or the microparticles can be produced using the PRINT (registered trademark) technology by LIQUIDA TECHNOLOGIES.RTM (Morrisville, N.C.) (see, International Patent Application Publication No. 2007024323 which is incorporated herein by reference in its entirety).

In some embodiments, the polynucleotide can be formulated into a nano-jacket and a nanoliposome by Keystone Nano (State College, PA). The nano-jacket is produced from a compound naturally found in vivo including calcium and phosphate or a compound that also includes a small amount of silicate. The nano jacket may range in size from 5 to 50 nm and can be used to deliver a hydrophilic compound and a hydrophobic compound, for example, without limitation, a polynucleotide, a major construct, and/or a polynucleotide. The nanoliposome is produced from a lipid, for example, without limitation, a lipid that naturally occurs in the body. The nanoliposome may range in size from 60 to 80 nm and can be used to deliver a hydrophilic compound and a hydrophobic compound, for example, without limitation, a polynucleotide, a major construct, and/or a polynucleotide. In one aspect, the polynucleotide disclosed herein is formulated into a nanoliposome, for example, without limitation, a ceramide nanoliposome.

In some embodiments, the polynucleotide, e.g., DNA, also contains a promoter operably linked to a nucleic acid sequence encoding a therapeutic protein. In a certain embodiment, the promoter is a tissue-specific promoter that drives gene expression in a specific tissue. In one embodiment, the tissue-specific promoter is a liver-specific enhancer/promoter derived from a SERPINA 1 and/or a TTR promoter. In another embodiment, the promoter is a CMV promoter. In another embodiment, the promoter is a ubiquitin C promoter.

In some embodiments, the polynucleotide also includes a "gene locus targeting nucleic acid sequence". A sequence targeting a gene locus allows a polynucleotide encoding a therapeutic protein to be incorporated into a genome of a recipient host cell. In some embodiments, the gene locus targeting sequence includes an adjacent homologous arm to enable homologous recombination. In some embodiments, the gene locus targeting sequence includes a guide RNA sequence and a type II Cas enzyme (i.e., a CRISPR-Cas9 method) that promotes incorporation. In some embodiments, the gene locus targeting sequence includes a guide zinc finger nuclease (ZFN) recognition sequence to promote incorporation. In some embodiments, the gene locus targeting sequence includes a transcription activator-like effector nuclease (TALEN) recognition sequence to promote incorporation. In still another embodiment, the gene locus targeting sequence includes a single residue pair nucleotide code used by a BuD-derived nuclease to promote incorporation.

In one embodiment, cell therapy using a cell including the composition of the present disclosure includes a therapy in which a retinal cell including the composition of the present disclosure is transplanted. In one embodiment, the cell including the composition of the present disclosure may be administered with an additional drug in addition to the cell. Such an additional drug may be a drug commonly used in ophthalmic therapy (e.g., a steroid, an antibiotic, an antibacterial substance, NSAID). Such an additional drug may be included as a medicament in a cellular medicament of the present disclosure or may be provided in a separately administered form. In the case of a separately provided or administered form, it is provided as a kit or a combination drug. When used as the kit or the combination drug, it may be combined with a package insert describing how to use it.

In a preferred embodiment, the present disclosure is preferably, but is not limited to, administered to a subject prior to or immediately after onset of the disease, disorder, or symptom as described above, e.g., within one year, preferably within six months, three months, or one month from the onset (e.g., development of a subjective symptom).

In one particular embodiment, a protein, a nucleic acid molecule, a nucleic acid construct, and/or a cell of the present disclosure, or a medicament containing them are administered once during a therapeutic period. As described in Examples, it has been confirmed that a medicament of the present disclosure is effective when being administered once, and thus patient compliance is considered to be good.

In one particular embodiment, when a medicament of the present disclosure is administered to the eye, a vector may be used in a unit dose of about 0.01 × 10¹¹ to about 100 × 10¹¹ vg/eye, for example, a lower limit thereof may be about 0.01 × 10¹¹ vg/eye, about 0.02 × 10¹¹ vg/eye, about 0.03 × 10¹¹ vg/eye, about 0.04 × 10¹¹ vg/eye, about 0.05 x 10¹¹ vg/eye, about 0.06 × 10¹¹ vg/eye, about 0.07 × 10¹¹ vg/eye, about 0.08 × 10¹¹ vg/eye, about 0.09 × 10¹¹ vg/eye, about 0.1 × 10¹¹ vg/eye, about 0.2 × 10¹¹ vg/eye, about 0.3 × 10¹¹ vg/eye, about 0.4 × 10¹¹ vg/eye, about 0.5 × 10¹¹ vg/eye, etc. and an upper limit thereof may be about 2 × 10¹¹ vg/eye, about 3 × 10¹¹ vg/eye, about 4 × 10¹¹ vg/eye, about 5 × 10¹¹ vg/eye, about 6 × 10¹¹ vg/eye, about 7 × 10¹¹ vg/eye, about 8 × 10¹¹ vg/eye, about 9 × 10¹¹ vg/eye, about 10 × 10¹¹ vg/eye, about 15 × 10¹¹ vg/eye, about 20 × 10¹¹ vg/eye, about 30 × 10¹¹ vg/eye, about 40 × 10¹¹ vg/eye, about 50 × 10¹¹ vg/eye, about 100 × 10¹¹vg/eye, etc.

In another embodiment, when a medicament of the present disclosure is administered to systemically (intravenous injection) or locally (intramuscular, intracerebral, intraaural, etc.), a vector may be used in a unit dose of about 0.1 × 10¹¹ to about 1000 × 10¹¹ vg/kg, for example, a lower limit thereof may be about 0.1 × 10¹¹ vg/eye, about 0.2 × 10¹¹ vg/eye, about 0.3 × 10¹¹ vg/eye, about 0.4 × 10¹¹ vg/eye, about 0.5 × 10¹¹ vg/eye, about 0.6 × 10¹¹ vg/eye, about 0.7 × 10¹¹ vg/eye, about 0.8 × 10¹¹ vg/eye, about 0.9 × 10¹¹ vg/eye, about 1.0 × 10¹¹vg/kg, etc. and an upper limit thereof may be about 20 × 10¹¹ vg/eye, about 30 × 10¹¹ vg/eye, about 40 × 10¹¹ vg/eye, about 50 × 10¹¹ vg/eye, about 60 × 10¹¹ vg/eye, about 70 × 10¹¹ vg/eye, about 80 × 10¹¹ vg/eye, about 90 × 10¹¹ vg/eye, about 100 × 10¹¹ vg/eye, about 150 × 10¹¹ vg/eye, about 200 × 10¹¹ vg/eye, about 300 × 10¹¹ vg/eye, about 400 × 10¹¹ vg/eye, about 500 × 10¹¹ vg/eye, about 1000 × 10¹¹vg/kg, etc.

### (Combination)

In one aspect of the present disclosure, two or more of an opsin of the present disclosure, or a protein, a nucleic acid molecule, a nucleic acid construct, and/or a cell including the opsin, or a medicament including them for prevention or inhibition of progression of a visual disorder can be used in combination. In one embodiment, when combining each of the above applications, the same active ingredient can be used in a combined application or different active ingredients can be used in combination.

### (General technologies)

Molecular biological, biochemical, and microbiological procedures used herein are well known and common in the art, and are described in, for example, Current Protocols in Molecular Biology (http://onlinelibrary.wiley.com/book/10.1002/0471142727) and Molecular Cloning: A Laboratory Manual (Fourth Edition) (http://www.molecularcloning.com), each of which is incorporated herein by reference in its relevant parts (may be entirety).

As used herein, the term "or" is used when "at least one or more" of matters enumerated in a text can be employed. The same is true for "alternatively". When the phrase "within a range" of "two values" is specified herein, the range includes the two values themselves.

References cited herein such as the scientific literature, patents, and patent applications are incorporated herein by reference in its entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

### [Example]

Examples will be described. If necessary, animals used in the following examples were handled in accordance with the Declaration of Helsinki and in compliance with the standards and other relevant ethical standards and guidelines stipulated in the institution to which the applicant belongs and elsewhere. The reagents used were specifically the products described in Examples, but equivalent products from other manufacturers (Sigma-Aldrich; FUJIFILM Wako Pure Chemical Corporation; NACALAI TESQUE, INC.; R&D Systems; USCN Life Science INC, etc.) can also be substituted.

### (Experimental Procedures)

In Examples below, experimental procedures were performed as follows.

### (Production of bovine rhodopsin mutant)

Bovine rhodopsin mutant cDNA (accession number: AB062417) was constructed using the In-Fusion cloning kit (manufactured by Clontech). Wild-type (Gene ID: 509933) and mutant bovine rhodopsin cDNAs were inserted into a mammalian expression vector pUSRα (Kayada et al.,1995) or pCAGGS (Niwa et al.,1991). HEK293T cells were authenticated by short tandem repeat profiling. The cells were negative for mycoplasma contamination. The thus-obtained plasmids were transfected into the HEK293T cells using a calcium phosphate method. After 2 days of culture, the thus-transfected cells were collected by centrifugation, suspended in a Buffer A (50 mM HEPES, 140 mM NaCl, 3 mM MgCl2, pH 6.5), and added with 11-cis or all-trans retinal to reconstitute a photopigment. These were solubilized in the Buffer A containing 1% dodecyl maltoside (DDM) and adsorbed on a Rho1D4 (anti-bovine rhodopsin monoclonal antibody) affinity column to purify the pigment. After washing the column with the Buffer A containing 0.02% DDM, a synthetic peptide with an epitope sequence was added thereto to elute the pigment. To purify an apoprotein of rhodopsin, transfected cell membranes without retinal were solubilized in the Buffer A containing 1% DDM and adsorbed on the Rho1D4 affinity column.

### (Spectroscopy)

UV-visible absorption spectra were recorded using a UV-visible spectrophotometer (UV-2450, UV-2400, manufactured by SHIMADZU CORPORATION). For detailed analysis of a thermal reaction of the pigment, samples were kept at 0°C, 20°C, or 37°C using cell holders equipped with temperature-controlled circulating water baths. The samples were irradiated with either yellow light from a 1 kW tungsten halogen lamp (Master HILUX-HR; RIKEN) through a Y-52 cutoff filter (TOSHIBA CORPORATION) or UV light through a UV D-36 glass filter (AGC TECHNO GLASS CO., LTD.).

To monitor a photocycle process of the G188C mutant of bovine rhodopsin, a time-resolved CCD spectrophotometer (C10000 system, HAMAMATSU PHOTONICS K.K.) was used (Sakai et al.). Spectra were obtained from the sample of the G188C mutant in the dark and at different time points after irradiation (170 µs, yellow light from a xenon flashlamp through a Y-52 cutoff filter). A temperature of the sample was maintained at 37°C by a temperature controller (pqod, QUANTUM Northwest). A change in absorbance at λmax was plotted as a function of time and fitted with a monoexponential function to determine a time constant for recovery to an original dark state.

### (Retinal isomer analysis)

Retinal isomers in the rhodopsin samples were analyzed by high-performance liquid chromatography (LC-10ATvp; Shimadzu) using a silica column (YMC-Pack SIL, particle size: 3 µm, 150 × 6.0 mm, YMC) as described previously (Tsutsui et al., 2007).

### (G protein activation assay)

Activation of a Gi-type G protein was measured based on GDP/GTPγS exchange of the G protein using a radioactive nucleotide filter binding assay (Yamashita et al., 2000; Yamashita et al., 2010). Giαβγ was prepared by mixing rat Giα1 expressed in an E. coli strain BL21 (Lee et al., 1994) and Gtβγ purified from bovine retina (Tachibanaki et al., 1997). All assay procedures were performed at 0°C. An assay mixture was composed of 10 nM a dye, 600 nM a G protein, 50 mM HEPES (pH 7.0), 140 mM NaCl, 5 mM MgCl2, 1 mM DTT, 0.01% DDM, 1 µM [35S]GTPγS, and 2 µM GDP. Wild type and G188C mutant bovine rhodopsins were reconstituted with 11-cis retinal, purified, and then mixed with a G protein solution and stored in the dark or irradiated with yellow light (>500 nm) for 1 min and then UV light for 1 min or yellow light for 1 min again. After the irradiation, a [35S]GTPγS solution was added to the mixture of the rhodopsin and the G protein to initiate a GDP/GTPγS exchange reaction. After incubation in the dark for the selected time, an aliquot (20 µL) was taken from the sample into 200 µL of a stop solution (20 mM Tris/Cl [pH 7.4], 100 mM NaCl, 25 mM MgCl2, 1 µMGTPγS, 2 µM GDP) and immediately filtered through a nitrocellulose membrane to trap [35S]GTPγS bound to the G protein. An amount of such [35S]GTPγS was quantified by measuring a membrane with a liquid scintillation counter (Tri-Carb 2910 TR; PerkinElmer).

### (Measurement of cAMP amount in cultured cells)

An amount of cAMP in HEK293T cells was measured using the GloSensor cAMP assay (Promega) according to the manufacturer's instruction and the previous report (Bailes and Lucas, 2013). The HEK293T cells were seeded in a 96-well plate at a density of 20,000 cells/well in a low serum medium (D-MEM/F12 containing 0.25% FBS). After 24 hours of culture, the cells were transfected with 50 ng of a rhodopsin plasmid and 50 ng of a Glosensor 22F plasmid per well by a polyethyleneimine transfection method. After overnight culture, the medium was replaced by an equilibrated medium containing a 2% dilution of a GloSensor cAMP reagent stock solution in the CO2-independent medium (Thermo Fisher Scientific), 10% FBS, and 5 µM retinal. After equilibration at room temperature for 2 hours, luminescence from the cells was measured using a microplate reader (Spect raMax L, Molecular Devices). For measurement of Gi activation by wild-type and mutant rhodopsins, the cells were first treated with 2 µM forskolin to increase cAMP-dependent luminescence to a plateau level, followed by stimulation with yellow light from a 1 kW tungsten halogen lamp through a Y-52 cutoff filter for 30 seconds.

### (Example 1: Acquisition of photocycle property of bovine rhodopsin G188C mutant)

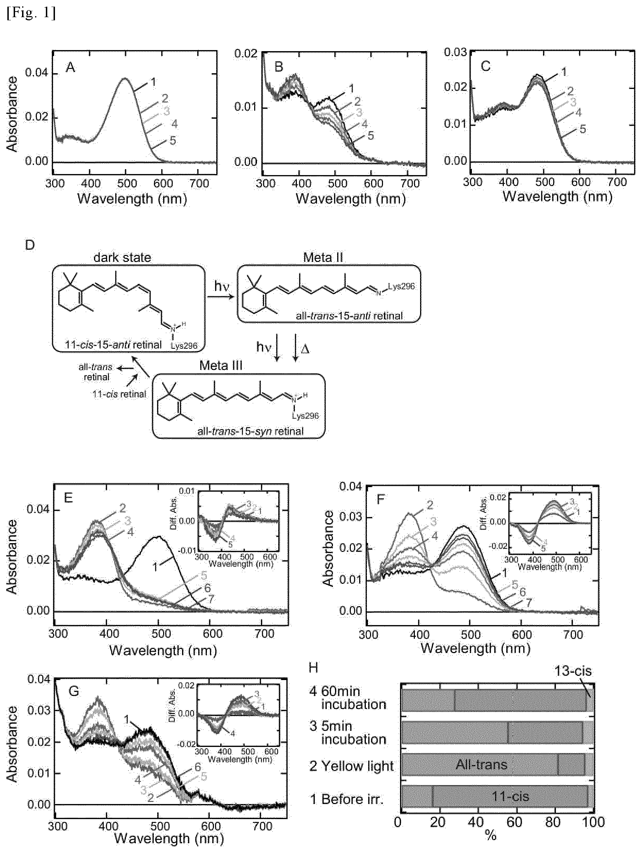

To analyze whether the G188C mutant of bovine rhodopsin had acquired a photocycle property, the G188C mutant was reconstituted with 11-cis retinal and then purified. FIG. 1 shows thermal stability of wild-type (FIG. 1A), G188C mutant (FIG. 1B), and G188C/N2C/D282C (FIG. 1C) mutant bovine rhodopsins that had been incubated with 11-cis-retinal and then purified. Absorption spectra were recorded after incubation in the dark at 37°C for 0, 5, 10, 15, or 20 min (Curves 1 to 5, respectively). FIG. 1D shows a schematic diagram of a change in retinal configuration in the wild-type bovine rhodopsin. A dark state, a Meta II, and a Meta III contain 11-cis-15-anti-retinal, all-trans-15-anti-retinal, and all-trans-15-syn retinal, respectively. FIG. 1E and F show absorption spectra of N2C/D282C (FIG. 1E) and G188C/N2C/D282C (FIG. 1F) mutant bovine rhodopsins that had been incubated with 11-cis-retinal and then purified. The spectra were recorded at 20°C in the dark (Curve 1) and at 0, 5, 15, 30, 60, and 120 min (Curves 2 to 7, respectively) after irradiation with yellow light. The inset shows differential spectra (Curves 1 to 5, respectively) each obtained by subtracting the spectra immediately after irradiation (Curve 2 in E and F) from each of the spectra measured after irradiation (Curves 3 to 7 in E and F). FIG. 1G shows absorption spectra of the G188C/N2C/D282C mutant as measured at 37°C. The spectra were recorded in the dark (Curve 1) and at 0.1, 10, 50, 100, and 1000 seconds (Curves 2 to 6, respectively) after irradiation with yellow light. The inset shows differential spectra (Curves 1 to 4, respectively) each obtained by subtracting the spectrum immediately after irradiation (Curve 2 in G) from each of the spectra measured after irradiation (Curves 3 to 6 in G). FIG. 1H shows an isomeric composition of retinal in the G188C/N2C/D282C mutant. Chromophores were extracted from samples before light irradiation and at 0, 5, and 60 minutes after irradiation with yellow light at 20°C and analyzed for retinal configuration by high-performance liquid chromatography (HPLC).

The G188C mutant was found to have very low thermal stability than that of the wild type. That is, the G188C mutant gradually decayed during incubation in the dark at 37°C (FIG. 1B), whereas the wild type was extremely stable under the same conditions (FIG. 1A). Therefore, the thermal stability of the G188C mutant was improved and molecular characteristics of the mutant was analyzed in detail. According to previous reports (Xie et al., 2003; Standfuss et al., 2007), two cysteine residues (N2C/D282C) were introduced into the mutant and the resultant was measured for a thermal decay rate when incubated at 37°C in the dark. It can be seen from time-dependent spectral changes that this G188C/N2C/D282C mutant decays much more slowly than the G188C mutant (FIG. 1C). Therefore, spectral changes at 20°C were compared among the wild-type, the N2C/D282C mutant, and the G188C/N2C/D282C mutant. After irradiation with yellow light, a spectrum of the wild type shifted to the UV region. This suggests formation of a Meta II intermediate containing all-trans-15-anti retinal (FIG. 1D). Subsequently, absorbance around 470 nm increased, indicating a transition from the Meta II to a Meta III intermediate containing all-trans-15-syn retinal (FIG. 1D). These spectral changes were also observed in the N2C/D282C (FIG. 1E). On the other hand, the G188C/N2C/D282C mutant had an absorption maximum (λmax) at 487 nm and its spectrum was also shifted to the UV region by yellow light irradiation, resulting in formation of the Meta II. Subsequently, when cultured in the dark, a decrease in absorbance in the UV region and an increase in absorbance around 485 nm accompanied therewith were observed (FIG. 1F). Analysis of retinal configuration showed that light irradiation isomerized retinal to an all-trans form, which was converted to an 11-cis form by subsequent incubation in the dark (FIG. 1H). This interconversion of retinal isomers can explain the spectral changes of the G188C/N2C/D282C mutant after light irradiation. Thermal recovery to an original dark state after light irradiation was also observed at 37°C (FIG. 1G). Furthermore, the G188C mutant exhibited thermal recovery of an absorption spectrum to the original dark state after light irradiation at 20°C and it was confirmed that an amount of 11-cis-retinal was increased during culture after light irradiation. These results indicate that the G188C mutation led to acquisition of an ability to thermally recover from a light-activated state to an original dark state.

Then, whether other G188 mutants had acquired a photocycle property was also analyzed. G188E and G188R mutants of human rhodopsin have been known to be unable to form a photopigment after reconstitution with 11-cis-retinal (Sung et al., 1993). Therefore, 16 other mutations were introduced at position 188 of bovine rhodopsin, reconstituted with 11-cis retinal, and then purified to prepare mutant proteins. The photopigment was successfully detected in eight of these mutants. The λmax of the mutants was blue-shifted from the wild type (500 nm), except for G188D (509 nm) (Table 3).

**[Table 3]**

| Comparison of λmax in the dark state and spectral components after UV light irradiation | | | | |
|---|---|---|---|---|
| | λmax | dark state (%) | meta II (%) | meta III (%) |
| wild-type | 500 | 21.6 | 13.7 | 64.7 |
| G188C | 487 | 41.2 | 48.5 | 10.3 |
| G188A | 494 | 18.1 | 51.7 | 30.2 |
| G188D | 509 | 30.9 | 67.7 | 1.4 |
| G188M | 491 | 9.2 | 10 | 80.8 |
| G188N | 492 | 0 | 0 | 100 |
| G188Q | 493 | 2.4 | 5.5 | 92.1 |
| G188S | 495 | 14.6 | 37.7 | 47.7 |
| G188T | 488 | 11 | 2.9 | 86.1 |
| G188V | 486 | 7.3 | 63.4 | 29.3 |

When these mutants were irradiated with yellow light, their spectra shifted to the UV region, resulting in formation of the Meta II. Subsequently, when cultured in the dark at 20°C, the mutants exhibited characteristic spectral changes. However, these spectral changes were different from a significant increase in absorbance around their λmax. These results indicate that thermal recovery to an original dark state after light irradiation is not clearly detectable in these mutants. Therefore, it was concluded that a photocycle property is specifically observed in the G188C mutant.

### (Example 2: Acquisition of photoreversibility of bovine rhodopsin G188C mutant)

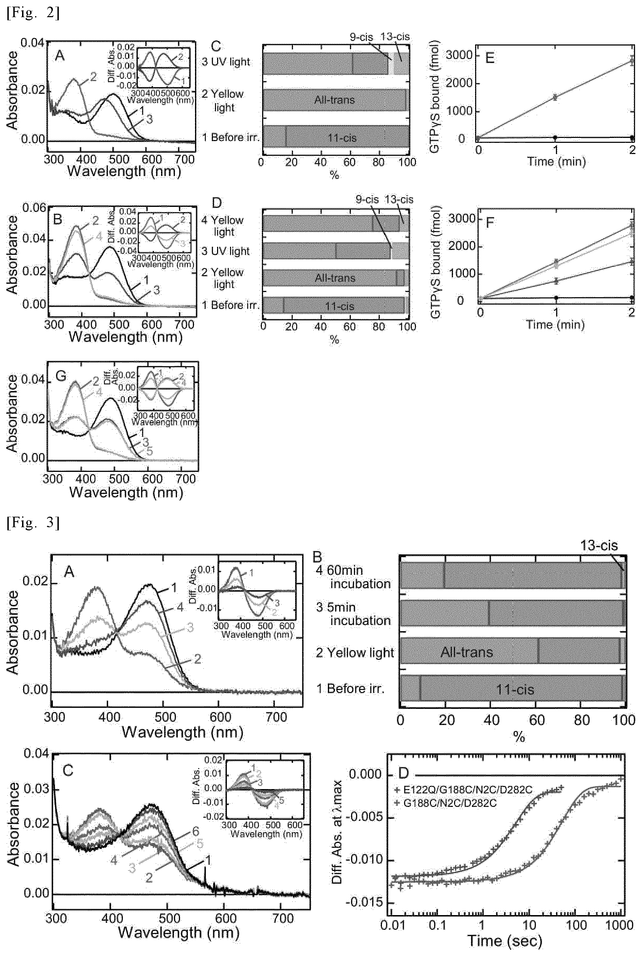

Whether the Meta II of the G188C mutant returns to the original dark state in a light-dependent manner was analyzed. FIG. 2 shows a light reaction, a retinal configuration, and a G protein activation of the G188C mutant of bovine rhodopsin. FIGs. 2A and B show absorption spectra of the wild-type (FIG. 2A) and the G188C mutant (FIG. 2B) bovine rhodopsin that had been incubated with 11-cis-retinal at 0°C and then purified. The spectra were recorded in the dark (Curve 1), after irradiation with yellow light (>500 nm) (Curve 2), after subsequent irradiation with UV light (360 nm) (Curve 3), and after reirradiation with yellow light (Curve 4). The inset shows spectral changes of the wild type (FIG. 2A) or the G188C mutant (FIG. 2B) upon irradiation with yellow light (Curve 1), subsequent irradiation with UV (Curve 2), and reirradiation with yellow light (Curve 3). Differential spectra were calculated from the spectra shown in FIGs. 2A and 2B. FIGs. 2C and D show isomeric compositions of retinal in the wild type (FIG. 2C) and the G188C mutant (FIG. 2D). Chromophores were extracted from samples before light irradiation, after irradiation with yellow light, after subsequent irradiation with UV, after reirradiation with yellow light at 0°C and analyzed for retinal configuration by high-performance liquid chromatography (HPLC). FIG. 2E shows a Gi-type G protein activation ability of the wild type. The activation ability was measured in the dark (closed circles) and after irradiation with yellow light (open circles). FIG. 2F shows a Gi-type G protein activation ability of the G188C mutant. The activation ability was measured in the dark (closed circles), after irradiation with yellow light (open circles), after subsequent irradiation with UV (open triangles), and after reirradiation with yellow light (open diamonds). The data shown in FIGs. 2E and F were obtained at 0°C and are presented as the mean ± SEM of three independent experiments. FIG. 2G shows absorption spectra (0°C) of the G188C/N2C/D282C mutant that had been incubated with 11-cis retinal and then purified. The spectra were recorded in the dark (Curve 1), after irradiation with yellow light (>500 nm) (Curve 2), after subsequent irradiation with UV (360 nm) (Curve 3), after reirradiation with yellow light (Curve 4), and after reirradiation with UV (Curve 5). The inset shows spectral changes caused by irradiation with yellow light (Curve 1), subsequent irradiation with UV (Curve 2), reirradiation with yellow light (Curve 3), and reirradiation with UV (Curve 4). Differential spectra were calculated based on the spectra shown in FIG. 2G.

The wild-type and the G188C mutant were cooled to 0°C to prevent a thermal reaction of the Meta II and measured for spectral changes caused by irradiation with yellow light and subsequent UV. For the wild type, irradiation with yellow light produced the Meta II and subsequent irradiation with UV caused a shift of the spectrum to the visible region and a blue shift of the λmax (about 470 nm) from the original dark state (FIG. 2A). Previous studies have revealed that this state is equivalent to the Meta III (FIG. 1D). Template absorption spectra for the dark state, the Meta II, and the Meta III modeled by a conventional method (Lamb, 1995; Govardovskii et al., 2000) were constructed and fitted with differential spectra obtained by subtracting the spectra after irradiation with yellow light from the spectra after irradiation with UV. As a result of the fitting, irradiation of Meta II with UV produced the Meta III much more efficiently than in an original dark state (Table 3). This spectral analysis is consistent with the observation that UV irradiation produces a very limited amount of 11-cis retinal from a large amount of all-trans retinal (FIG. 2C). These results confirm that irradiation of the Meta II with UV induces a syn/anti isomerization of a C=N double bond in a Schiff base more efficiently than a cis/trans isomerization of retinal.

For the G188C mutant, irradiation with yellow light produced the Meta II and subsequent irradiation with UV caused a shift of the spectrum to the visible region and made the λmax almost the same as that in the original dark state (FIG. 2B). Reirradiation with yellow light resulted in formation of a state in which the spectrum nearly overlapped with that induced by the initial yellow light irradiation (Curve 4 in FIG. 2B). Spectral changes induced by irradiation with UV and reirradiation with yellow light were mirror images of each other (Curves 2 and 3 in the inset of FIG. 2B). It was shown that irradiation of Meta II with UV resulted in formation of the original dark state much more efficiently than the Meta III by fitting UV-dependent spectral changes to template spectra (Table 3). This was supported by the observation that irradiation of the G188C mutant with UV increased 11-cis retinal more efficiently than irradiation of the wild type with UV (FIG. 2D). These results suggest that the Meta II of the G188C mutant can efficiently photoconvert to its original dark state. Next, since bovine rhodopsin can activate not only transducin but also a Gi/Go-type G protein (Yamashita et al., 2000; Terakita et al., 2002), an ability of the G188C mutant to activate a Gi-type G protein was measured. The GTPγS binding assay revealed that a light-dependent Gi activation ability of G188C is equivalent to that of the wild type (FIG. 2E, F). Subsequently, irradiation of the G188C mutant with UV suppressed the ability and reirradiation with yellow light increased the ability (FIG. 2F). This can be explained by changes in absorption spectra and retinal isomers (FIG. 2B, D). Furthermore, the G188C/N2C/D282C mutant could also show interconversion between the original dark state and the Meta II upon irradiation with yellow light at 0°C and UV (FIG. 2G). These data revealed that the G188C mutant has acquired photoreversibility between the dark state and the Meta II.

Light reactions of the other eight mutants were also analyzed. In all of these mutants, it was confirmed that irradiation with yellow light caused a spectral shift to the UV region and subsequent irradiation with UV light increased absorbance in the visible region again. Information on component ratios of the dark state, the Meta II, and the Meta III after UV irradiation were obtained by fitting differential spectra calculated before and after UV irradiation using spectra of the dark state, the Meta II, and the Meta III as templates (Table 3). These results indicate that recovery to the original dark state by UV irradiation is most efficiently caused in the G188C mutant.

### (Example 3: Faster photocycle in G188C mutant)

Next, whether a change in lifetime of the Meta II alters a photocycle rate of the G188C mutant was examined. FIG. 3 showed faster recovery of a photocycle property of the G188C mutant of bovine rhodopsin by introducing an E122Q mutation. FIG. 3A shows absorption spectra of the E122Q/G188C/N2C/D282C mutant as measured at 0°C. The spectra were recorded in the dark (Curve 1) and at 0, 5, and 60 min (Curves 2 to 4, respectively) after irradiation with yellow light (> 500 nm). The inset shows differential spectra (Curves 1 to 3, respectively) each obtained by subtracting the spectrum before irradiation (Curves 1 in FIG. 3A) from each of the spectra measured after irradiation (Curves 2 to 4 in FIG. 3A). FIG. 3B shows an isomeric composition of retinal of the E122Q/G188C/N2C/D282C mutant. Chromophores were extracted from samples before light irradiation and at 0, 5, and 60 minutes after irradiation with yellow light and analyzed for retinal configuration by high-performance liquid chromatography (HPLC). FIG. 3C shows absorbance spectra of the E122Q/G188C/N2C/D282C mutant as measured at 37°C. The spectra were recorded in the dark (Curve 1) and at 0.1, 1, 5, 10, and 50 seconds (Curves 2 to 6, respectively) after irradiation with yellow flashlight. The inset shows differential spectra (Curves 1 to 5, respectively) each obtained by subtracting the spectra before irradiation (Curves 1 in FIG. 3C) from each of the spectra measured after irradiation (Curves 2 to 6 in FIG. 3C). FIG. 3D is a graph showing comparison between thermal recovery processes of G188C/N2C/D282C and E122Q/G188C/N2C/D282C. Differential absorbance at λmax each obtained by subtracting the spectrum before irradiation from each of the spectra measured after irradiation shown in FIGs. 1G and 3C was plotted against elapsed time after irradiation. Time constants for thermal recovery to the dark state at 37°C for the G188C/N2C/D282C and E122Q/G188C/N2C/D282C mutants were 57.4 seconds and 5.1 seconds, respectively.

E122Q mutation in vertebrate rhodopsin has been reported to promote a decay of the Meta II and shorten lifetime of the Meta II (Imai et al., 1997; Imai et al., 2007). Therefore, an E122Q/G188C/N2C/D282C mutant was produced and measured for spectral changes after light irradiation. Spectral and retinal isomer analyses showed that the E122Q/G188C/N2C/D282C mutant thermally recovered to the original dark state after light irradiation at 0°C (FIGs. 3A, B). A photocycle rate of the E122Q/G188C/N2C/D282C mutant at 37°C (FIG. 3C) was about 12 times faster than that of the G188C/N2C/D282C mutant (FIG. 3D). Thus, a photocycle reaction of the G188C mutant was successfully faster by changing lifetime of Meta II due to a single mutation.

### (Example 4: Change in G-protein activation ability by photocycle property)

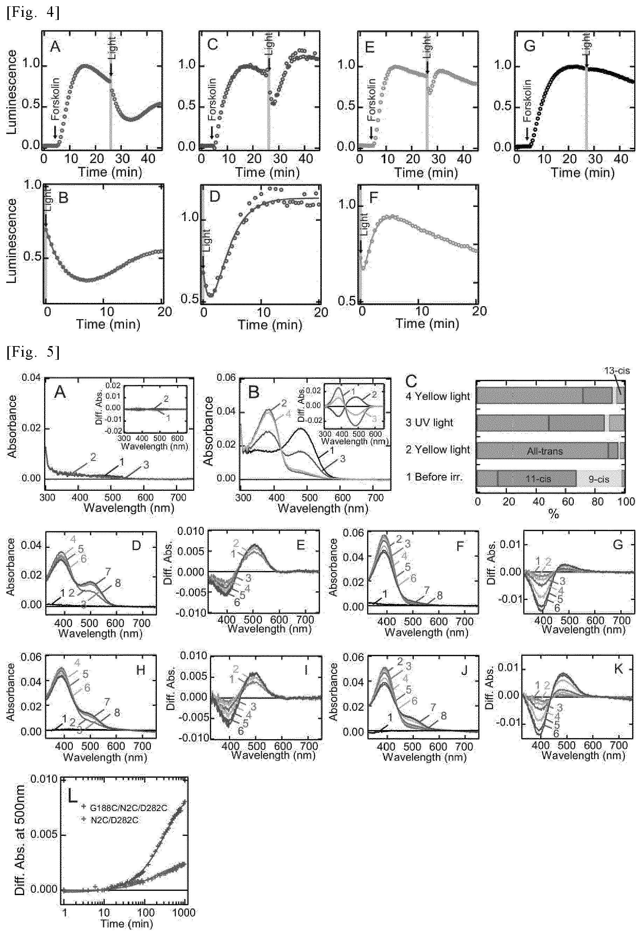

Whether acquisition of a photocycle property by the G188C mutation affects a G protein activation ability was also examined. FIG. 4 shows suppression of an intracellular cAMP level by light in a bovine rhodopsin mutant. HEK293T cells transfected with N2C/D282C- (FIGs. 4A, B), G188C/N2C/D282C-(FIGs. 4C, D), E122Q/G188C/N2C/D282C- (FIGs. 4E, F), and mock (FIG. 4G) were measured for a cAMP level using the GloSensor cAMP assay at room temperature. The cells were incubated with 5 µM 11-cis retinal for 2 hours and then treated with 2 µM forskolin prior to exposure to yellow light (> 500 nm). Data were normalized to the maximum point before light irradiation. Detailed profiles of changes in a light-dependent cAMP level in the N2C/D282C, the G188C/N2C/D282C, and the E122Q/G188C/N2C/D282C are shown in FIGs. 4B, 4D, and 4F, respectively.

As shown in FIG. 2, a light-dependent Gi activation ability was comparable between the wild type and the G188C mutant at 0°C, and significant thermal recovery to the original dark state was not observed in the G188C mutant. Therefore, an amount of intracellular cAMP in cultured cells using the cAMP biosensor (GloSensor) was measured and changes in biosensor emission triggered by bovine rhodopsin were compared. It was revealed that an increase in a cAMP level induced by addition of forskolin was attenuated by yellow light irradiation in N2C/D282C bovine rhodopsin-transfected cells (FIGs. 4A, B) but not in mock-transfected cells (FIG. 4G), followed by a gradual recovery. On the other hand, it was confirmed that the G188C/N2C/D282C mutant-introduced cells recovered quickly after a decrease in a cAMP level induced by irradiation with yellow light (FIGs. 4C, D). It was also confirmed that the E122Q/G188C/N2C/D282C mutant-introduced cells more rapidly recovered from the decrease in a cAMP level induced by irradiation with yellow light (FIGs. 4E, F). These results indicate that acquisition of a photocycle property by the G188C mutation promotes rapid recovery to the original dark state and changes a G protein activation profile.

### (Example 5: Pigment formation by G188C mutant upon addition of all-trans retinal)

Whether the G188C mutant forms a photopigment after reconstitution with all-trans retinal was analyzed. FIG. 5 shows formation of a photopigment in the G188C mutant of bovine rhodopsin after incubation with all-trans retinal. Absorption spectra of the wild type (FIG. 5A) or the G188C mutant (FIG. 5B) that had been prepared by adding all-trans retinal to a suspension of a rhodopsin-expressing cell membrane at 0°C and then purifying are shown. The spectra were measured in the dark (Curve 1), after irradiation with yellow light (>500 nm) (Curve 2), after subsequent irradiation with UV light (360 nm) (Curve 3), and after reirradiation with yellow light (Curve 4). The inset shows spectral changes caused by irradiation with yellow light (Curve 1), subsequent irradiation with UV light (Curve 2), and reirradiation with yellow light (Curve 3). FIG. 5C shows an isomeric composition of retinal of the G188C mutant that had been added with all-trans retinal and then purified. Chromophores were extracted from samples after irradiation with yellow light, after subsequent irradiation with UV, and after reirradiation with yellow light and analyzed for retinal configuration by high-performance liquid chromatography (HPLC). Regeneration of a photopigment when 11-cis retinal (FIG. 5D, E) or all-trans retinal (FIG. 5F, G) is added to a purified apoprotein of N2C/D282C is shown. Spectra in FIG. 5D were measured before addition (Curve 1) and at 0, 3, 6, 15, 30, 60, and 120 min after addition of 1.1 µM 11-cis retinal (Curves 2 to 8). FIG. 5E shows differential spectra each calculated by subtracting each of the spectra measured 3, 6, 15, 30, 60, and 120 min after addition of 11-cis-retinal (Curves 3 to 8 in FIG. 5D) from the spectrum immediately after addition of 11-cis-retinal (Curve 2 in FIG. 5D) (Curves 1 to 6, respectively). FIG. 5F shows spectra measured before (Curve 1) and at 0, 0.5, 1, 2, 6, 12, and 16 hours after addition of 1.1 µM all-trans retinal (Curves 2 to 8). The differential spectra in FIG. 5G were calculated by subtracting the spectrum immediately after addition of all-trans retinal (Curve 2 in FIG. 5F) from each of the spectra measured at 0.5, 1, 2, 6, 12, and 16 hours after addition of all-trans retinal (Curves 3 to 8 in FIG. 5F) (Curves 1 to 6, respectively). Regeneration of a photopigment when 11-cis retinal (FIGs. 5H, I) or all-trans retinal (FIGs. 5J, G) is added to a purified apoprotein of G188C/N2C/D282C is shown. The spectra in FIG. 5H were measured before addition (Curve 1) and at 0, 3, 6, 15, 30, 60, and 120 min after addition of 1.1 µM 11-cis retinal (Curves 2 to 8). FIG. 5I shows differential spectra each calculated by subtracting each of the spectra measured at 3, 6, 15, 30, 60, and 120 min after addition of 11-cis-retinal (Curves 3 to 8 in FIG. 5H) from the spectrum immediately after addition of 11-cis-retinal (Curve 2 in FIG. 5H) (Curves 1 to 6, respectively). FIG. 5J shows spectra measured before (Curve 1) and at 0, 0.5, 1, 2, 6, 12, and 16 hours after addition of 1.1 µM all-trans retinal (Curves 2 to 8). The differential spectra in FIG. 5K were calculated by subtracting the spectrum immediately after addition of all-trans retinal (Curve 2 in FIG. 5J) from each of the spectra measured at 0.5, 1, 2, 6, 12, and 16 hours after addition of all-trans retinal (Curves 3 to 8 in FIG. 5J) (Curves 1 to 6, respectively). FIG. 5L is a graph showing regeneration processes of photopigment formation by the N2C/D282C and the G188C/N2C/D282C induced by addition of all-trans retinal shown in FIGs. 5F and 5J as monitored by a change in absorbance at 500 nm.

All-trans retinal was added to a suspension of a rhodopsin-expressing cell membrane and then the wild-type and the G188C mutant were purified. The absorption spectrum of the wild type had few peaks in the visible and near-ultraviolet regions (FIG. 5A). On the other hand, the absorption spectrum of the G188C mutant had a peak in the visible region (Curve 1 in FIG. 5B), which was due to predominant uptake of 11-cis and 9-cis retinal rather than all-trans retinal (FIG. 5C). Irradiation of this pigment with yellow light converted retinal to its all-trans form and shifted the spectrum to the UV region and subsequent irradiation with UV light caused retinal to isomerize from the all-trans form to a 11-cis form at 0°C and absorbance in the visible region increased again (FIGs. 5B, C). This was similar to the finding for the G188C mutant that had been reconstituted with 11-cis retinal and then purified (FIGs. 2B, D).

Purified apoproteins of N2C/D282C and G188C/N2C/D282C were also prepared and a regeneration process of photopigment by addition of 11-cis or all-trans retinal was examined. Addition of 11-cis retinal to the N2C/D282C and the G188C/N2C/D282C immediately increased absorbance around 505 nm (FIG. 5D, E) and 490 nm (FIGs. 5H, I), respectively, indicating formation of an 11-cis retinal-bound dark state. Addition of all-trans retinal to the N2C/D282C slightly increased absorbance around 480 nm (FIGs. 5F, G), while addition of all-trans retinal to the G188C/N2C/D282C greatly increased absorbance around 485 nm (FIGs. 5J, K). A regenerative capacity of the G188C/N2C/D282C in response to the addition of all-trans retinal was much higher than that of the N2C/D282C (FIG. 5L). These results indicate that the G188C mutant can uniquely form the photopigment by adding not only 11-cis retinal but also all-trans retinal.

### (Example 6: Chimeric opsin)

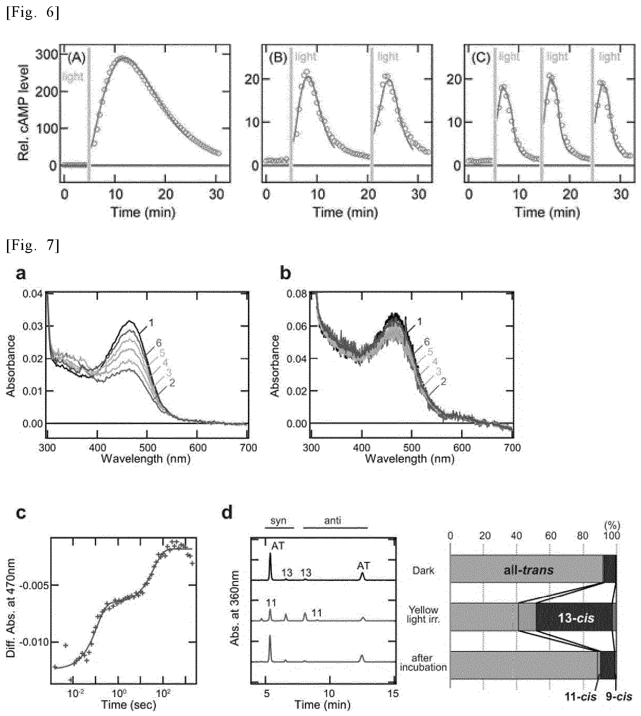

For bovine rhodopsin N2C/D282C, as in the above example, the G188C mutant, the E122Q/G188C mutant, and a chimeric opsin in which intracellular second and third loops (amino acid numbers 140 to 152 and 225 to 251 of Gene ID: 509933, respectively) had been replaced by those of a mouse histamine H2 receptor (amino acid numbers 121 to 134 and 203 to 232 of Gene ID: 15466, respectively) was expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. A transient increase and then an immediate decrease in luminescence was observed with bovine rhodopsin N2C/D282C to which a G188C mutation (FIG. 6B) or an E122Q/G188C mutation (FIG. 6C) had been introduced compared to bovine rhodopsin N2C/D282C (FIG. 6A) in which the intracellular second and third loops had been replaced. These results indicate that introduction of the G188C or E122Q/G188C mutation allows bovine rhodopsin to be modified so as to transiently increase an intracellular cAMP concentration and return it with light.

### (Example 7: Acquisition of photocycle property in Western clawed frog Opn5m T188C mutant)

Western clawed frog (Xenopus tropicalis) Opn5m binds to 11-cis retinal and its absorption spectrum showed the absorption maximum at 360 nm. When it received UV light, the retinal isomerized to the all-trans form and the absorption maximum of the absorption spectrum shifted to 474 nm. When it further received the visible light, the retinal isomerized to the 11-cis form and returned to an absorption spectrum with an absorption maximum at 360 nm. In other words, the Opn5m was a bistable opsin (Yamashita et al., 2014). A T188C mutant was produced since Western clawed frog Opn5m has a threonine at position 188. The T188C mutant binds to only all-trans and its absorption spectrum showed an absorption maximum at 470 nm. When it received the visible light, absorption at 470 nm decreased once and then recovered over time at both 20°C (FIG. 7a) and 37°C (FIGs. 7b and 7c). Furthermore, during this process, the retinal changed from the all-trans form to the 11-cis or 13-cis form upon reception of the visible light and returned to the all-trans form over time (FIG. 7d). These results indicate that the Western clawed frog Opn5mT188C mutant spontaneously returns after photoreception and thus it can be said that the mutant had acquired a photocycle property.

### (Example 8: Human rhodopsin)

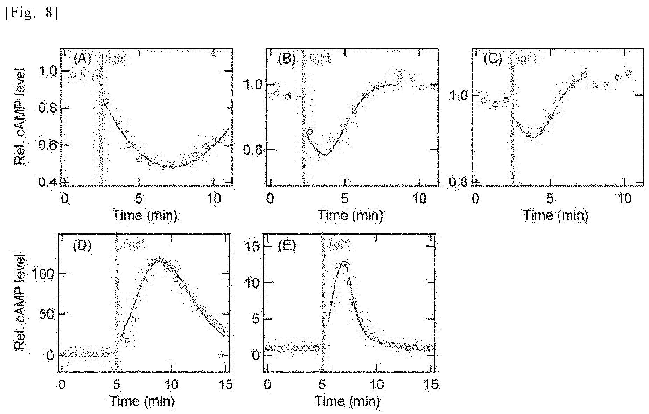

For human rhodopsin N2C/N282C, as in Example for bovine rhodopsin, the G188C and E122Q/G188C mutants were expressed in human-derived cultured cells HEK293. In this case, modified luciferase (Promega, GloSensor) was coexpressed as a probe for cAMP so that luminescence was higher when a cAMP concentration was higher. The cultured cells were irradiated with yellow light and luminescence derived from the modified luciferase was compared. A transient decrease and then an immediate return in luminescence upon light irradiation was observed with the human rhodopsin N2C/N282C to which the G188C mutation (FIG. 8B) or the E122Q/G188C mutation (FIG. 8C) had been introduced compared to human rhodopsin N2C/N282C (FIG. 8A). This result indicates that introduction of the G188C or E122Q/G188C mutation allows human rhodopsin to be modified so as to decrease an intracellular cAMP concentration for a short period of time with light. For human rhodopsin N2C/N282C and G188C/N2C/N282C mutants, a chimeric opsin in which intracellular second and third loops (amino acid numbers 140 to 152 and 225 to 251 of SEQ ID NO: 1, respectively) had been further replaced by those of a human histamine H2 receptor (amino acid numbers 121 to 134 and 204 to 233 of Gene ID: 3274, respectively) was expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. A transient increase and then an immediate decrease in luminescence was observed with human rhodopsin N2C/N282C to which a G188C mutation (FIG. 8E) had been introduced compared to human rhodopsin N2C/N282C in which the intracellular second and third loops had been replaced (FIG. 8D). This result indicates that introduction of the G188C mutation allows human rhodopsin to be modified so as to increase an intracellular cAMP concentration for a short period of time and return it with light.

### (Example 9: Canine Rhodopsin)

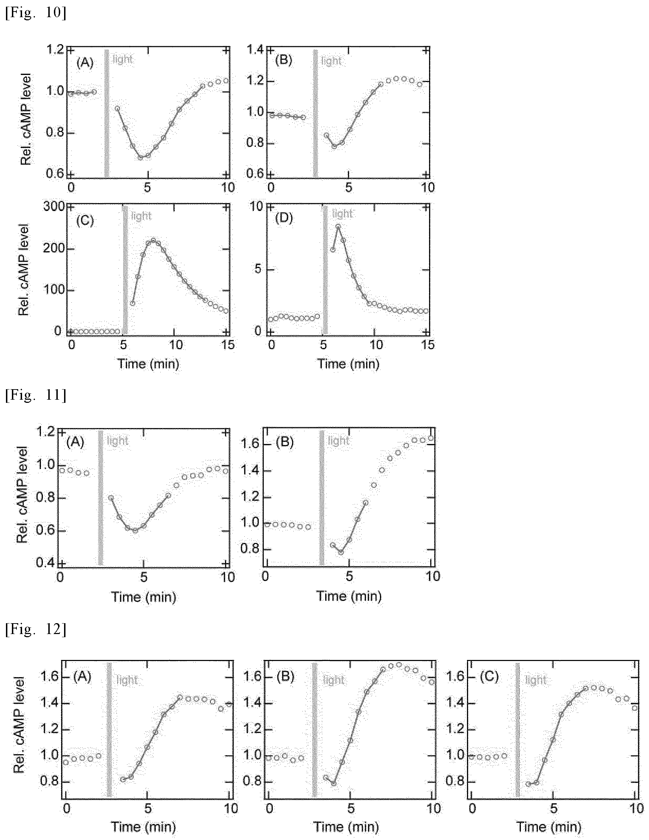

For canine (Canis familiaris) rhodopsin N2C/D282C, as in Example for bovine rhodopsin, the G188C mutant was expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. A transient increase and then an immediate decrease in luminescence upon light irradiation was observed with the canine rhodopsin N2C/D282C to which the G188C mutation had been introduced (FIG. 10B) compared to canine rhodopsin N2C/D282C (FIG. 10A). This result indicates that introduction of the G188C mutation allows canine rhodopsin to be modified so as to decrease an intracellular cAMP concentration for a short period of time with light. For canine rhodopsin N2C/D282C and G188C/N2C/D282C mutants, a chimeric opsin in which intracellular second and third loops had been further replaced by those of a human histamine H2 receptor was expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. A transient increase and then an immediate decrease in luminescence was observed with the canine rhodopsin N2C/D282C to which the G188C mutation had been introduced (FIG. 10D) compared to canine rhodopsin N2C/D282C in which the intracellular second and third loops had been replaced (FIG. 10C). This result indicates that introduction of the G188C mutation allows canine rhodopsin to be modified so as to increase an intracellular cAMP concentration for a short period of time and return it with light.

### (Example 10: Medaka rhodopsin)

For medaka (Oryzias latipes) rhodopsin N2C/E282C, as in Example for bovine rhodopsin, the G188C mutant was expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. A transient decrease and then an immediate return in luminescence upon light irradiation was observed with medaka rhodopsin N2C/E282C to which the G188C mutation had been introduced (FIG. 11B) compared to medaka rhodopsin N2C/E282C (FIG. 11A). This result indicates that introduction of the G188C mutation allows medaka rhodopsin to be modified so as to decrease an intracellular cAMP concentration for a short period of time with light.

### (Example 11: Additional mutation other than at positions 188 and 122)

Conservative amino acid substitutions were made in a N-terminal domain (positions 1 to 34) and a C-terminal domain (positions 308 and later) when aligned with SEQ ID NO: 1. Specifically, a G6A mutation or a V337A mutation was introduced into the human rhodopsin G188C/N2C/D282C mutant and expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. Similar to the human rhodopsin G188C/N2C/D282C mutant (FIG. 12A), a transient decrease and then an immediate return in luminescence upon light irradiation was observed with the human rhodopsin G6A/G188C/N2C/D282C mutant (FIG. 12B) and the human rhodopsin V337A/G188C/N2C/D282C mutant (FIG. 12C). This result indicates that these three mutants can decrease an intracellular cAMP concentration for a short period of time with light. In other words, this indicates that even if the conservative amino acid substitutions were made in the N-terminal domain (positions 1 to 34) and the C-terminal domain (positions 308 and later) when aligned with SEQ ID NO: 1, an ability to decrease an intracellular cAMP concentration for a short period of time with light is retained.

### (Example 12: Introduction of cysteine mutation other than at positions 2 and 282)

In combinations other than amino acids corresponding to positions 2 and 282 when aligned with SEQ ID NO: 1, amino acids corresponding to an amino acid belonging to a portion of a N-terminal domain (positions 1 to 11) and an amino acid belonging to a portion of an extracellular third loop (positions 278 to 285) when aligned with SEQ ID NO: 1 were modified to cysteine. Specifically, a N2C/G3C/G280C mutation, a N2C/G3C/S281C mutation, or a G3C/N282C mutation was introduced into the human rhodopsin G188C mutant and expressed in human-derived cultured cells HEK293. In this case, the modified luciferase (Promega, GloSensor) was coexpressed as a probe for cAMP so that luminescence was higher at a higher cAMP concentration. The cultured cells were irradiated with yellow light and luminescence derived from the modified luciferase was compared. Similar to the human rhodopsin G188C/N2C/D282C mutant (FIG. 13A), a transient decrease and then an immediate return in luminescence upon light irradiation was observed with the human rhodopsin G188C/N2C/G3C/G280C mutant (FIG. 13B), the human rhodopsin G188C/N2C/G3C/S281C mutant (FIG. 13C), and the human rhodopsin G188C/G3C/N282C mutant (FIG. 13D). These results indicate that these four mutants can decrease an intracellular cAMP concentration for a short period of time with light. In other words, this indicates that even if cysteine is introduced into the amino acids corresponding to a portion of the N-terminal domain (positions 1 to 11) and a portion of the extracellular third loop (positions 278 to 285) when aligned with SEQ ID NO: 1 other than the combination of amino acids corresponding to positions 2 and 282, an ability to decrease an intracellular cAMP concentration for a short period of time with light is retained.

### (Example 13: Restoration of light-induced activity from retina by G188C mutant)

A viral vector (rAAV-DJ) containing a sequence encoding the G188C mutant under the control of a CMV promoter was injected into the vitreous of 10-week-old rd1 mice (retinitis pigmentosa blindness model mice). An AAV-DJ vector was employed for more efficient and extensive gene transfer and an AAV-2 was employed as a benchmark that has already been clinically applied. The retina was taken one month later. A reporter gene (EGFP) was expressed throughout the retina and in both the ganglion cell layer (GCL) and the internal granular layer (INL). To evaluate a function of the G188C mutant ectopically induced in the mouse retina, a multi-electrode array (MEA) test that can record an extracellular potential of RGCs was performed. The test was performed as shown in FIG. 14. FIG. 14A shows the case of an untreated rd1 mouse. FIG. 14B shows the case in which a G188C/N2C/D282C mutant of human rhodopsin was introduced with a viral vector, FIG. 14C shows the case in which intracellular second and third loops of a G188C/N2C/D282C mutant of human rhodopsin were replaced by those of a human histamine H2 receptor, and FIG. 14D shows the case in which intracellular second and third loops of an E122Q/G188C/N2C/D282C mutant of human rhodopsin were replaced by those of a human histamine H2 receptor. These mutants are the same as those described in Example 8 and elsewhere.

### (Results)

The results are presented in Table 14. No photoresponse is observed in the untreated rd1 mouse because it is blind (FIG. 14A), but when the G188C/N2C/D282C mutant of human rhodopsin was introduced with a viral vector (FIG. 14B), when intracellular second and third loops of the G188C/N2C/D282C mutant of human rhodopsin were replaced by those of the human histamine H2 receptor (FIG. 14C), and when intracellular second and third loops of the E122Q/G188C/N2C/D282C mutant of human rhodopsin were replaced by those of a human histamine H2 receptor (FIG. 14D), the photoresponse was observed. This result indicates that introduction of the G188C mutant can give a vision regenerative effect. (

As a result of degeneration of a photoreceptor, a control retina without treatment showed no response from RGCs detected by the MEA (FIG. 14A). In contrast, the treated retinae repeatedly showed clear light-induced responses (FIGs. 14B, C, D).

### (Example 14: Evaluation of visual evoked potential)

A visual evoked potential (VEP) from the visual cortex is examined to investigate whether photoreception in the retina is transmitted to the visual cortex. In this experiment, a rd1 mouse of which both eyes had been treated with a G188C mutant construct linked to AAV-DJ-CAGGS and a control EGFP virus (AAV-DJ-CAGGS-EGFP) were used. As a result, no VEP response is obtained in the control mouse, but a significant VEP response is regenerated in the mice administered with the G188C mutant construct linked to AAV-DJ-CAGGS.

### (Example 15: Evaluation of light/dark recognition function)

A light/dark box transition test (LDT) was performed to investigate whether ectopic expression of the G188C mutant in the degenerative retina leads to a behavioral change following visual recovery. Rodents are nocturnal and tend to remain in the dark according to their visual function because they are anxious in a bright environment, while blind animals spend almost half their time in a light area and almost half in a dark area. A mouse treated with the G188C mutant spent significantly less time in the light area compared to an untreated rd1 mutant mouse, indicating a visual recovery in its behavior.

### (Example 16: Optogenetics <regulation of nerve>)

Since cAMP is essential for induction of neuroaxonal branching and elongation, the G188C mutant and the chimeric opsin in which intracellular second and third loops had been replaced by those of the human histamine H2 receptor as in Example 8 were expressed in mouse hippocampal neurons to attempt light manipulation of axonal branching and elongation in neurons. As a result, axonal branching and elongation were suppressed in hippocampal neurons that had been transfected with the G188C mutant irradiated with blue light for 30 minutes compared to those not irradiated. On the other hand, hippocampal neurons that had been transfected with the G188C mutant chimeric opsin irradiated with blue light for 30 minutes showed enhanced axonal branching and elongation compared to those not irradiated.

### (Example 17: Calcium ion-altered chimeric opsin)

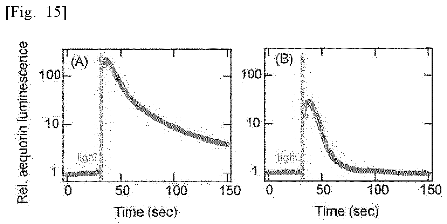

For bovine rhodopsin N2C/D282C, as in Example 6, the G188C mutant and a chimeric opsin in which intracellular second and third loops had been further replaced by those of a human α1A adrenergic receptor were expressed in human-derived cultured cells HEK293. In this case, aequorin from Aequorea victoria was coexpressed as a probe for a calcium ion so that luminescence was higher at a higher calcium ion concentration. The cultured cells were irradiated with yellow light and luminescence derived from aequorin was compared. A transient increase and then an immediate decrease in luminescence was observed with those with the G188C mutation (FIG. 15B) compared to bovine rhodopsin N2C/D282C in which the intracellular second and third loops had been replaced (FIG. 15A). This result indicates that introduction of the G188C mutation allows the bovine rhodopsin N2C/D282C to be modified so as to increase an intracellular calcium ion concentration only for a short period of time and return it with light.

### (Notes)

Although the present disclosure has been illustrated with reference to preferred embodiments of the present disclosure, it is understood that the scope of the present disclosure should be construed only by the claims. It is understood that the patents, patent applications, and other references cited herein should be incorporated herein by reference in its entirety as if the contents themselves are specifically set forth herein. This application claims priority to Japanese Patent Application No. 2022-138707 filed on August 31, 2022 with the Japan Patent Office, the content of which is incorporated herein by reference in their entirety, as necessary.

### [Industrial Applicability]

According to the present disclosure, an evolutionary mechanism of nature that gave a photocycle property to a specific opsin can be used to artificially evolve another opsin to have the photocycle property as well. The present disclosure provides techniques available in production of various molecular tools and in industries based on such techniques (e.g., pharmaceuticals).

### [Sequence Listing Free Text]

SEQ ID NO 1: Amino acid sequence of Homo sapiens rhodopsin NP_001372054.1
SEQ ID NO 2: Amino acid sequence of Mus musculus rhodopsin NP_663358.1
SEQ ID NO 3: Amino acid sequence of Canis familiaris rhodopsin CAA50502.1
SEQ ID NO 4: Amino acid sequence of Gallus gallus rhodopsin NP_001384426.1
SEQ ID NO 5: Amino acid sequence of Oryzias latipes rhodopsin BAD99136.1
SEQ ID NO 6: Amino acid sequence of Homo sapiens blue cone opsin NP_001372054.1
SEQ ID NO 7: Amino acid sequence of Homo sapiens red cone opsin NP_064445.2
SEQ ID NO 8: Amino acid sequence of Homo sapiens green cone opsin NP_000504.1
SEQ ID NO 9: Amino acid sequence of Mus musculus UV cone opsin AAG17989.1
SEQ ID NO 10: Amino acid sequence of Mus musculus green cone opsin AAB64302.1
SEQ ID NO 11: Amino acid sequence of Gallus gallus green cone opsin AAA48786.1
SEQ ID NO 12: Amino acid sequence of Gallus gallus blue cone opsin AAA48633.1
SEQ ID NO 13: Amino acid sequence of Gallus gallus violet cone opsin AAA49141.1
SEQ ID NO 14: Amino acid sequence of Gallus gallus red cone opsin CAA40727.1
SEQ ID NO 15: Amino acid sequence of Gallus gallus pinopsin AAA64223.1
SEQ ID NO 16: Amino acid sequence of Homo sapiens Opn3 AAH36773.1
SEQ ID NO 17: Amino acid sequence of Homo sapiens Opn4 AAI13559.1
SEQ ID NO 18: Amino acid sequence of Homo sapiens Opn5 AAR21109.1
SEQ ID NO 19: Amino acid sequence of Homo sapiens Rgr AAA56748.1
SEQ ID NO 20: Amino acid sequence of Homo sapiens Rrh AAC51757.1
SEQ ID NO 21: Amino acid sequence of Mus musculus Opn3 AAD32670.1
SEQ ID NO 22: Amino acid sequence of Mus musculus Opn4 AAF24979.1
SEQ ID NO 23: Amino acid sequence of Mus musculus Opn5 AAR08201.1
SEQ ID NO 24: Amino acid sequence of Mus musculus Rgr AAC69836.1
SEQ ID NO 25: Amino acid sequence of Mus musculus Rrh AAC53344.1
SEQ ID NO 26: Amino acid sequence of Gallus gallus VAL opsin ACX32474.1
SEQ ID NO 27: Amino acid sequence of Gallus gallus Opn3 BAV92607.1
SEQ ID NO 28: Amino acid sequence of Gallus gallus TMT opsin BAV93805.1
SEQ ID NO 29: Amino acid sequence of Gallus gallus Opn4x ABX10830.1
SEQ ID NO 30: Amino acid sequence of Gallus gallus Opn4m BAL14786.1
SEQ ID NO 31: Amino acid sequence of Gallus gallus Opn5m BAG65738.1
SEQ ID NO 32: Amino acid sequence of Gallus gallus Opn5L2 BAG65739.2
SEQ ID NO 33: Amino acid sequence of Gallus gallus Rrh AAR02098.1
SEQ ID NO 34: Amino acid sequence of Gallus gallus Rgr AAR02099.1
SEQ ID NO 35: Nucleic acid sequence of Homo sapiens rhodopsin NM_000539.3
SEQ ID NO 36: Nucleic acid sequence of Mus musculus rhodopsin NM_145383.2
SEQ ID NO 37: Nucleic acid sequence of Canis familiaris rhodopsin X71380.1
SEQ ID NO 38: Nucleic acid sequence of Gallus gallus rhodopsin NM_001397497.1
SEQ ID NO 39: Nucleic acid sequence of Oryzias latipes rhodopsin AB180742.1
SEQ ID NO 40: Nucleic acid sequence of Homo sapiens blue cone opsin NM_001385125.1
SEQ ID NO 41: Nucleic acid sequence of Homo sapiens red cone opsin NM_020061.6
SEQ ID NO 42: Nucleic acid sequence of Homo sapiens green cone opsin NM_000513.2
SEQ ID NO 43: Nucleic acid sequence of Mus musculus UV cone opsin AF190670.1
SEQ ID NO 44: Nucleic acid sequence of Mus musculus green cone opsin AF011389.1
SEQ ID NO 45: Nucleic acid sequence of Gallus gallus green cone opsin M92038.1
SEQ ID NO 46: Nucleic acid sequence of Gallus gallus blue cone opsin M92037.1
SEQ ID NO 47: Nucleic acid sequence of Gallus gallus violet cone opsin M92039.1
SEQ ID NO 48: Nucleic acid sequence of Gallus gallus red cone opsin X57490.1
SEQ ID NO 49: Nucleic acid sequence of Gallus gallus pinopsin U15762.1
SEQ ID NO 50: Nucleic acid sequence of Homo sapiens Opn3 BC036773.1
SEQ ID NO 51: Nucleic acid sequence of Homo sapiens Opn4 BC113558.1
SEQ ID NO 52: Nucleic acid sequence of Homo sapiens Opn5 AY377391.1
SEQ ID NO 53: Nucleic acid sequence of Homo sapiens Rgr U14910.1
SEQ ID NO 54: Nucleic acid sequence of Homo sapiens Rrh AF012270.1
SEQ ID NO 55: Nucleic acid sequence of Mus musculus Opn3 AF140241.1
SEQ ID NO 56: Nucleic acid sequence of Mus musculus Opn4 AF147789.1
SEQ ID NO 57: Nucleic acid sequence of Mus musculus Opn5 AY318865.1
SEQ ID NO 58: Nucleic acid sequence of Mus musculus Rgr AF076930.1
SEQ ID NO 59: Nucleic acid sequence of Mus musculus Rrh AF012271.1
SEQ ID NO 60: Nucleic acid sequence of Gallus gallus VAL opsin GQ280390.1
SEQ ID NO 61: Nucleic acid sequence of Gallus gallus Opn3 AB436160.1
SEQ ID NO 62: Nucleic acid sequence of Gallus gallus TMT opsin AB519059.1
SEQ ID NO 63: Nucleic acid sequence of Gallus gallus Opn4x EU124630.1
SEQ ID NO 64: Nucleic acid sequence of Gallus gallus Opn4m AB295599.1
SEQ ID NO 65: Nucleic acid sequence of Gallus gallus Opn5m AB368182.1
SEQ ID NO 66: Nucleic acid sequence of Gallus gallus Opn5L2 AB368183.3
SEQ ID NO 67: Nucleic acid sequence of Gallus gallus Rrh AY339626.1
SEQ ID NO 68: Nucleic acid sequence of Gallus gallus Rgr AY339627.1

## Claims

1. A protein comprising
an amino acid sequence of an opsin,
the protein comprising a modification at an amino acid corresponding to position 188 when aligned with SEQ ID NO: 1 in the amino acid sequence of the opsin.

2. A protein comprising
an amino acid sequence of an opsin,
the protein comprising a modification at an amino acid corresponding to G at position 188 when aligned with SEQ ID NO: 1 in the amino acid sequence of the opsin.

3. The protein according to claim 1 or 2, comprising a modification to cysteine of an amino acid corresponding to position G188 when aligned with SEQ ID NO: 1 in the amino acid sequence.

4. The protein according to any one of claims 1 to 3, wherein the protein is inactivated without releasing a photoreceptive factor after being activated by photostimulation.

5. The protein according to any one of claims 1 to 4, wherein the amino acid sequence comprises
1) an amino acid sequence comprising the modification in an amino acid sequence represented by any one of SEQ ID NOs: 1 to 34;
2) an amino acid sequence comprising a sequence other than a site of the modification having an identity of at least about 80% to the sequence in 1), and encoding a protein having substantially a same biological activity as that of a protein obtained from the sequence in 1);
3) an amino acid sequence having one or more mutations in the sequence in 1) other than the site of the modification and encoding a protein having substantially a same biological activity as that of a protein obtained from the sequence in 1);
4) an amino acid sequence comprising the modification in an amino acid sequence encoded by a nucleic acid to which a nucleic acid encoding the sequence in 1) hybridizes; or
5) an amino acid sequence comprising the modification in an amino acid sequence encoded by an allelic variant of the nucleic acid encoding the sequence in 1).

6. The protein according to any one of claims 1 to 5, further comprising a modification of an amino acid corresponding to position 122 when aligned with SEQ ID NO: 1 in the amino acid sequence of the opsin.

7. The protein according to any one of claims 1 to 6, further comprising a modification of an amino acid corresponding to E at position 122 when aligned with SEQ ID NO: 1 in the amino acid sequence of the opsin.

8. The protein according to any one of claims 1 to 7, further comprising a modification to glutamine of an amino acid corresponding to position 122 when aligned with SEQ ID NO: 1 in the amino acid sequence of the opsin.

9. The protein according to claim 4, wherein the photoreceptive factor comprises retinal.

10. The protein according to any one of claims 1 to 9, further comprising modifications of amino acids corresponding to an amino acid belonging to a portion of an N-terminal domain (positions 1 to 11) and an amino acid belonging to a portion of an extracellular third loop (positions 278 to 285) when aligned with SEQ ID NO: 1 in the amino acid sequence of the opsin.

11. The protein according to any one of claims 1 to 10, further comprising modifications to cysteine of amino acids corresponding to an amino acid belonging to a portion of the N-terminal domain (positions 1 to 11) and an amino acid belonging to a portion of the extracellular third loop (positions 278 to 285) when aligned with SEQ ID NO: 1 in the amino acid sequence of the opsin.

12. The protein according to any one of claims 1 to 11, further comprising modifications of amino acids corresponding to positions 2 and 282 when aligned with SEQ ID NO: 1 in the amino acid sequence of the opsin.

13. The protein according to claim 12, wherein the modifications of the amino acids corresponding to positions 2 and 282 improves a thermal stability of the opsin.

14. The protein according to claim 12 or 13, wherein the modifications of the amino acids corresponding to positions 2 and 282 comprises a modification to cysteine of the amino acids corresponding to positions 2 and 282.

15. The protein according to any one of claims 1 to 14, wherein the opsin is a chimeric opsin.

16. The protein according to any one of claims 1 to 14, comprising a sequence of SEQ ID NO: 1, 3, or 5.

17. A nucleic acid molecule comprising a nucleic acid encoding an amino acid sequence of the protein according to any one of claims 1 to 16.

18. A nucleic acid construct comprising the nucleic acid molecule according to claim 17.

19. A cell comprising:
the protein according to any one of claims 1 to 16;
the nucleic acid molecule according to claim 17; and/or
the nucleic acid construct according to claim 18.

20. A medicament comprising:
the protein according to any one of claims 1 to 16;
the nucleic acid molecule according to claim 17;
the nucleic acid construct according to claim 18; and/or
the cell according to claim 19.

21. The medicament according to claim 20 for regenerating vision or for preventing or treating a visual disorder or disease.

22. A composition comprising
opsin that is inactivated without releasing a photoreceptive factor.

23. The composition according to claim 22, wherein the opsin makes a transient change in a cAMP concentration upon photostimulation.

24. The composition according to claim 23, wherein the transient change in the cAMP concentration is a decrease in the cAMP concentration.
